# EUROPEAN PATENT SPECIFICATION

(11) **EP 1 581 480 B1**
(45) Date of publication and mention of the grant of the patent: **23.08.2006**
(21) Application number: 03813673.5
(22) Date of filing: 10.12.2003
(51) Int. Cl.: C07C 235/40, C07C 233/63, C07C 255/54, A61K 31/44, A61K 31/194, A61K 31/275, A61K 31/22, A61K 31/4453, C07D 213/30, C07D 295/182

(54) **CYCLOPENTYL-SUBSTITUTED GLUTARAMIDE COMPOUNDS AS ENDOPEPTIDASE-V INHIBITORS**
CYCLOPENTYLSUBSTITUIERTEGLUTARAMIDVERBINDUNGEN ALS ENDOPEPTIDASE-V-INHIBITOREN
COMPOSES GLUTARAMIDE A SUBSTITUTION CYCLOPENTYLE UTILISES COMME INHIBITEURS DE L'ENDOPEPTIDASE-V

(30) Priority: 23.12.2002 GB 0230036
(43) Date of publication of application: 05.10.2005
(73) Proprietor: Pfizer Limited, Sandwich, Kent CT13 9NJ (GB); PFIZER INC., New York, N.Y. 10017 (US)
(72) Inventor: DACK, Devin Pfizer Global Res. and Development, Sandwich Kent, CT13 9NJ (GB); MAGUIRE, Robert Pfizer Global Res. and Development, Sandwich Kent CT13 9NJ (GB)
(74) Representative: Ruddock, Keith Stephen
(86) International application number: PCT/IB2003/005888
(87) International publication number: WO 2004/056750

(56) References cited:
- EP-A- 0 358 398
- WO-A-91/07386
- US-A- 5 362 902

## Description

The invention relates to a series of cyclopentyl substituted glutaramide derivatives and compositions and uses thereof. The derivatives are potent and selective inhibitors of neutral endopeptidase (NEP) and may be used to treat a number of diseases and conditions particularly cardiovascular disorders, especially hypertension.

WO91/08195 discloses a series of 3-styryl β-alanine substituted glutaramide derivatives which have inhibitory activity against NEP.

According to a first aspect, the invention provides a compound of formula (I), a pharmaceutically acceptable salt or solvate thereof 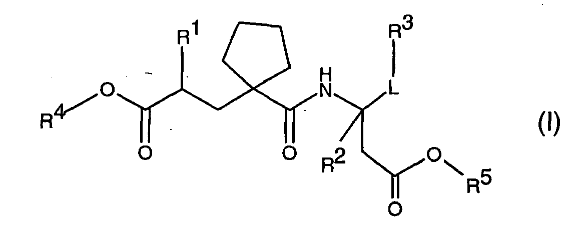 wherein
R¹ is C₁-C₆alkyl, C₁-C₆alkoxyC₁-C₃alkyl or C₁-C₆alkoxyC₁-C₆alkoxyC₁-C₃alkyl;
R² is hydrogen or C₁-C₆alkyl;
L is a three atom linkage selected from -CH₂-X-CH₂- and -CH₂-CH₂-X- where the right hand side of the linkage is attached to R³ and where X is oxygen, sulfur or methylene;
R³ is phenyl or aromatic heterocyclyl, either of which may be independently substituted by one or more groups selected from: C₁-C₆alkyl, halo, haloC₁-C₆alkyl, C₁₋C₆alkoxy, haloC₁-C₆alkoxy, C₁-C₆alkylthio, haloC₁-C₆alkylthio and nitrile; and
R⁴ and R⁵ are either both hydrogen, or one of R⁴ and R⁵ is hydrogen and the other is a biolabile ester-forming group that in the body of a patient is replaced by hydrogen.

Unless otherwise indicated, any alkyl group may be straight or branched and is of 1 to 6 carbon atoms, preferably 1 to 4.

Unless otherwise indicated, any carbocyclyl group contains 3 to 8 ring-atoms, and may be saturated, unsaturated or aromatic. Preferred saturated carbocyclyl groups are cyclopropyl, cyclobutyl, cyclopentyl or cyclohexyl. Preferred unsaturated carbocyclyl groups contain up to 3 double bonds. A preferred aromatic carbocyclyl group is phenyl. The term carbocylic should be similarly construed. In addition, the term carbocyclyl includes any fused combination of carbocyclyl groups, for example naphthyl, phenanthryl, indanyl and indenyl.

Unless otherwise indicated, any heterocyclyl group contains 5 to 7 ring-atoms up to 4 of which may be hetero-atoms such as nitrogen, oxygen and sulfur, and may be saturated, unsaturated or aromatic. Examples of heterocyclyl groups are furyl, thienyl, pyrrolyl, pyrrolinyl, pyrrolidinyl, imidazolyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridyl, piperidinyl, dioxanyl, morpholino, dithianyl, thiomorpholino, pyridazinyl, pyrimidinyl, pyrazinyl, piperazinyl, sulfolanyl, tetrazolyl, triazinyl, azepinyl, oxazepinyl, thiazepinyl, diazepinyl and thiazolinyl. In addition, the term heterocyclyl includes fused heterocyclyl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, dihydroquinazolinyl, benzothiazolyl, phthalimido, benzofuranyl, benzodiazepinyl, indolyl and isoindolyl. The term heterocyclic should be similarly construed.

Halo means fluoro, chloro, bromo or iodo.

Unless otherwise indicate, any haloalkyl, haloalkoxy or haloalkylthio group contains one or more halo atoms which halo atoms may be the same or different.

Preferably R¹ is C₁-C₆alkyl or C₁-C₆alkoxyC₁-C₃alkyl. More preferably R¹ is propyl or methoxyethyl.

Preferably R² is hydrogen.

Preferably L is a three atom linkage selected from -CH₂-O-CH₂-, -CH₂-CH₂-O- and CH₂₋CH₂-CH₂-, where the right hand side of the linkage is attached to R³. More preferably L is -CH₂-CH₂-O- or CH₂-CH₂-CH₂-.

Preferably R³ is phenyl which may be independently substituted by one or more groups selected from: C₁-C₆alkyl, halo, haloC₁-C₆alkyl, C₁-C₆alkoxy, haloC₁-C₆alkoxy, C₁₋C₆alkylthio, haloC₁-C₆alkylthio and nitrile. More preferably R³ is phenyl which may be independently substituted by one or more groups selected from: C₁-C₆ alkyl, halo, haloC₁-C₆alkyl, C₁-C₆alkoxy and halo C₁-C₆alkoxy. More preferably still R³ is 4-fluorophenyl, 4-chlorophenyl, 4-methoxyphenyl or 4-methylphenyl.

The term biolabile ester-forming group is well understood in the art as meaning a group which provides an ester that can be readily cleaved in the body to liberate the corresponding diacid of formula (I) wherein R⁴ and R⁵ are both hydrogen. A number of such ester groups are well known, for example in the penicillin area or in the case of the ACE inhibitor hypertensives such as enalapril and quinapril. Compounds of formula (I) containing such biolabile ester-forming groups are particularly advantageous in providing compounds suitable for oral administration. The suitability of any particular ester-forming group can be assessed by conventional animal or *in vitro* enzyme hydrolysis studies. For optimum effect, the ester should only be hydrolysed after absorption. Accordingly the ester should be resistant to hydrolysis before absorption by digestive enzymes but should be readily hydrolysed by, for example, liver or plasma enzymes. In this way the active diacid is released into the blood stream following oral absorption.

Preferred biolabile ester-forming groups are C₁-C₆alkyl, carbocyclyl or heterocyclyl each of which may be substituted.

More preferred biolabile ester-forming groups are: i) C₁-C₆alkyl optionally substituted by hydroxy, oxo, halo, haloC₁-C₆alkyl, C₁-C₆alkoxy, haloC₁-C₆alkoxy, C₁-C₆alkylthio, haloC₁₋C₆alkylthio, nitrile, carbocyclyl, heterocyclyl, carbocyclyloxy, heterocyclyloxy, C₁-C₇alkylcarbonyloxy, carbocyclylcarbonyloxy, heterocyclylcarbonyloxy, alkylcarbonylamino, and alkylaminocarbonyl, wherein any carbocyclyl or heterocyclyl group is optionally substituted by C₁-C₆alkyl, halo, haloC₁-C₆alkyl, C₁-C₆alkoxy, haloC₁₋C₆alkoxy, C₁-C₆alkylthio, haloC₁-C₆alkylthio or nitrile; or ii) carbocyclyl or heterocyclyl optionally substituted by C₁-C₆alkyl, halo, haloC₁-C₆alkyl, C₁-C₆alkoxy, haloC₁-C₆alkoxy, C₁-C₆alkylthio, haloC₁-C₆alkylthio or nitrile. In the definitions of i) and ii) any carbocyclic group is preferably phenyl and any heterocyclic group is aromatic.

Still more preferred biolabile ester-forming groups are selected from the list: ethyl, propyl, butyl, isobutyl, cyclopentyl, benzyl, 1-(2,2-diethylbutyryloxy)ethyl, 2-ethylpropionyloxymethyl, 1-(2-ethylpropionyloxy)ethyl, 1-(2,4-dimethylbenzoyloxy)ethyl, 1-benzoyloxy)benzyl, 1-(benzoyloxy)ethyl, 2-methyl-1-propionyloxypropyl, 2,4,6-trimethylbenzoyloxymethyl, 1-(2,4,6-trimethylbenzyloxy)ethyl, pivaloyloxymethyl, phenethyl, phenpropyl, 2,2,2-trifluororethyl, 1-naphthyl, 2-naphthyl, 2,4-dimethylphenyl, 4-t-butylphenyl, 5-(4-methyl-1,3-dioxalynyl-2-onyl)methyl, N,N-diethylaminocarbonylmethyl and 5-indanyl.

Preferably R⁴ and R⁵ are both hydrogen.

A preferred compound is of formula (Ia) wherein R¹, R², R³, R⁴, R⁵ and L are as defined in the first aspect.

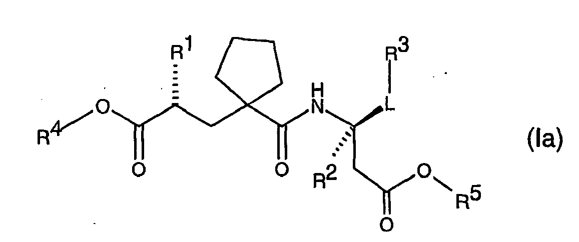

A preferred compound of formula (Ia) is where
- R¹: is C₁-C₆alkyl or C₁-C₆alkoxyC₁-C₃alkyl;
- R²: is hydrogen;
- L: is a three atom linkage selected from: -CH₂-O-CH₂-, -CH₂-CH₂-O- and CH₂-CH₂-CH₂-, where the right hand side of the linkage is attached to R³;
- R³: is phenyl which may be independently substituted by one or more groups selected from: C₁-C₆alkyl, halo, haloC₁-C₆alkyl, C₁-C₆alkoxy, haloC₁-C₆alkoxy, C₁₋C₆alkylthio, haloC₁-C₆alkylthio and nitrile; and
- R⁴ and R⁵: are either both hydrogen, or one of R⁴ and R⁵ is hydrogen and the other is a biolabile ester-forming group that in the body of a patient is replaced by hydrogen.

A more preferred compound of formula (Ia) is where
- R¹: is C₁-C₆alkyl or C₁-C₆alkoxyC₁-C₃alkyl;
- R²: is hydrogen;
- L: is -CH₂-CH₂-O- or CH₂-CH₂-CH₂- where the right hand side of the linkage is attached to R³;
- R³: is phenyl which may be independently substituted by one or more groups selected from: C₁-C₆alkyl, halo, haloC₁-C₆alkyl, C₁-C₆alkoxy and haloC₁-C₆alkoxy; and
- R⁴: and R⁵ are either both hydrogen, or one of R⁴ and R⁵ is hydrogen and the other is a biolabile ester-forming group that in the body of a patient is replaced by hydrogen.

A still more preferred compound of formula (Ia) is where
- R¹: is C₁-C₆alkyl or C₁-C₆alkoxyC₁-C₃alkyl;
- R²: is hydrogen;
- L: is -CH₂-CH₂-O- or CH₂-CH₂-CH₂- where the right hand side of the linkage is attached to R³;
- R³: is phenyl which may be independently substituted by one or more groups selected from: C₁-C₆alkyl, halo, haloC₁-C₆alkyl, C₁-C₆alkoxy and haloC₁-C₆alkoxy; and
- R⁴ and R⁵: are both hydrogen.

A still more preferred compound of formula (Ia) is where
- R¹: is propyl or methoxyethyl;
- R²: is hydrogen;
- L: is -CH₂-CH₂-O- or CH₂-CH₂-CH₂-, where the right hand side of the linkage is attached to R³;
- R³: is 4-fluorophenyl, 4-chlorophenyl, 4-methoxyphenyl or 4-methylphenyl; and
- R⁴ and R⁵: are either both hydrogen, or one of R⁴ and R⁵ is hydrogen and the other is a biolabile ester-forming group that in the body of a patient is replaced by hydrogen.

A still more preferred compound of formula (la) is where
- R¹: is propyl or methoxyethyl;
- R²: is hydrogen;
- L: is -CH₂-CH₂-O- or CH₂-CH₂-CH₂-, where the right hand side of the linkage is attached to R³;
- R³: is 4-fluorophenyl, 4-chlorophenyl, 4-methoxyphenyl or 4-methylphenyl; and
- R⁴ and R⁵: are either both hydrogen, or one of R⁴ and R⁵ is hydrogen and the other is a biolabile ester-forming group that in the body of a patient is replaced by hydrogen, selected from: i) C₁-C₆alkyl optionally substituted by hydroxy, oxo, halo, haloC₁-C₆alkyl, C₁-C₆alkoxy, haloC₁-C₆alkoxy, C₁-C₆alkylthio, haloC₁₋C₆alkylthio, nitrile, carbocyclyl, heterocyclyl, carbocyclyloxy, heterocyclyloxy, alkylcarbonyloxy, carbocyclylcarbonyloxy, heterocyclylcarbonyloxy, alkylcarbonylamino, and alkylaminocarbonyl, wherein any carbocyclyl or heterocyclyl group is optionally substituted by C₁-C₆alkyl, halo, haloC₁-C₆alkyl, C₁₋C₆alkoxy, haloC₁-C₆alkoxy, C₁-C₆alkylthio, haloC₁-C₆alkylthio or nitrile; or ii) carbocyclyl or heterocyclyl optionally substituted by C₁-C₆alkyl, halo, haloC₁₋C₆alkyl, C₁-C₆alkoxy, haloC₁-C₆alkoxy, C₁-C₆alkylthio, haloC₁-C₆alkylthio or nitrile.

A most preferred compound of formula (la) is where
- R¹: is propyl or methoxyethyl;
- R²: is hydrogen;
- L: is -CH₂-CH₂-O- or CH₂-CH₂-CH₂-, where the right hand side of the linkage is attached to R³;
- R³: is 4-fluorophenyl, 4-chlorophenyl, 4-methoxyphenyl or 4-methylphenyl; and
- R⁴ and R⁵: are both hydrogen.

A particularly preferred compound of formula (I) is selected from:
(2*S*)-2-[(1-{[((1*R*)-2-carboxy-1-{[(4-chlorobenzyl)oxy]methyl}ethyl)-amino]carbonyl}cyclopentyl)methyl]-4-methoxybutanoic acid (Example 16);
(3*S*)-3-[({1-[(2*R*)-2-carboxypentyl]cyclopentyl}carbonyl)amino]-5-(4-chlorophenoxy)pentanoic acid (Example 41);
Ethyl (3*S*)-3-[({1-[(2*S*)-2-Carboxy-4-methoxybutyl]cyclopentyl}carbonyl)amino]-5-(4-chlorophenoxy)pentanoate (Example 47);
(3*S*)-3-[({1-[(2*S*)-2-carboxy-4-methoxybutyl]cyclopentyl}carbonyl)amino]-5-(4-chlorophenoxy)pentanoic acid (Example 48);
(2*S*)-2-({1-[({(1*S*)-3-butoxy-1-[2-(4-chlorophenoxy)ethyl]-3-oxopropyl}amino)carbonyl]cyclopentyl}methyl)-4-methoxybutanoic acid (Example 49);
(3*S*)-3-[({1-[(2*S*)-2-carboxy-4-methoxybutyl]cyclopentyl}carbonyl)amino]-6-(4-chlorophenyl)hexanoic acid (Example 53);
Ethyl (3*S*)-3-[({1-[(2*S*)-2-carboxy-4-methoxybutyl]cyclopentyl}carbonyl)amino]-6-(4-chlorophenyl)hexanoate (Example 52); and
(3*S*)-3-[({1-[(2*S*)-2-carboxy-4-methoxybutyl]cyclopentyl}carbonyl)amino]-6-(4-methoxyphenyl)hexanoic acid (Example 54).

For the avoidance of doubt, unless otherwise indicated, the term substituted means substituted by one or more defined groups. In the case where groups may be selected from a number of alternative groups, the selected groups may be the same or different.

For the avoidance of doubt, the term independently means that where more than one substituent is selected from a number of possible substituents, those substituents may be the same or different.

The pharmaceutically or veterinarily acceptable salts of the compounds of the invention which contain a basic centre are, for example, non-toxic acid addition salts formed with inorganic acids such as hydrochloric, hydrobromic, hydroiodic, sulfuric and phosphoric acid, with carboxylic acids or with organo-sulfonic acids. Examples include the HCl, HBr, HI, sulfate or bisulfate, nitrate, phosphate or hydrogen phosphate, acetate, benzoate, succinate, saccharate, fumarate, maleate, lactate, citrate, tartrate, gluconate, camsylate, methanesulfonate, ethanesulfonate, benzenesulfonate, p-toluenesulfonate and pamoate salts. Compounds of the invention can also provide pharmaceutically or veterinarily acceptable metal salts, in particular non-toxic alkali and alkaline earth metal salts, with bases. Examples include the sodium, potassium, aluminium, calcium, magnesium, zinc and diethanolamine salts. For reviews on suitable pharmaceutical salts see Berge *et al*, J. Pharm, Sci., 66, 1-19, 1977; P L Gould, International Journal of Pharmaceutics, 33 (1986), 201-217; and Bighley *et al*, Encyclopedia of Pharmaceutical Technology, Marcel Dekker Inc, New York 1996, Volume 13, page 453-497.

The pharmaceutically acceptable solvates of the compounds of the invention include the hydrates thereof.

Also included within the scope of the invention and various salts of the invention are polymorphs thereof.

Hereinafter, compounds their pharmaceutically acceptable salts, their solvates or polymorphs, defined in any aspect of the invention (except intermediate compounds in chemical processes) are referred to as "compounds of the invention".

The compounds of the invention may possess one or more chiral centres and so exist in a number of stereoisomeric forms. All stereoisomers and mixtures thereof are included in the scope of the present invention. Racemic compounds may either be separated using preparative HPLC and a column with a chiral stationary phase or resolved to yield individual enantiomers utilising methods known to those skilled in the art. In addition, chiral intermediate compounds may be resolved and used to prepare chiral compounds of the invention.

The compounds of the invention may exist in one or more tautomeric forms. All tautomers and mixtures thereof are included in the scope of the present invention. For example, a claim to 2-hydroxypyridinyl would also cover its tautomeric form, α-pyridonyl.

The invention also includes all suitable isotopic variations of a compound of the invention. An isotopic variation of a compound of the invention is defined as one in which at least one atom is replaced by an atom having the same atomic number but an atomic mass different from the atomic mass usually found in nature. Examples of isotopes that can be incorporated into compounds of the invention include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the invention, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. Isotopic variations of the compounds of the invention can generally be prepared by conventional procedures such as by the illustrative methods or by the preparations described in the Examples and Preparations hereafter using appropriate isotopic variations of suitable reagents.

The compounds of the invention, by inhibiting NEP (particularly EC.3.4.24.11), can potentiate the biological effects of bioactive peptides and thus there is rationale for the compounds of the invention to treat or prevent number of diseases and conditions.

Shepperson *et al* have demonstrated that the NEP inhibitor candoxatrilat lowers systolic blood pressure in hypertensive rats [see Clin.Sci (Lond), Vol 80(3):265-9]. Kosoglou *et al* have demonstrated that the NEP inhibitor SCH34826 significantly lowered supine blood pressure in a clinical study of 24 black patients with essential hypertension [Circulation, Supplement III, Vol 82, No. 4, page 554, 2201]. Stergiou *et al* have demonstrated that when added to the ACE inhibitor lisinopril, candoxatril (the oral prodrug of candoxatrilat) led to a marked reduction in both supine and erect blood pressure in a clinical study involving 37 hypertensive patients [J. Hypertens, Vol 12, No 11, page 1310-1311]. Accordingly, the compounds of the invention should treat or prevent cardiovascular diseases and conditions, particularly hypertension, pulmonary hypertension, peripheral vascular disease, heart failure, angina, renal insufficiency, acute renal failure, cyclical oedema, Menières disease, hyperaldosteroneism (primary and secondary) and hypercalciuria. The term hypertension includes all diseases characterised by supranormal blood pressure, such as essential hypertension, pulmonary hypertension, secondary hypertension, isolated systolic hypertension, hypertension associated with diabetes, hypertension associated with atherosclerosis, and renovascular hypertension, and further extends to conditions for which elevated blood pressure is a known risk factor. Accordingly, the term "treatment of hypertension" includes the treatment or prevention of complications arising from hypertension, and other associated co-morbidities, including congestive heart failure, angina, stroke, glaucoma, impaired renal function, including renal failure, obesity, and metabolic diseases (including Metabolic Syndrome). Metabolic diseases include in particular diabetes and impaired glucose tolerance, including complications thereof, such as diabetic retinopathy and diabetic neuropathy.

The compounds of the invention should be particularly efficacious in treating or preventing hypertension.

Wayman *et al* [WO 02/079143] have demonstrated that NEP inhibitors increase vaginal and clitoral blood flow in a rabbit model of female sexual arousal disorder (FSAD). Accordingly the compounds of the invention should treat or prevent female sexual dysfunction, particularly FSAD.

Wayman *et al* (WO 02/03995) have demonstrated that NEP inhibitors potentiate nerve-stimulated erections in anaesthetised dog model of penile erections. Accordingly the compounds of the invention should treat or prevent male erectile dysfunction (MED).

As a result of their ability to inhibit NEP, the compounds of the invention should treat or prevent menstrual disorders, pre-term labour, pre-eclampsia, endometriosis, and reproductive disorders (especially male and female infertility, polycystic ovarian syndrome, implantation failure).

In addition, the compounds of the invention should: treat or prevent asthma, inflammation, leukemia, pain, epilepsy, affective disorders, dementia and geriatric confusion, septic shock, obesity and gastrointestinal disorders (especially diarrhoea and irritable bowel syndrome); promote wound healing (especially diabetic and venous ulcers and pressure sores); modulate gastric acid secretion; and treat hyperreninaemia, cystic fibrosis, restenosis, diabetic complications and atherosclerosis.

As used herein, the terms "treat", "treating" and "treatment" include palliative, curative and prophylactic treatment.

Compounds of the invention may be prepared, in known manner in a variety of ways. In the following reaction schemes and hereafter, unless otherwise stated, R¹ to R⁵, L and X are as defined in the first aspect. These processes form further aspects of the invention.

Throughout the specification, general formulae are designated by Roman numerals I, II, III, IV etc. Subsets of these general formulae are defined as Ia, Ib, Ic etc, .... IVa, IVb, IVc etc.

Compounds of formula (Ib), i.e. compounds of general formula (I) where R⁴ and R⁵ are hydrogen and L is -CH₂OCH₂-, may be prepared according to reaction scheme 1, by deprotecting a compound of general formula (II) where P¹ is a suitable carboxyl protecting group, such as C₁-C₄ alkyl or benzyl, preferably ethyl or t-butyl.

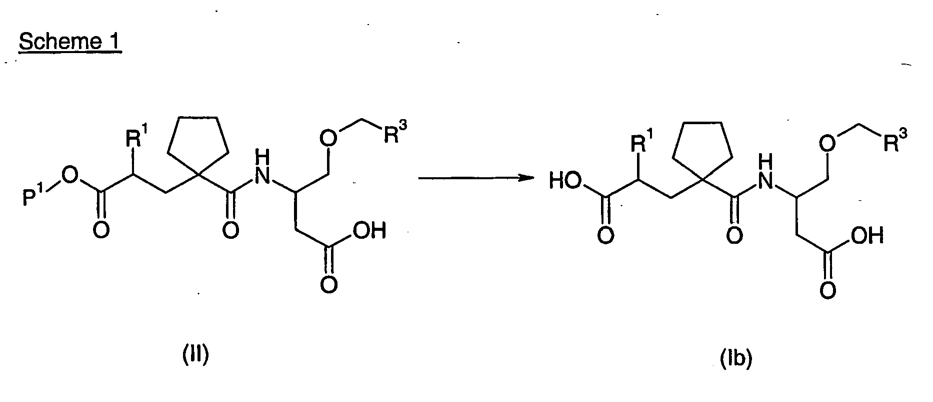

Deprotection can be performed using standard methodology, as described in "Protecting Groups in Organic Synthesis" by T.W. Greene and P. Wutz". When P¹ is t-butyl, then the preferred conditions are 9-40% trifluoroacetic acid in dichloromethane (by volume) at room temperature for 1 to 72 hours. When P¹ is allyl, preferred conditions are pyrrolidine (4 equiv), tetrakistriphenylphosphine paladium (0) (catalytic) in tetrahydrofuran at room temperature for 2-3 hours. When P¹ is ethyl, preferred conditions are 1N sodium hydroxide in dioxan, methanol or tetrahydrofuran at a temperature between room temperature and reflux for 2-18 hours.

Compounds of formula (II) may be prepared by reacting compounds of formula (III) with R³CH₂Y (where Y is a halogen atom, preferably bromine or iodine).

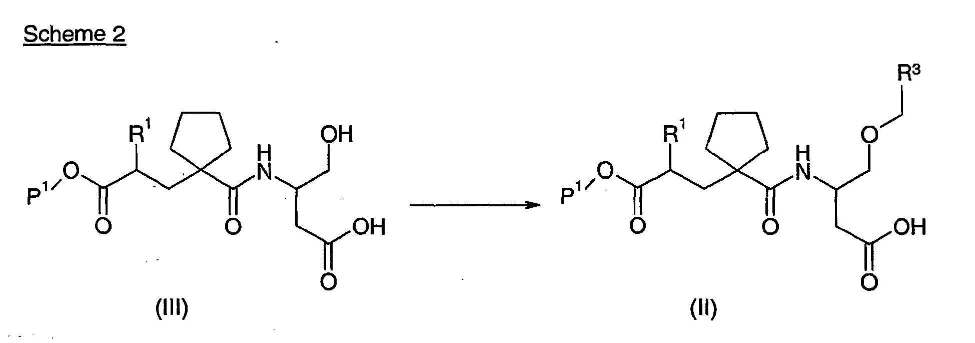

Preferred conditions comprise reacting (III) with R³CH₂Y in the presence of a suitable alkali metal base (for example sodium hydride, potassium carbonate or caesium carbonate) in a suitable solvent (for example tetrahydrofuran or N,N-dimethylformamide), optionally in the presence of a catalyst (for example imidazole or 4-dimethylaminopyridine). Particularly preferred conditions are sodium hydride (6-10 equiv), imidazole (0.2 equiv) and R³CH₂Y (1.1 equiv) in tetrahydrofuran at between -15°C and room temperature for 2-3 hrs.

Compounds of formula (III) may be prepared from compounds of formula (IV) according to reaction scheme 3.

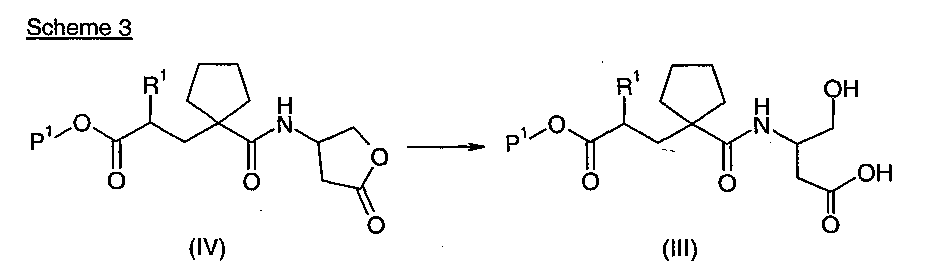

Preferred reaction conditions comprise reacting compounds of formula (IV) under acid conditions (for example hydrochloric acid) or basic conditions (for example sodium hydroxide or caesium hydroxide) in the presence of a catalyst in a suitable solvent (for example methanol or dioxan) at a temperature of between room temperature and the reflux temperature of the reaction mixture. Preferred conditions are 1N sodium hydroxide:methanol (25:75, by volume) at room temperature for 18 hours.

Compounds of formula (IV) may be prepared by reacting compounds of formula (V) with compounds of formula (VI) according to reaction scheme 4.

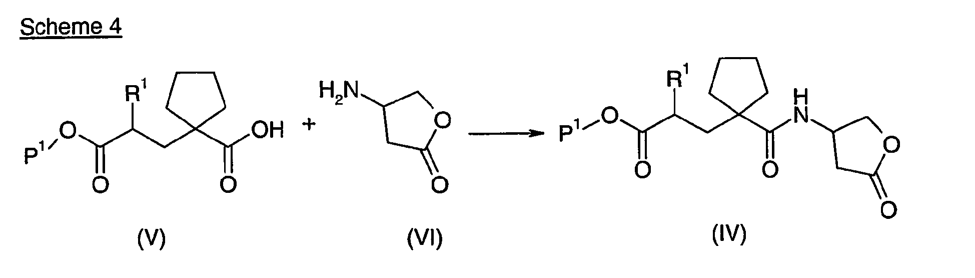

Typical reaction conditions comprise generating the acid chloride of compounds of formula (V) followed by addition of compounds of formula (VI), optionally in the presence of an excess of tertiary amine (such as triethylamine, Hünig's base or N-methylmorpholine) in a suitable solvent (such as dichloromethane or tetrahydrofuran) at room temperature for between 1 to 24 hours.

Alternative reaction conditions comprise reacting compounds of formula (V), 1-(3-dimethylaminopropyl)-3-ethylcarbodiimide hydrochloride (WSCDI) /1,3-dicyclohexylcarbodiimide (DCC), 1-hydroxybenzotriazole hydrate (HOBT) /HOAT and compounds of formula (VI), with an excess of a tertiary amine (such as N-methylmorpholine, triethylamine or Hünig's base) in a suitable solvent (for example tetrahydrofuran, dichloromethane or ethyl acetate) at room temperature for between 4 to 48 hours.

Further reaction conditions comprise reacting a compound of formula (V), PYBOP® /PyBrOP® /Mukaiyama's reagent and an excess of compound of formula (VI), with an excess of tertiary amine (N-methylmorpholine, triethylamine or Hünig's base) in a suitable solvent (such as tetrahydrofuran, dichloromethane or ethyl acetate) at room temperature for 4 to 24 hours.

Preferred reaction conditions comprise reacting compounds of formula (VI) (1 equiv), a compound of formula (V) (1-1.1 equiv), WSCDI (1-1.1 eq), 1-hydroxybenzotriazole hydrate (HOBT) (1-1.1 eq) and N-methylmorpholine (2 equiv) in dichloromethane at room temperature for about 18 hours.

Compounds of formula (V) may be prepared by published methods, or by simple modifications of these methods. See for example: A.S. Cook *et al*., European Patent Application EP 1 258 474 A2 (Pfizer Ltd. *et al.);* S. Challenger *et al.,* International Patent Application WO02/79143 (Pfizer Ltd. *et al*.); C.G. Barber *et al*., International Patent Application WO02/02513 (Pfizer Ltd. *et al*.); C.J. Cobley et al., Tetrahedron Letts. 42(42), 7481-7483 (2001); S. Challenger, European Patent Application 0 644 176 A1 (Pfizer Ltd.).

Compounds of formula (Ic), i.e. compounds of general formula (I) where R⁵ is hydrogen, R⁴ is nor hydrogen and L is -CH₂OCH₂-, may be prepared from compounds of formula (II) according to reaction scheme 5.

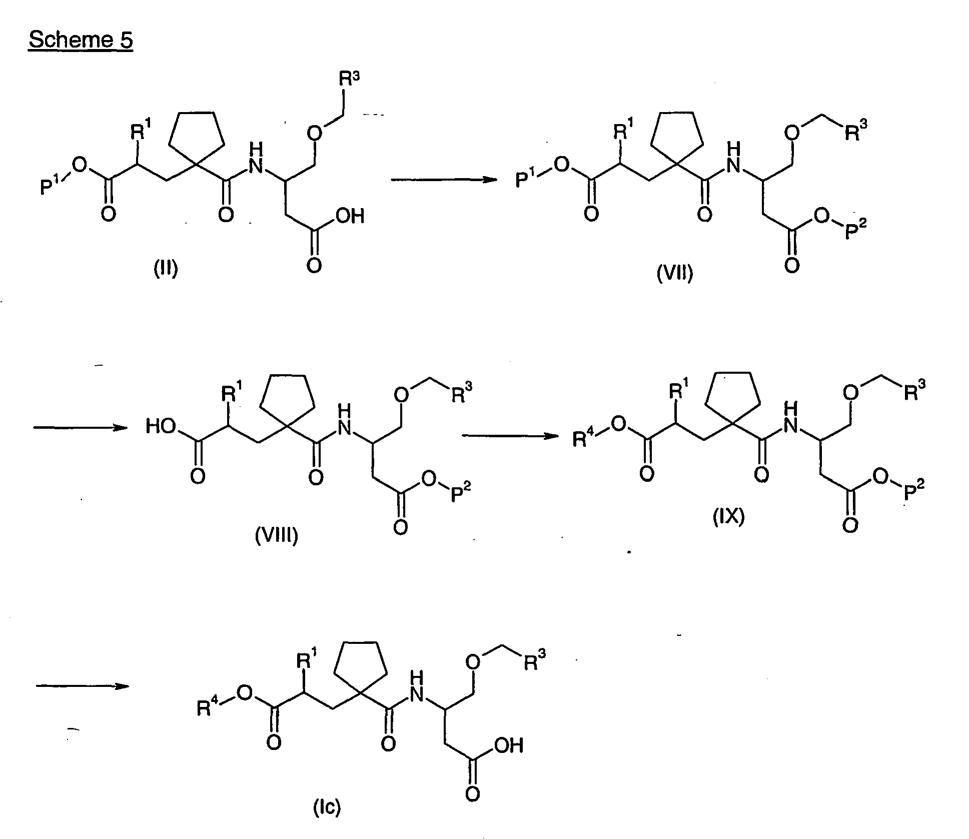

Compounds of formula (VII) may be prepared by reacting a compound of formula (II) (where P¹ is a protecting group removable under acidic conditions, such as t-butyl) with P²Y¹ (where P² is an acid stable protecting group, such as benzyl or allyl, and Y¹ is a leaving group, typically halogen, preferably bromine or iodine), with an excess of an alkali metal base (for example potassium carbonate or caesium carbonate), or a tertiary amine base (for example triethylamine) in a suitable solvent (for example N,N-dimethylformamide or acetonitrile) at room temperature. Preferred reaction conditions comprise reacting a compound of formula (II) with of P²Y¹ (2.3 equiv) and potassium carbonate (1-3 equiv) in N,N-dimethylformamide for 18 to 72 hours.

Compounds of formula (VIII) may be prepared from compounds of formula (VII). When P¹ is t-butyl, then the preferred conditions are 9-40% trifluoroacetic acid in dichloromethane (by volume) at room temperature for 1 to 72 hours.

Compounds of formula (IX) may be prepared from compounds of formula (VIII) using analogous methods described for the preparation of compounds of formula (VII) replacing P²Y¹ with R⁴Y¹.

Compounds of formula (Ic) may be prepared from compounds of formula (IX). When P² is benzyl, preferred conditions comprise hydrogenation. When P² is allyl preferred conditions comprise pyrrolidine, tetrakistriphenylphosphine paladium (0) (catalytic) in tetrahydrofuran at room temperature for 2-3 hours.

Compounds of formula (Id), i.e. compounds of general formula (I) where R⁴ is hydrogen, R⁵ is not hydrogen and L is -CH₂OCH₂-, may be prepared according to reaction scheme 6, where P¹ is a suitable carboxyl protecting group, preferably t-butyl.

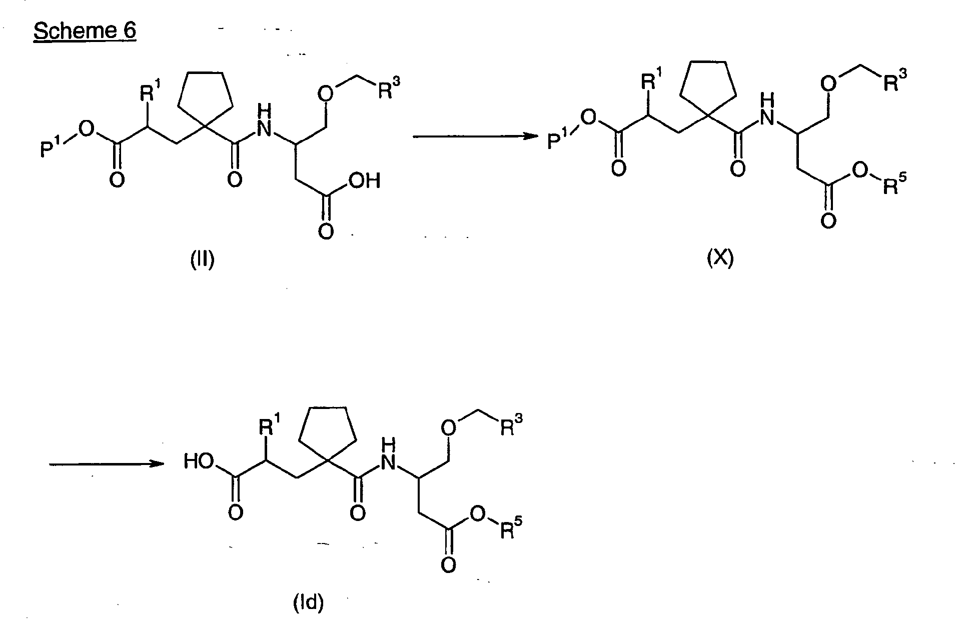

Compounds of formula (X) where P¹ is a suitable carbonyl protecting group preferably t-butyl, may be prepared from compounds of formula (II) in analogous fashion to the preparation of compounds of formula (IX) (see scheme 5).

Compounds of formula (Id) may be prepared from compounds of formula (X) in analogous fashion to scheme 1. For example when P¹ is t-butyl preferred reaction conditions comprise trifluoroacetic acid or hydrochloric acid.

Alternatively compounds of formula VII may be prepared according to reaction scheme 7.

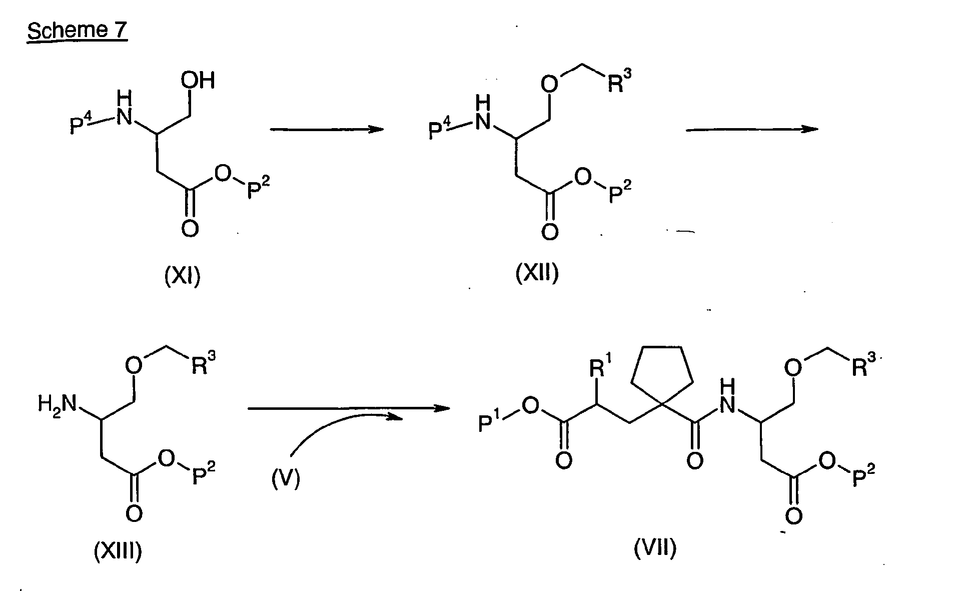

Compounds of formula (XII) may be prepared by treating a compound of formula (XI) with R³CH₂Hal in analogous fashion the methods described for Scheme 2 where P⁴ is a suitable nitrogen protecting group, such as tert-butyloxycarbonyl (Boc) or benzyloxycarbonyl (CBz), preferably Boc. The amine protecting group P⁴ in compounds of formula (XII) may then be selectively removed using standard methodology, as described in "Protecting Groups in Organic Synthesis" by T.W. Greene and P. Wutz". Preferred conditions, when P⁴ is Boc are hydrochloric acid in ethyl acetate, ethanol or dichloromethane or, trifluoroacetic acid in dichloromethane at room temperature for between 15 minutes and 3 hours. Preferred conditions when P⁴ is CBz are hydrobromic acid in acetic acid at room temperature for about 5 hrs. Compounds of formula (VII) may then be obtained by reaction compounds of formula (XIII) with compounds of formula (V) in analogous fashion to the methods described for Scheme 4.

Compounds of formula (Id), i.e. compounds of general formula (I) where R⁴ and R⁵ are hydrogen and L is -CH₂CH₂O-, may be prepared according to reaction scheme 8. 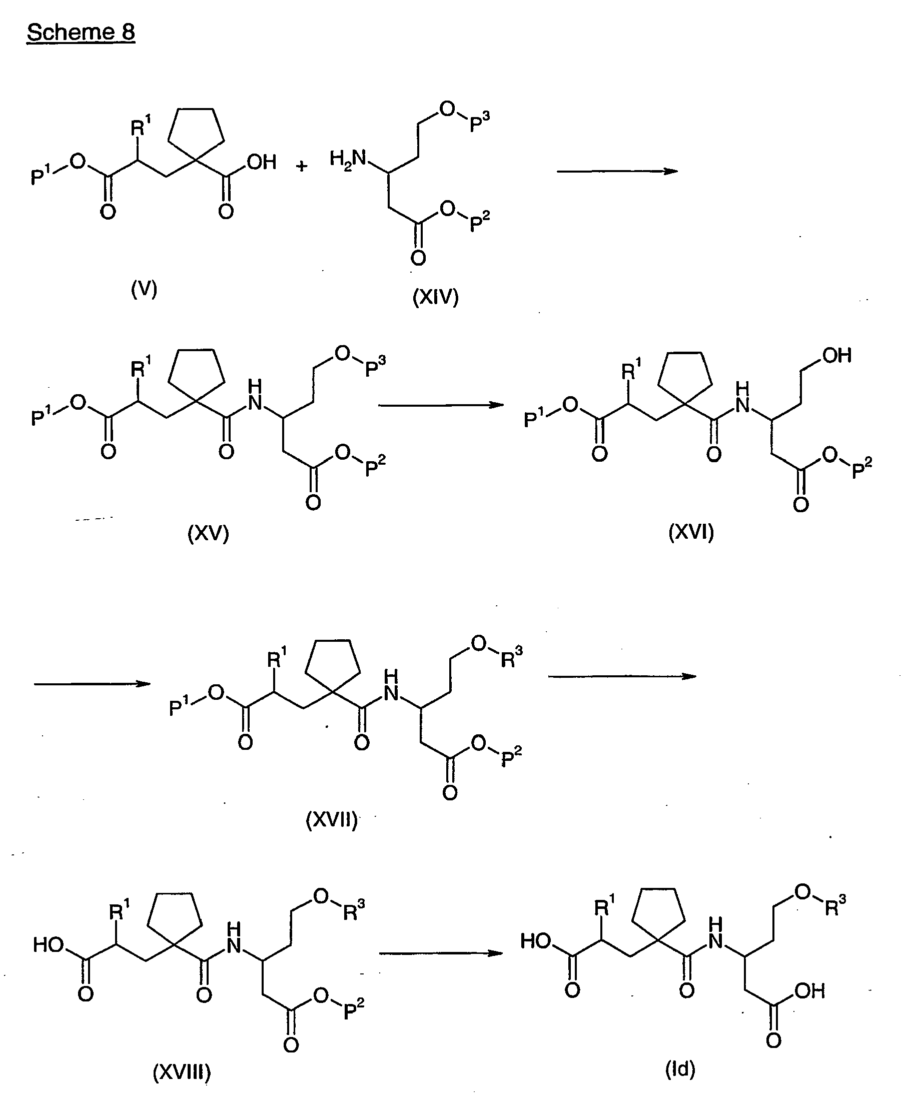 Compounds of formula (XV) may be prepared by reacting a compound of formula (V) with a compound of formula (XIV) using analogous conditions to those described for Scheme 4, where P¹ and P² are suitable carboxyl protecting groups, typically C₁-C₄ alkyl or benzyl, preferably ethyl or t-butyl, and P³ is a suitable hydroxyl-protecting group, typically silyl. Compounds of formula (XVI) may be prepared by removal of the alcohol protecting group using standard methodology, as described in "Protecting Groups in Organic Synthesis" by T.W. Greene and P. Wutz". When P³ is a silyl group, such as t-butyldimethylsilyl, preferred conditions are acetic acid:tetrahydrofuran:water (50:25:25) at room temperature for 2 hours. Compounds of formula (XVII) may be prepared by reacting (XVI) with R³OH using Mitsunobu conditions described in Synthesis 1 (1981) or Org. React. 42; 335 (1992). Preferred reaction conditions comprise diisopropylazadicarboxylate (DIAD) (1.3 equiv), triphenylphosphine (1.3 equiv), R³OH (1.3 equiv) in tetrahydrofuran for 18 hours at room temperature. Compounds of formula (Id) may be prepared in two steps from (XVII) by deprotection methodology described previously in Scheme 1.

Compounds of formula (Id) where X is S, may be prepared by reaction of (XVI) with R³SH, under the Mitsunobu conditions described for the preparation of (XVII), followed by removal of the carboxyl protecting groups, as described in step for scheme 1.

Compounds of formula (XIV) may be prepared according to reaction scheme 9 by reacting compounds of formula (XIX) where P² is a suitable carboxyl protecting group, typically C₁-C₄alkyl or benzyl, preferably ethyl or t-butyl, and P³ is a suitable hydroxyl protecting group, typically silyl, with a suitable chiral amine according to the methods described by Davies et. al. (Tet. Asymm. 1991; 2; 183).

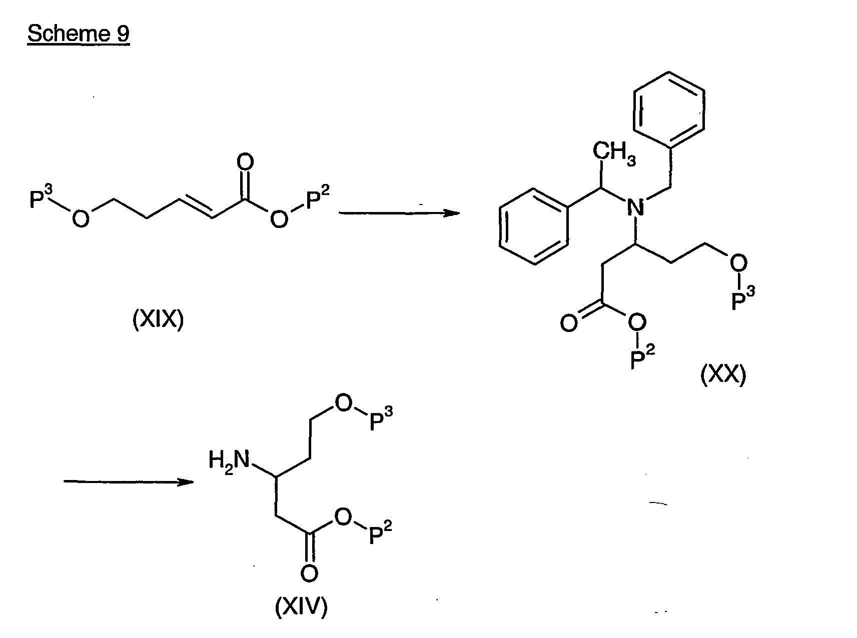

Preferred conditions comprise n-butyl lithium (1.5 equiv) and the chiral auxiliary NH(benzyl)(α-methylbenzyl) (1.6 equiv) in tetrahydrofuran at -78°C for about 3 hours. The N-benzyl protecting groups may be removed by reacting (XX) in the presence of a suitable palladium catalyst (such as palladium on charcoal or palladium hydroxide) in a suitable solvent (such as acetic acid or ethanol). Preferred conditions are 10% palladium on charcoal in acetic acid at room temperature for up to 72 hours.

Compounds of formula (Ie), i.e. compounds of general formula (I) where R⁴ is hydrogen, R⁵ is not hydrogen and L is -CH₂CH₂O- may be prepared from compounds of formula (XVII) according to reaction scheme 10, wherein P¹ is a suitable carboxyl protecting group, typically C₁-C₄alkyl or benzyl, preferably, t-butyl.

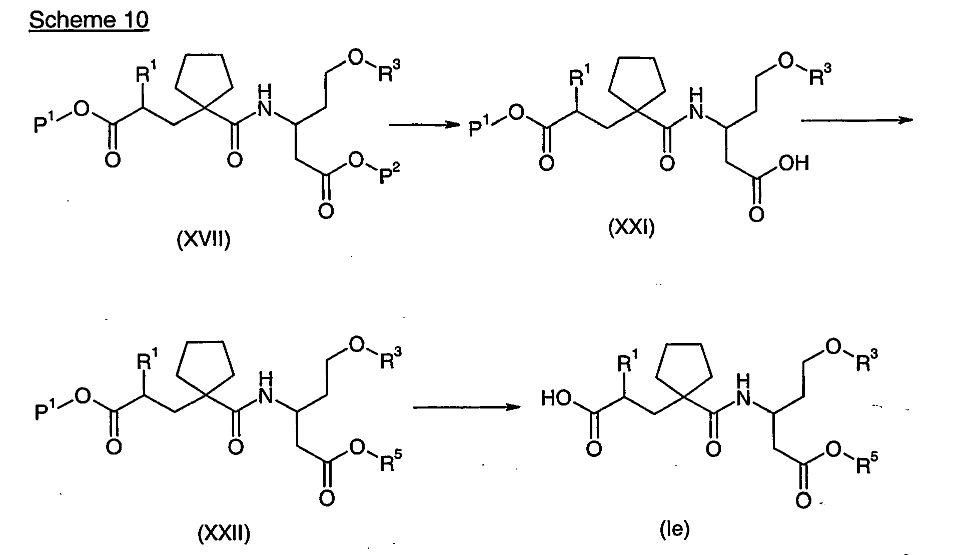

Compounds of formula (XXI) may be prepared by removal of the protecting group P² from compounds of formula (XVII) using analogous methods to those described for Scheme 5 and 6. Compounds of formula (XXII) may be prepared in turn by reaction of compounds of formula (XXI) with R⁵Hal using analogous methods described for the preparation of compounds of formula (VII). Compounds of formula (Ie) may be prepared from compounds of formula (XXII) by deprotection methods as described for Scheme 6.

Compounds of formula (If), i.e. compounds of general formula (I) wherein L is -CH₂CH₂CH₂-, and R⁴ and R⁵ are both hydrogen may be prepared according to reaction scheme 11 where P¹ and P² are suitable carboxyl protecting groups, typically C₁-C₄ alkyl or benzyl, preferably t-butyl. P⁴ is a suitable amino protecting group, preferably benzyloxycarbonyl.

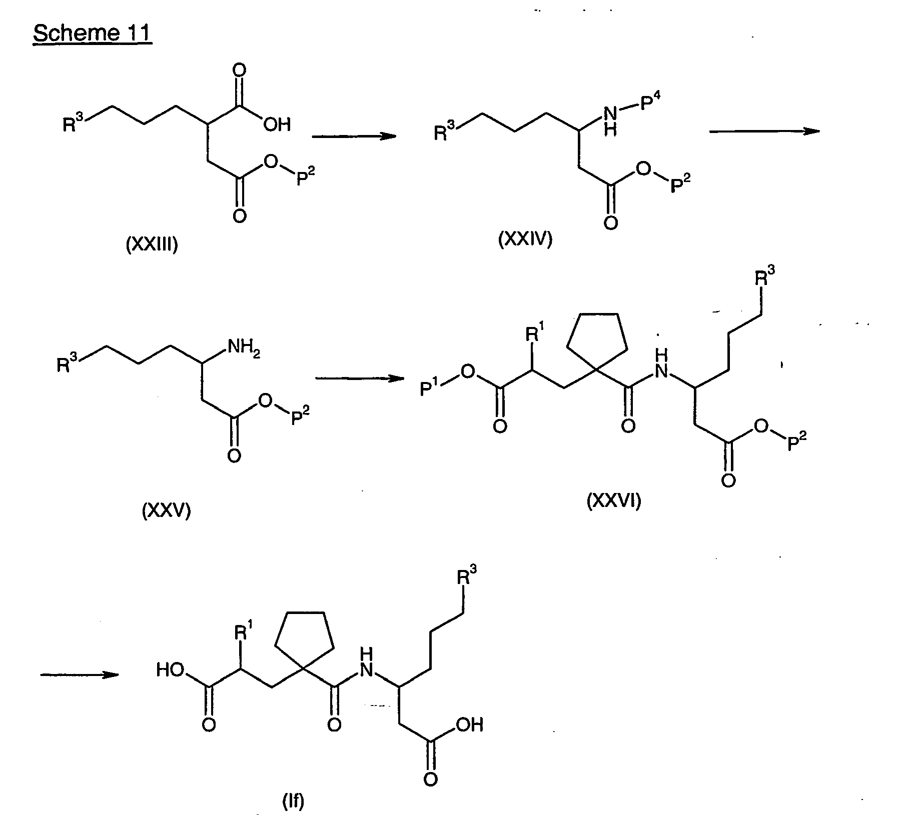

Compounds of formula (XXIV) may be prepared from compounds of formula (XXIII) under the conditions described by Evans et. al (J.Org.Chem. 1999; 64; 6411) or Talley (EP 0561758). Compounds of formula (XXIII) may be prepared in analogous fashion to the methods described by Beeley et. al. in WO 9504033. Preferred conditions for the preparation of compounds of formula (XXIV) are diphenylphosphoryl azide (1 equiv), triethylamine (1.2 equiv) in toluene at reflux for 2 to 3.5 hours, followed by addition of benzyl alcohol (3 equiv) at a temperature between room temperature and reflux for about 18 hours. Compounds of formula (XXV) may be obtained from compounds of formula (XXIV) by removal of protecting group P⁴ using methods analogous to those described for the preparation of compounds of formula (XIII) (see scheme 7). Compounds of formula (XXVI) may be prepared by coupling compounds of formula (XXV) with the compounds of formula (V) using analogous methods to those described for Scheme 4. Removal of the protecting groups from compounds of formula (XXVI) using analogous methods to those described for Scheme 1 gave compounds of formula (If).

Compounds of formula (Ig), i.e. compounds of general formula (I) where L is -CH₂CH₂CH₂-, R⁴ is hydrogen and R⁵ is not hydrogen may be prepared according to reaction scheme 12 where P¹ and P² are suitable carboxyl protecting groups, typically C₁-C₄ alkyl or benzyl, preferably t-butyl, and P⁴ is a suitable amino protecting group, preferably CBz.

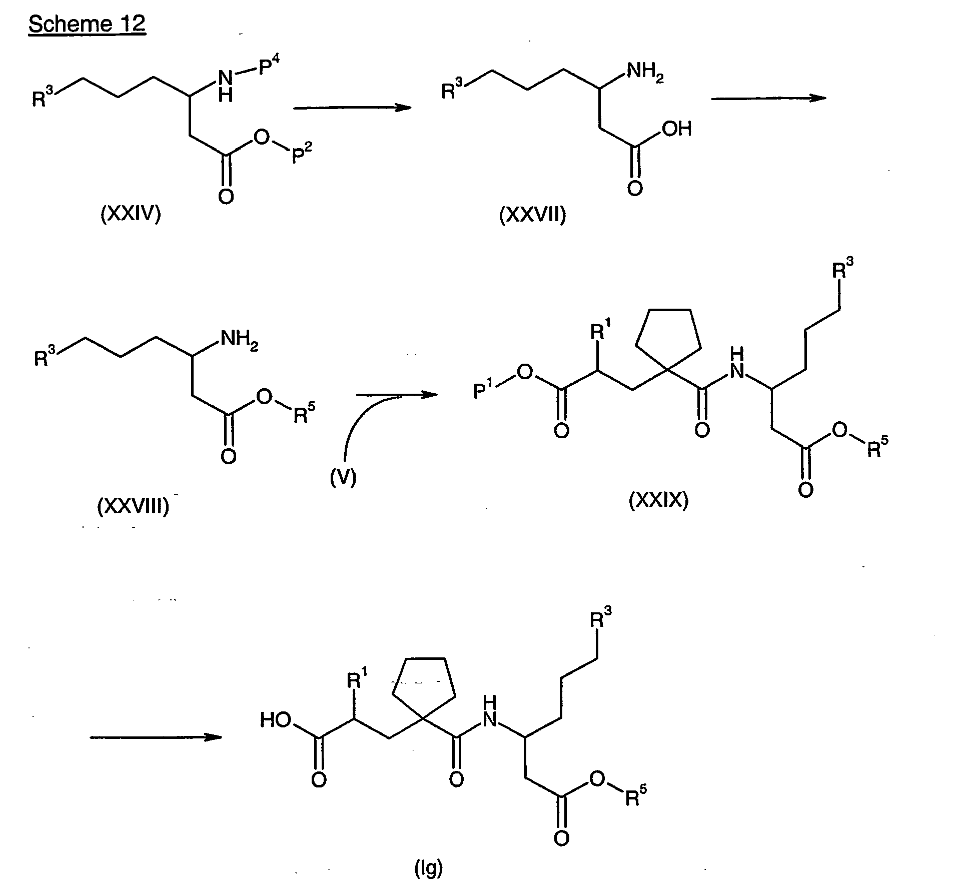

Compounds of formula (XXVII) may be prepared by deprotecting compounds of formula (XXIV). When P² is t-butyl and P⁴ is benzyloxycarbonyl, preferred conditions are hydrobromic acid in acetic acid at room temperature for about 5 hours. Compounds of formula (XXVIII) may be obtained by reacting compounds of formula (XXVII) with R⁵OH under acid catalysis. Compounds of formula (XXIX) may be prepared by coupling the compounds of formula (XXVIII) with compounds of formula (V) using methods analogous to those described for Scheme 4. Removal of the protecting groups from compounds of formula (XXIX) using methods analogous to those described for Scheme 6 gave compounds of formula (Ig).

It will be appreciated by those skilled in the art, that certain compounds of formula (I) where R⁴ or R⁵ are not hydrogen may be converted to compounds of formula (I) where R⁴ or R⁵ are hydrogen, using standard ester hydrolysis conditions analogous to those described for Scheme 1.

In addition, it will be appreciated by those skilled in the art, that certain compounds of formula (I) where R⁴ or R⁵are hydrogen, may be converted to compounds of formula (I) where R⁴ or R⁵ are not hydrogen using standard esterification conditions analogous to those described for the preparation of compounds of formula (VII) in scheme 5.

Alternatively it will be appreciated by those skilled in the art, that certain compounds of formula (I) where R⁴ or R⁵ are not hydrogen, may be converted to compounds of formula (I) with alternative R⁴ or R⁵ groups, using standard transesterification conditions.

A pharmaceutically acceptable salt of a compound of the formula (I) may be readily prepared by mixing together solutions of a compound of the formula (I) and the desired acid or base, as appropriate. The salt may precipitate from solution and be collected by filtration or may be recovered by evaporation of the solvent.

Procedures for isolating the desired products will be well-known to those skilled in the art with reference to literature precedents and the Examples and Preparations hereto.

The compounds of the invention can be administered alone but will generally be administered in admixture with a suitable pharmaceutical excipient, diluent or carrier selected with regard to the intended route of administration and standard pharmaceutical practice.

For example, the compounds of the invention can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations of the compounds of the invention may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain between about 0.01 mg and 500mg of active compound whilst tablet fill weights may range from 50mg to 1000mg. An example of a formulation for a 10mg tablet is illustrated below:

| Ingredient | %w/w |
|---|---|
| Free acid, Free base or Salt form | 10.000* |
| Lactose | 64.125 |
| Starch | 21.375 |
| Croscarmellose Sodium | 3.000 |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating.

Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is insoluble a fast dispersing dosage form can be prepared and where the drug substance is soluble a fast dissolving dosage form can be prepared.

The compounds of the invention can also be administered parenterally, for example, intracavernouslly, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The following dosage levels and other dosage levels herein are for the average human subject having a weight range of about 65 to 70 kg. The skilled person will readily be able to determine the dosage levels required for a subject whose weight falls outside this range, such as children and the elderly.

For oral and parenteral administration to human patients, the daily dosage level of the compounds of the invention will usually be from 0.01 mg/kg to 10 mg/kg (in single or divided doses).

Thus tablets or capsules of the compound of the invention may contain from 1 mg to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The compounds of the invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or "puff" contains from 1 to 50 mg of a compound of the invention for delivery to the patient. The overall daily dose with an aerosol will be in the range of from 1 to 50 mg which may be administered in a single dose or, more usually, in divided doses throughout the day.

Alternatively, the compounds of the invention can be administered in the form of a suppository or pessary, or they may be applied topically in the form of a gel, hydrogel, lotion, solution, cream, ointment or dusting powder. The compounds of the invention may also be dermally or transdermally administered, for example, by the use of a skin patch. They may also be administered by the pulmonary, vaginal or rectal routes.

They may also be administered by the ocular route, particularly for treating disorders of the eye. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

For treating cardiovascular disorders, particular hypertension, the compounds of the invention may be combined with one or more active ingredient selected from the list:
a) angiotensin receptor blockers (ARB), such as losartan, valsartan, telmisartan, candesartan, irbesartan, eprosartan and olmesartan;
b) calcium channel blockers (CCB) such as amlodipine;
c) statins, such as atorvastatin;
d) PDE5 inhibitors, such as sildenafil, tadalafil, vardenafil, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and; the pyrazolo[4,3-d]pyrimidin-4-ones disclosed in WO00/27848 particularly N-[[3-(4,7-dihydro-1-methyl-7-oxo-3-propyl-1H-pyrazolo[4,3-d]-pyrimidin-5-yl)-4-propxyphenyl]sulfonyl]-1-methyl2-pyrrolidinepropanamide [DA-8159 (Example 68 of WO00/27848)];
e) beta blockers, such as atenolol or carvedilol;
f) ACE inhibitors, such as quinapril, enalapril and lisinopril;
g) alpha-blockers such as doxazosin;
h) selective aldosterone receptor antagonists (SARA), such as eplerenone or spironolactone;
i) imidazoline I₁ agonists, such as rilmenidine or monoxidine; and
j) endothelin receptor antagonists and endothelin converting enzyme inhibitors. For treating FSAD, the compounds of the invention may be combined with one or more active ingredient selected from the list:
   a) PDE5 inhibitors, such as sildenafil, tadalafil, vardenafil, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and; the pyrazolo[4,3-d]pyrimidin-4-ones disclosed in WO00/27848 particularly N-[[3-(4,7-dihydro-1-methyl-7-oxo-3-propyl-1H-pyrazolo[4,3-d]-pyrimidin-5-yl)-4-propxyphenyl]sulfonyl]-1-methyl2-pyrrolidinepropanamide [DA-8159 (Example 68 of WO00/27848)];
   b) dopaminergic agents, preferably apomorphine or a selective D₂, D₃ or D₂/D₃agonist such as, pramipexole and ropirinol (as claimed in WO-00/23056), PNU95666 (as claimed in WO-00/40226);
   c) melanocortin receptor agonists, such as melanotan II; PT-14; PT-141; compounds claimed in WO-99/64002, WO-00/74679, WO-99/55679, WO-01/05401, WO-00/58361, WO-01/14879, WO-01/13112 and WO-99/54358; selective MC4 receptor agonists such as those disclosed by Martin *et al* [European Journal of Pharmacology, 454 71-79 (2002)] particularly (*N*-[(3*R*)-1,2,3,4-tetrahydroisoquinolinium-3-ylcarbonyl]-(1*R*)-1-(4-chlorobenzyl)-2-[4-cyclohexyl-4-(1H-1,2,4-triazol-1-ylmethyl)piperidin-1-yl]-2-oxoethylamine (THIQ); and selective MC3 receptor agonists
   d) selective estrogen receptor modulators (SERMs) such as lasofoxifene and raloxifene;
   e) tibolone;
   f) an androgen such as androsterone, dehydro-androsterone, testosterone, androstanedione and a synthetic androgen; and
   g) an oestrogen, such as oestradiol, oestrone, oestriol and a synthetic estrogen, such as oestrogen benzoate.

For treating MED, the compounds of the invention may be combined with one or more active ingredient selected from the list:
a) PDE5 inhibitors, such as sildenafil, tadalafil, vardenafil, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and; the pyrazolo[4,3-d]pyrimidin-4-ones disclosed in WO00/27848 particularly N-[[3-(4,7-dihydro-1-methyl-7-oxo-3-propyl-1H-pyrazolo[4,3-d]-pyrimidin-5-yl)-4-propxyphenyl]sulfonyl]-1-methyl2-pyrrolidinepropanamide [DA-8159 (Example 68 of WO-00/27848)];
b) dopaminergic agents, preferably apomorphine or a selective D₂, D₃ or D₂/D₃agonist such as, pramipexole and ropirinol (as claimed in WO-00/23056), PNU95666 (as claimed in WO-00/40226); and
c) melanocortin receptor agonists, such as melanotan II; PT-14; PT-141; compounds claimed in WO-99/64002, WO-00/74679, WO-99/55679, WO-01/05401, WO-00/58361, WO-01/14879, WO-01/13112 and WO-99/54358; selective MC4 receptor agonists such as those disclosed by Martin *et al* [European Journal of Pharmacology, 454 71-79 (2002)] particularly (*N*-[(3*R*)-1,2,3,4-tetrahydroisoquinolinium-3-ylcarbonyl]-(1*R*)-1-(4-chlorobenzyl)-2-[4-cyclohexyl-4-(1H-1,2,4-triazol-1-ylmethyl)piperidin-1-yl]-2-oxoethylamine (THIQ); and selective MC3 receptor agonists.

If a combination of active agents are administered, then they may be administered simultaneously, separately or sequentially.

The invention also includes the following aspects. The preferred embodiments specified hereinabove for the first aspect extend to these aspects.

The invention additionally includes, but is not limited to:
(i) A pharmaceutical composition including a compound of the invention, together with a pharmaceutically acceptable excipient, diluent or carrier.
(ii) A compound of the invention for use as a medicament.
(iii) The use of a compound of the invention as a medicament for treating or preventing a condition for which a beneficial therapeutic response can be obtained by the inhibition of neutral endopeptidase.
(iv) The use of a compound of the invention as a medicament for treating or preventing cardiovascular diseases and conditions, preferably essential hypertension, pulmonary hypertension, secondary hypertension, isolated systolic hypertension, hypertension associated with diabetes, hypertension associated with atherosclerosis, renovascular hypertension, congestive heart failure, angina, stroke, glaucoma, impaired renal function, renal failure, obesity, metabolic diseases (including Metabolic Syndrome), diabetes and impaired glucose tolerance, including complications thereof, such as diabetic retinopathy and diabetic neuropathy.
(v) A cardiovascular disease treating pharmaceutical composition comprising a compound of the invention together with a pharmaceutically acceptable excipient, diluent or carrier.
(vi) A compound of the invention for use in treating or preventing cardiovascular diseases and conditions.
(vii) The use of a compound of the invention in the manufacture of a medicament for treating or preventing cardiovascular disease and conditions.

The invention is illustrated by the following non-limiting examples in which the following abbreviations and definitions are used:
- ES⁺: electrospray ionisation positive scan
- ES⁻: electrospray ionisation negative scan
- HPLC: high pressure liquid chromatography
- m/z: mass spectrum peak
- MS: mass spectrum
- TS⁺: thermospray ionisation positive scan

¹H Nuclear magnetic resonance (NMR) spectra were in all cases consistent with the proposed structures. Characteristic chemical shifts (δ) are given in parts-per-million downfield from tetramethylsilane using conventional abbreviations for designation of major peaks: e.g. s, singlet; d, doublet; t, triplet; q, quartet; m, multiplet; br, broad. The following abbreviations have been used for common solvents: CDCl₃, deuterochloroform; DMSO, dimethylsulphoxide. The abbreviation psi means pounds per square inch and LRMS means low resolution mass spectrometry. Where thin layer chromatography (TLC) has been used it refers to silica gel TLC using silica gel 60 F₂₅₄ plates, R_{f} is the distance travelled by a compound divided by the distance travelled by the solvent front on a TLC plate. Melting points were determined using a Perkin Elmer DSC7 at a heating rate of 20°C/minute).

### Example 1

### (2R)-2-[(1-{[((1S)-2-Carboxy-1-{[(3-fluorobenzyl)oxy]methyl}ethyl)amino]-carbonyl}cyclopentyl)methyl]pentanoic acid

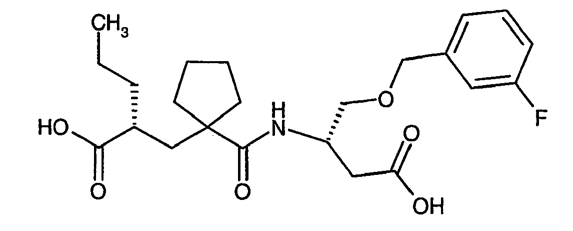

Trifluoroacetic acid (2ml) was added to a solution of the ester from preparation 11 (80mg, 0.16mmol) in dichloromethane (3ml), and the reaction stirred at room temperature for 90 minutes. The solution was concentrated under reduced pressure and the trifluoroacetic acid removed by azeotrope with toluene, ethyl acetate and dichloromethane to afford the title compound as a colourless oil, 44 mg. ¹H NMR (CDCl₃, 400MHz) δ: 0.87 (t, 3H), 1.22-1.70 (m, 10H), 1.77 (dd, 1H), 1.84 (m, 1H), 1.99 (dd, 1H), 2.17 (m, 1H), 2.38 (m, 1H), 2.71 (d, 2H), 3.59 (m, 2H), 4.50 (m, 3H), 6.58 (d, 1H), 6.95-7.08 (m, 3H), 7.26 (m, 1H). LRMS : m/z (ES⁺) 438 [MH⁺]

### Examples 2 to 4

The following examples of general formula: 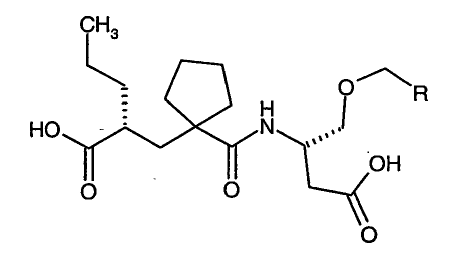 were obtained quantitatively as colourless oils, from the corresponding *tert*-butyl esters, following a similar procedure to that described in Example 1.

| Ex. No | R² | Data |
|---|---|---|
| 2 | 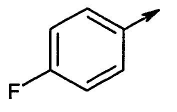 | ¹H NMR (CDCl₃, 400MHz) δ: 0.89 (t, 3H), 1.30 (m, 2H), 1.38-1.76 (m, 8H), 1.84 (m, 2H), 1.99 (dd, 1H), 2.17 (m, 1H), 2.39 (m, 1H), 2.75 (d, 2H), 3.59 (m, 2H), 4.50 (m, 3H), 6.62 (d, 1H), 7.02 (m, 2H), 7.27 (d, 2H). LRMS : m/z (ES⁺) 438.3 [MH⁺] |
| 3 | 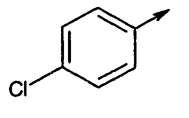 | ¹H NMR (CDCl₃, 400MHz) δ: 0.85 (t, 3H), 1.22-1.68 (m, 10H), 1.80 (m, 2H), 1.98 (dd, 1H), 2.16 (m, 1H), 2.38 (m, 1H), 2.69 (d, 2H), 3.58 (m, 2H), 4.46 (m, 3H), 6.58 (d, 1H), 7.20-7.34 (m, 4H). LRMS : m/z (ES⁺) 454 [MH⁺] Microanalysis found: C, 59.16; H, 6.97; N, 2.95. C₂₃H₃₂ClNO₆;0.20CH₂Cl₂ requires C, 59.17; H, 6.93; N, 2.97%. |
| 4 | 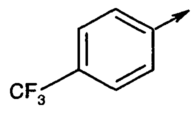 | ¹H NMR (CDCl₃, 400MHz) δ: 0.86 (t, 3H), 1.22-1.72 (m, 10H), 1.82 (m, 2H), 1.98 (dd, 1H), 2.17 (m, 1H), 2.38 (m, 1H), 2.72 (d, 2H), 3.60 (m, 2H), 4.50 (m, 1H), 4.58 (s, 2H), 6.60 (d, 1H), 7.40 (d, 2H), 7.60 (d, 2H). LRMS : m/z (ES⁺) 488 [MH⁺] |

### Example 5

### (2R)-2-[(1-{[((1R)-2-Carboxy-1-{[(4-chlorobenzyl)oxy]methyl}ethyl)amino]-carbonyl}cyclopentyl)methyl]pentanoic acid

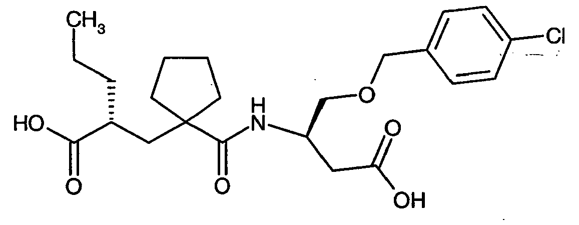

Trifluoroacetic acid (1 ml) was added to a solution of the ester from preparation 15 (125mg, 0.25mmol) in dichloromethane (5ml), and the reaction stirred at room temperature for 18 hours. The solution was concentrated under reduced pressure and the residue azeotroped with dichloromethane (6x). The crude product was purified by column chromatography on silica gel using dichloromethane:methanol:acetic acid (97:3:0.3) as eluant to afford the title compound, 30mg. ¹H NMR (CDCl₃, 400MHz) δ: 0.84 (t, 3H), 1.18-1.38 (m, 3H), 1.42-1.52 (m, 7H), 1.79 (dd, 1H), 1.96 (m, 2H), 2.10 (m, 1H), 2.38 (m, 1H), 2.68 (m, 2H), 3.60 (m, 2H), 4.44 (m, 3H), 7.04 (d, 1H), 7.24 (m, 4H). LRMS : m/z (ES⁺) 476 [MNa⁺]; Microanalysis found: C, 58.72; H, 7.05; N, 2.88. C₂₃H₃₂CINO₆;H₂O requires C, 58.53; H, 7.26; N, 2.97%

### Example 6

### (2R)-2-[(1-{[((1R)-2-Carboxy-1-{[(4-fluorobenzyl)oxy]methyl}ethyl)amino]-carbonyl}cyclopentyl)methyl]pentanoic acid

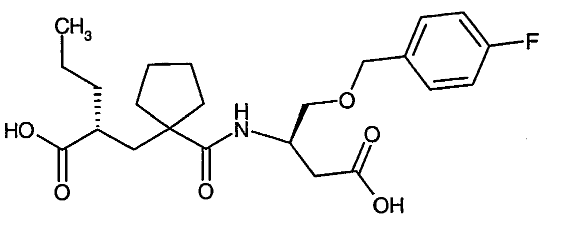

Imidazole (50mg, 0.73mmol) and sodium hydride (60% dispersion in mineral oil, 1.24g, 31mmol) were added to a cooled (-15°C) solution of the alcohol from preparation 7 (1.2g, 3.1 mmol) in tetrahydrofuran (70ml), and the mixture stirred for 45 minutes. A solution of 4-fluorobenzylbromide (650mg, 3.4mmol) was added and the reaction stirred for a further 2 hours at -15°C, and then allowed to warm to room temperature. Stirring was continued for a further 3 hours, then the flask cooled in an ice-bath. The reaction was quenched by the addition of water, diluted with 1N hydrochloric acid, and the mixture extracted with ethyl acetate. The combined organic solutions were dried (MgSO₄) and evaporated under reduced pressure. The product was purified by column chromatography on silica gel using an elution gradient of dichloromethane:methanol (98:2 to 92:8). This intermediate was dissolved in trifluoroacetic acid (2ml) and dichloromethane (4ml), and the reaction stirred at room temperature for 18 hours. The solution was concentrated under reduced pressure and the residue azeotroped with dichloromethane (3x). The crude product was purified by column chromatography on silica gel using dichloromethane:methanol:acetic acid (96:4:0.4) as eluant to afford the title compound, 100mg. ¹H NMR (CDCl₃, 400MHz) δ: 0.82 (t, 3H), 1.25 (m, 3H), 1.40-1.81 (m, 8H), 1.96 (m, 2H), 2.15 (m, 1H), 2.38 (m, 1H), 2.58-2.80 (m, 2H), 3.48-3.72 (m, 2H), 4.48 (m, 3H), 6.82 (m, 1H), 7.01 (d, 2H), 7.24 (d, 2H). LRMS : m/z (ES⁻) 436 [M-H]⁻

### Example 7

### (2R)-2-[(1-{[((1R)-2-Carboxy-1-{[(3-methoxybenzyl)oxy]methyl}ethyl)amino]-carbonyl}cyclopentyl)methyl]pentanoic acid

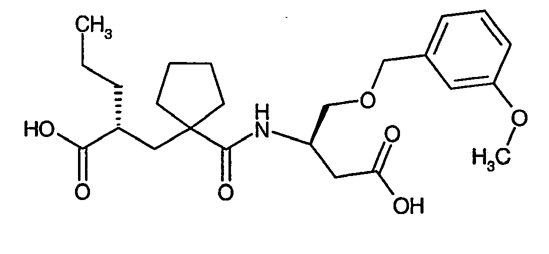

The title compound was obtained in 81 % yield from the ester from preparation 16, following the procedure described in Example 5. ¹H NMR (CDCl₃, 400MHz) δ: 0.84 (t, 3H), 1.20-1.38 (m, 3H), 1.44-1.70 (m, 7H), 1.79 (dd, 1H), 1.90 (m, 1H), 2.02 (m, 2H), 2.38 (m, 1H), 2.70 (m, 2H), 3.60 (m, 2H), 3.80 (s, 3H), 4.44 (m, 3H), 6.84 (m, 2H), 7.02 (d, 1H), 7.24 (m, 2H), 7.82 (br s, 2H). LRMS : m/z (ES⁺) 472 [MNa⁺].

### Example 8

### (2R)-2-[(1-{[((1R)-2-Carboxy-1-{[(4-cyanobenzyl)oxy]methyl}ethyl)amino]-carbonyl}cyclopentyl)methyl]pentanoic acid

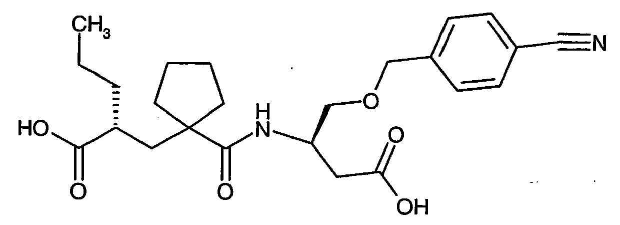

A solution of the ester from preparation 52 (60mg, 0.13mmol) and 1M sodium hydroxide (2ml) in dioxan (6ml) was stirred at room temperature for 3 hours. The reaction was concentrated under reduced pressure the residue suspended in water (3ml), the pH adjusted to 1 using 2N hydrochloric acid, and the solution extracted with ethyl acetate (3x3ml). The combined organic extracts were washed with brine, dried (MgSO₄) and evaporated under reduced pressure to afford the title compound as a colourless gum, 55mg. ¹H NMR (CDCl₃, 400MHz) δ: 0.82 (t, 3H), 1.18-1.38 (m, 3H), 1.42-1.72 (m, 7H), 1.79 (d, 1H), 1.98 (m, 2H), 2.18 (m, 1H), 2.40 (m, 1 H), 2.62 (dd, 1H), 2.79 (dd, 1H), 3.62 (m, 2H), 4.52 (m, 1H), 4.59 (s, 2H), 6.94 (d, 1H), 7.40 (d, 2H), 7.62 (d, 2H). LRMS : m/z (ES⁻) 443 [M-H]⁻

### Example 9

### Sodium (2R)-2-[(1-{[((1R)-1-{[(5-methyl-2-pyridinyl)methoxy]methyl}-3-oxido-3-oxopropyl)amino]carbonyl}cyclopentyl)methyl]pentanoate

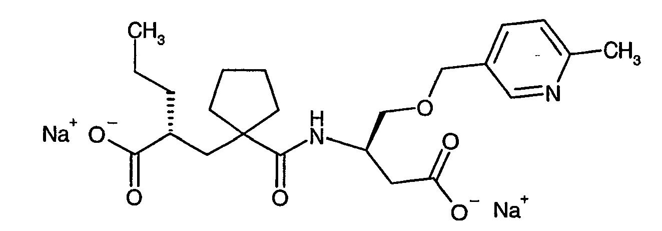

A solution of the ester from preparation 53 (40mg, 0.07mmol) and 1N sodium hydroxide solution (210µl, 0.21 mmol) in dioxan (0.6ml) was stirred at room temperature for 2 hours. The mixture was evaporated under reduced pressure and the residue azeotroped with toluene to afford the title compound, 40mg. ¹H NMR (CD₃OD, 400MHz) δ: 0.82 (t, 3H), 1.25 (m, 3H), 1.42-1.64 (m, 7H), 1.76 (dd, 1H), 1.98 (m, 2H), 2.08 (m, 1H), 2.18 (m, 1H), 2.42 (m, 2H), 2.50 (s, 3H), 3.60 (m, 2H), 4.41 (m, 1H), 4.58 (q, 2H), 7.25 (d, 1H), 7.78 (d, 1H), 8.38 (s, 1H). HRMS : m/z (ES⁺) 435.2488 [MH⁺] C₂₃H₃₄N₂O₆ requires 435.2490.

### Example 10

### (2S)-2-[(1-{[((1S)-2-Carboxy-1-{[(3-methoxybenzyl)oxy]methyl}ethyl)amino]-carbonyl}cyclopentyl)methyl]-4-methoxybutanoic acid

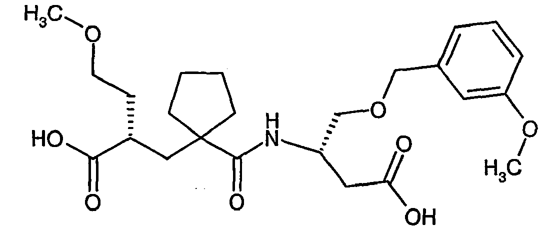

Trifluoroacetic acid (1 ml) was added to a solution of the *tert*-butyl ester from preparation 17 (26mg, 0.05mmol) in dichloromethane (4ml), and the reaction stirred at room temperature for 18 hours. The solution was concentrated under reduced pressure and the trifluoroacetic acid removed by azeotrope with toluene, ethyl acetate and dichloromethane to afford the title compound as a colourless oil, 17mg. ¹H NMR (CDCl₃ + drop D₂O, 400MHz, rotamers) δ: 1.23-2.15 (m, 12H), 2.48 (m, 1H), 2.63 (m, 2H), 3.30 (2xs, 3H), 3.39 (m, 2H), 3.57 (m, 2H), 3.79 (m, 3H), 4.21 (m, 1H), 4.47 (m, 2H), 6.57, 6.60 (2xd, 1H), 6.82 (m, 3H), 7.22 (m, 1H). HRMS : m/z (ES⁺) 466.2450 [MH⁺] C₂₄H₃₅NO₈ requires 466.2436

### Examples 11 to 15

The following examples of general formula: 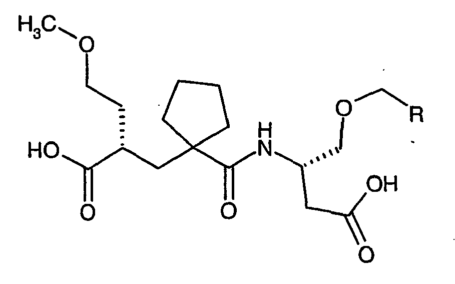 were obtained quantitatively from the corresponding tert-butyl esters, following a similar procedure to that described in Example 10.

| Ex. No | R | Data |
|---|---|---|
| 11 | 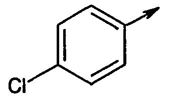 | ¹H NMR (CDCl₃+ D₂O, 400MHz, rotamers) δ: 1.45-1.76 (m, 6H), 1.79-2.14 (m, 6H), 2.48 (m, 1H), 2.63 (m, 2H), 3.30 (d, 3H), 3.33-3.60 (m, 4H), 4.48 (m, 3H), 6.45, 6.56 (2xd, 1H), 7.23 (m, 2H), 7.32 (m, 2H). HRMS : m/z (ES) 470.1956 [MH⁺] C₂₃H₃₂ClNO₇ requires 470.1940 [MH⁺] |
| 12 | 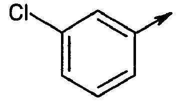 | ¹H NMR (CDCl₃ + D₂O, 400MHz) δ: 1.48-1.76 (m, 7H), 1.79-1.97 (m, 3H), 2.00-2.16 (m, 2H), 2.52 (m, 1H), 2.75 (d, 2H), 3.34 (s, 3H), 3.40 (m, 2H), 3.62 (m, 2H), 4.57 (m, 1H), 4.61 (s, 2H), 7.22 (m, 2H), 7.39 (m, 2H). HRMS : m/z (ES) 470.1947 [MH⁺] C₂₃H₃₂ClNO₇ requires 470.1940 [MH⁺] |
| 13 | 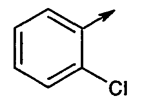 | ¹H NMR (CDCl₃ + D₂O, 400MHz) δ: 1.45-1.77 (m, 7H), 1.83 (m, 3H), 2.00-2.17 (m, 2H), 2.50 (m, 1H), 2.68 (d, 2H), 3.32 (s, 3H), 3.40 (m, 2H), 3.58 (m, 2H), 4.50 (m, 3H), 7.18 (m, 1H), 7.26 (m, 3H); HRMS : m/z (ES) 470.1944 [MH⁺] C₂₃H₃₂CINO₇ requires 470.1940 [MH⁺] |
| 14 | 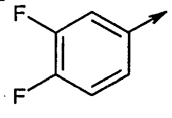 | ¹H NMR (CDCl₃, 400MHz) δ: 1.44-1.74 (m, 6H), 1.78-193 (m, 4H), 1.99-2.16 (m, 2H), 2.48 (m, 1H), 2.70 (d, 2H), 3.30 (s, 3H), 3.40 (m, 2H), 3.57 (m, 2H), 4.46 (m, 3H), 6.62 (d, 1H), 6.99 (m, 1H), 7.12 (m, 2H), 8.36 (br, s, 2H). HRMS : m/z (ES) 472.2144 [MH⁺] C₂₃H₃₁F₂NO₇ requires 472.2142 [MH⁺] |
| 15 | 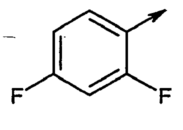 | ¹H NMR (CDCl₃, 400MHz) δ: 1.42-1.76 (m, 8H), 1.84 (m, 2H), 1.99-2.18 (m, 2H), 2.46 (m, 1H), 2.62 (m, 2H), 3.26 (s, 3H), 3.39 (m, 2H), 3.57 (m, 2H), 4.50 (m, 3H), 6.58 (d, 1H), 6.76-6.86 (m, 2H), 7.30 (m, 1H). HRMS : m/z (ES) 472.2143 [MH⁺] C₂₃H₃₁F₂NO₇ requires 472.2142 [MH⁺] |

### Example 16

### (2S)-2-[(1-{[((1R)-2-Carboxy-1-{[(4-chlorobenzyl)oxy]methyl}ethyl)amino]-carbonyl}cyclopentyl)methyl]-4-methoxybutanoic acid

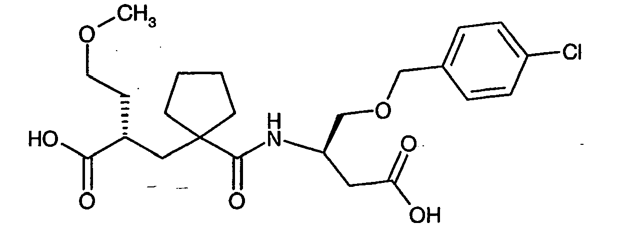

A solution of the ester from preparation 23 (100mg, 0.19mmol) and trifluoroacetic acid (1ml) in dichloromethane (5ml) was stirred at room temperature for 18 hours. The mixture was concentrated under reduced pressure and azeotroped with toluene. The residue was partitioned between dichloromethane and water, the layers separated, and the organic phase dried (MgSO₄) and evaporated under reduced pressure to afford the title compound as a colourless oil, 73mg. ¹H NMR (CDCl₃, 400MHz) δ: 1.45-1.78 (m, 7H), 1.81-2.02 (m, 4H), 2.18 (m, 1H), 2.47-2.75 (m, 3H), 3.38 (m, 4H), 3.57 (m, 3H), 4.46 (s, 2H), 4.73 (m, 1H), 6.40, 6.58 (2xd, 1H), 7.21 (d, 2H), 7.30 (d, 2H).

### Example 17

### (2R)-2-({1-[({(1S)-1-[(Benzyloxy)methyl]-3-ethoxy-3-oxopropyl}amino)carbonyl]-cyclopentyl}methyl)pentanoic acid

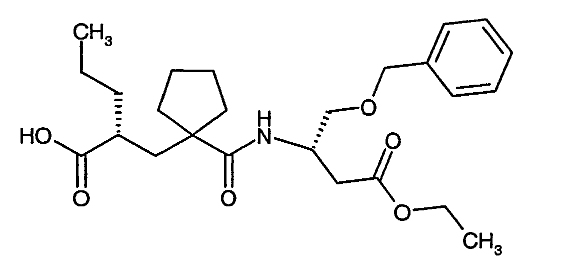

A solution of the ester from preparation 46 (110mg, 0.22mmol) in trifluoroacetic acid (0.5ml) and dichloromethane (5ml) was stirred at room temperature for 72 hours. The reaction was concentrated under reduced pressure and the residue azeotroped with toluene and dichloromethane to afford the title compound, 95mg. ¹H NMR (CDCl₃, 400MHz) δ: 0.90 (t, 3H), 1.20-1.43 (m, 6H), 1.50-1.78 (m, 7H), 2.00 (m, 3H), 2.39 (m, 2H), 2.62 (m, 2H), 3.59 (m, 2H), 4.10 (q, 2H), 4.50 (m, 3H), 6.77 (d, 1H), 7.35 (m, 5H). LRMS : m/z (ES⁺) 448.5 [MH⁺]

### Example 18

### (2R)-2-[(1-{[((1S)-3-Ethoxy-1-{[(3-methoxybenzyl)oxy]methyl}-3-oxopropyl)amino]-carbonyl}cyclopentyl)methyl]pentanoic acid

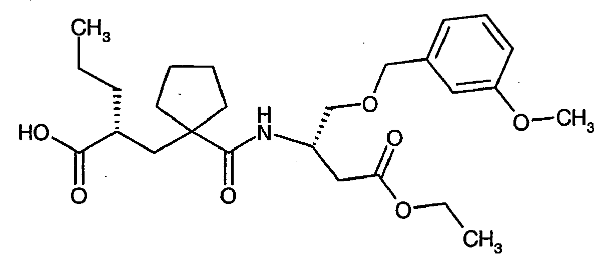

Trifluoroacetic acid (1 ml) was added to a solution of the ester from preparation 24 (280mg, 0.53mmol) in dichloromethane (5ml), and the solution stirred at room temperature for 4 hours. The reaction was concentrated under reduced pressure and the residue azeotroped with ethyl acetate. The crude product was purified by column chromatography on silica gel using dichloromethane:methanol (98:2) to afford the title compound as a colourless oil. ¹H NMR (CDCl₃, 400MHz) δ: 0.86 (t, 3H), 1.23 (t, 3H), 1.34 (m, 3H), 1.50-1.76 (m, 8H), 2.00 (m, 3H), 2.38 (m, 1H), 2.62 (m, 2H), 3.58 (m, 2H), 3.81 (s, 3H), 4.14 (q, 2H), 4.45 (m, 3H), 6.62 (d, 1H), 6.84 (m, 3H), 7.24 (m, 1H). [α]_{D} = -2.13° (c = 0.3, methanol) Microanalysis found: C, 65.10; H, 8.29; N, 3.01. C₂₆H₃₉NO₇ requires C, 65.39; H, 8.23; N, 2.93%

### Example 19

### (2R)-2-[(1-{[((1S)-1-{[(4-Chlorobenzyl)oxy]methyl}-3-ethoxy-3-oxopropyl)amino]-carbonyl}cyclopentyl)methyl]pentanoic acid

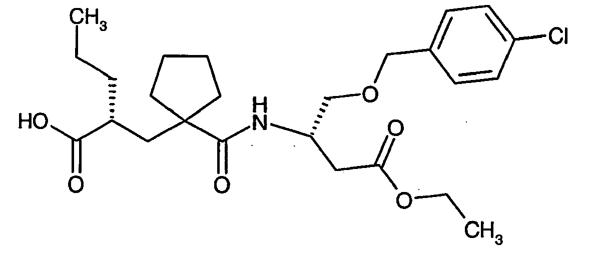

Trifluoroacetic acid (1.2ml) was added to a solution of the ester from preparation 25 (175mg, 0.33mmol) in dichloromethane (10ml), and the solution stirred at room temperature for 18 hours. The mixture was concentrated under reduced pressure and the residue azeotroped with toluene (3x20ml), ethyl acetate (20ml), and dichloromethane (20ml). The crude product was purified by column chromatography on silica gel using an elution gradient of dichloromethane:methanol (98:2 to 95:5) to give the title compound as a colourless oil. ¹H NMR (CDCl₃, 400MHz) δ: 0.87 (t, 3H), 1.21-1.41 (m, 6H), 1.49-1.78 (m, 8H), 2.00 (m, 3H), 2.38 (m, 1H), 2.62 (m, 2H), 3.57 (m, 2H), 4.14 (q, 2H), 4.45 (m, 3H), 6.60 (d, 1H), 7.23 (m, 2H), 7.35 (d, 2H). HRMS : m/z (ES⁺) 482.2314 [MH⁺] C₂₅H₃₆ClNO₆ requires 482.2306; [α]_{D} = -13.2° (c =0.1, methanol)

### Examples 20 to 24

The following examples of general formula: 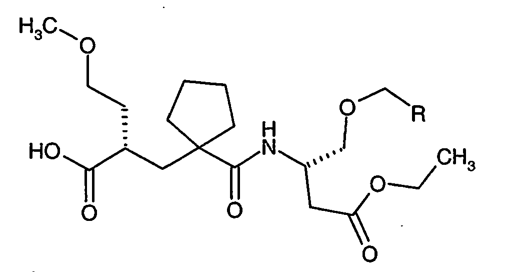 were obtained, from the corresponding *tert*-butyl esters, following a similar procedure to that described in Example 19.

| Ex. No | R | Data |
|---|---|---|
| 20 | 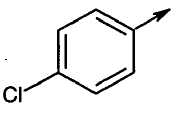 | ¹H NMR (CDCl₃, 400MHz) δ: 1.22 (t, 3H), 1.52-1.75 (m, 8H), 1.99 (m, 3H), 2.10 (dd, 1H), 2.56 (m, 1H), 2.61 (m, 2H), 3.26 (s, 3H), 3.38 (m, 2H), 3.57 (m, 2H), 4.10 (q, 2H), 4.45 (m, 3H), 6.63 (d, 1H), 7.22 (d, 2H), 7.32 (d, 2H); LRMS : m/z (APCI) 498 [MH⁺] |
| 21 | 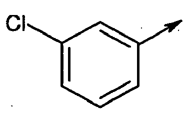 | ¹H NMR (CDCl₃, 400MHz) δ: 1.22 (t, 3H), 1.50-1.75 (m, 8H), 1.88-2.10 (m, 4H), 2.54 (m, 1H),-2.64 (m, 2H), 3.26 (s, 3H), 3.38 (m, 2H), 3.62 (m, 2H), 4.09 (q, 2H), 4.48 (m, 1H), 4.60 (s, 2H), 6.74 (d, 1H), 7.22 (m, 2H), 7.38 (d, 1H), 7.40 (d, 1H); HRMS : m/z (ES⁺) 498.2261 [MH⁺] C₂₅H₃₆CINO₇ requires 498.2253 |
| 22 | 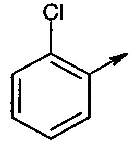 | ¹H NMR (CDCl₃, 400MHz) δ: 1.22 (t, 3H), 1.54-1.76 (m, 8H), 1.88-2.14 (m, 4H), 2.52 (m, 1H), 2.62 (m, 2H), 3.24 (s, 3H), 3.38 (m, 2H), 3.58 (m, 2H), 4.18 (q, 2H), 4.44 (m, 3H), 6.74 (d, 1H), 7.18 (m, 1H), 7.25 (m, 3H). HRMS : m/z (ES⁺) 498.2255 [MH⁺] C₂₅H₃₆ClNO₇ requires 498.2253 |
| 23^{a} | 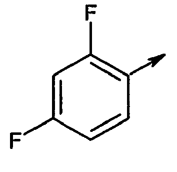 | ¹H NMR (CDCl₃, 400MHz) δ: 1.22 (t, 3H), 1.52-1.76 (m, 8H), 1.98 (m, 3H), 2.05 (m, 1H), 2.50 (m, 1H), 2.60 (m, 2H), 326 (s, 3H), 3.38 (m, 2H), 3.58 (m, 2H), 4.07 (q, 2H), 4.43 (m, 1H), 4.52 (s, 2H), 6.68 (d, 1H), 6.78-6.90 (m, 2H), 7.35 (m, 1H); HRMS : m/z (ES⁺) 500.2451 [MH⁺] C₂₅H₃₅F₂NO₇ requires 500.2455 |
| 24 | 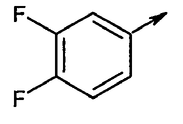 | ¹H NMR (CDCl₃, 400MHz) δ: 1.22 (t, 3H), 1.50-1.74 (m, 8H), 1.98 (m, 3H), 2.07 (dd, 1H), 2.56 (m, 1H), 2.61 (m, 2H), 3.26 (s, 3H), 3.38 (m, 2H), 3.58 (m, 2H), 4.12 (q, 2H), 4.43 (m, 3H), 6.66 (d, 1H), 7.00 (m, 1H), 7.14 (m, 2H); HRMS : m/z (ES⁺) 500.2458 [MH⁺] C₂₅H₃₅F₂NO₇ requires 500.2455 |

| | | |
|---|---|---|
| a-isolated without column chromatography | | |

### Example 25

### (2R)-2-[(1-{[((1R)-1-{[(4-Chlorobenzyl)oxy]methyl}-3-ethoxy-3-oxopropyl)amino]-carbonyl}cyclopentyl)methyl]pentanoic acid

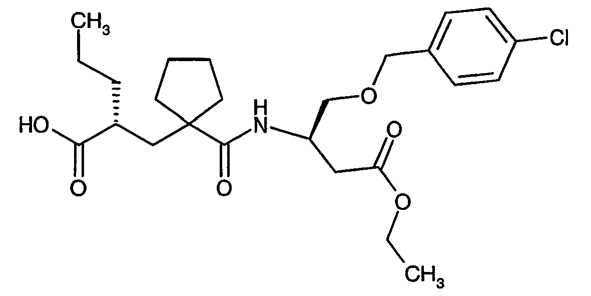

A solution of the ester from preparation 99 (98mg, 0.18mmol) and trifluoroacetic acid (2ml) in dichloromethane (4ml) was stirred at room temperature for 18 hours. The mixture was concentrated under reduced pressure and the residue azeotroped with dichloromethane to afford the title compound, 73mg. ¹H NMR (CDCl₃, 400MHz) δ: 0.87 (t, 3H), 1.21-1.41 (m, 6H), 1.50-1.78 (m, 8H), 1.96 (m, 1H), 2.02 (m, 2H), 2.38 (m, 1H), 2.60 (m, 2H), 3.57 (m, 2H), 4.10 (q, 2H), 4.45 (m, 3H), 6.58 (d, 1H), 7.23 (m, 2H), 7.35 (d, 2H); HRMS : m/z (ES⁺) 504 [MNa⁺]; [α]_{D} = +10.00 (c =0.11, methanol).

### Example 26

### (2R)-2-{[1-({[(1S)-1-[(Benzyloxy)methyl]-3-(2-butoxyethoxy)-3-oxopropyl]amino}-carbonyl)cyclopentyl]methyl}pentanoic acid

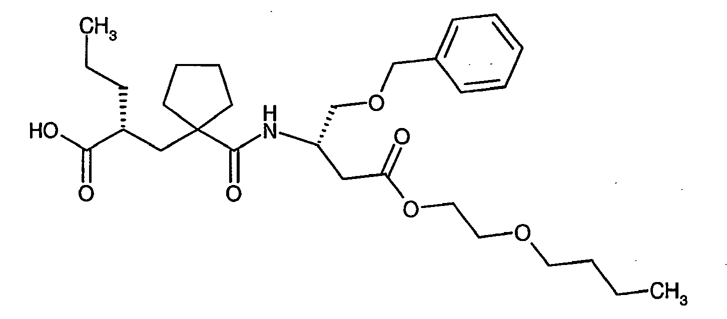

A solution of the ester from preparation 50 (100mg, 0.17mmol) in trifluoroacetic acid (0.3ml) and dichloromethane (3ml) was stirred at room temperature for 18 hours, and then evaporated under reduced pressure to afford the title compound. ¹H NMR (CDCl₃, 400MHz) δ: 0.88 (m, 6H), 1.30 (m, 5H), 1.48-1.74 (m, 9H), 1.93 (m, 1H), 1.99-2.16 (m, 2H), 2.38 (m, 1H), 2.68 (d, 2H), 3.45 (m, 3H), 3.58 (m, 2H), 3.61 (m, 2H), 4.17 (m, 1H), 4.23 (m, 1H), 4.50 (m, 3H), 6.59 (d, 1H), 7.22 (m, 5H); LRMS : m/z (ES⁻) 518 [M-H]⁻.

### Example 27

### (2R)-2-({1-[({(1S)-1-[(Benzyloxy)methyl]-3-oxo-3-[2-oxo-2-(1-piperidinyl)ethoxy]-propyl}amino)carbonyl]cyclopentyl}methyl)pentanoic acid

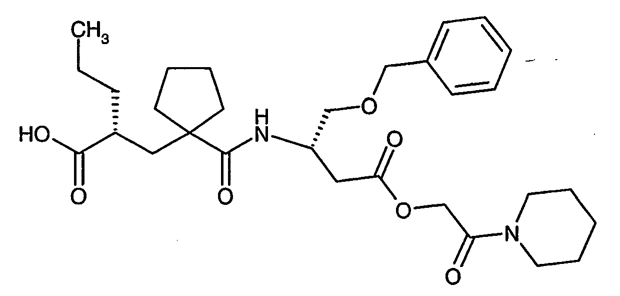

A solution of the ester from preparation 51 (100mg, 0.16mmol) in trifluoroacetic acid (0.3ml) and dichloromethane (3ml) was stirred at room temperature for 18 hours, and concentrated under reduced pressure. The residue was azeotroped with toluene, and the crude product then purified by column chromatography on silica gel using dichloromethane:methanol (98:2 to 95:5) to afford the title compound, 30mg. ¹H NMR (CDCl₃, 400MHz) δ: 0.85 (t, 3H), 1.24-1.78 (m, 17H), 1.90 (m, 1H), 1.99 (dd, 1H), 2.18 (m, 1H), 2.37 (m, 1H), 2.80 (d, 2H), 3.25 (m, 2H), 3.50 (m, 1H), 3.60 (m, 3H), 4.50 (s, 2H), 4.61 (m, 2H), 4.78 (d, 1H), 6.63 (d, 1H), 7.28 (m, 5H); LRMS : m/z (ES⁻) 543 [M-H]⁻.

### Example 28

### (2R)-2-{[1-({[(1R)-3-Ethoxy-3-oxo-1-(2-phenoxyethyl)propyl]amino}carbonyl)-cyclopentyl]methyl}pentanoic acid

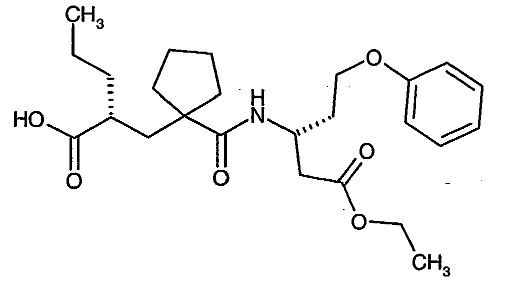

Trifluoroacetic acid (1 ml) was added to a solution of the ester from preparation 67 (60mg, 0.12mmol) in dichloromethane (5ml), and the solution stirred at room temperature for 18 hours. The mixture was concentrated under reduced pressure, the residue dissolved in dichloromethane, washed with water, dried (MgSO₄) and evaporated under reduced pressure to afford the title compound, 40mg. ¹H-NMR (CDCl₃, 400MHz) δ: 0.83 (t, 3H), 1.26 (m, 8H), 1.55-1.77 (m, 7H), 2.00 (m, 2H), 2.10 (m, 2H), 2.35 (m, 1H), 2.65 (m, 2H), 4.02 (m, 2H), 4.17 (q, 2H), 4.48 (m, 1H), 6.86 (m, 3H), 6.99 (m, 1H), 7.25 (m, 2H).

### Examples 29 to 30

The compounds of the following general formula: 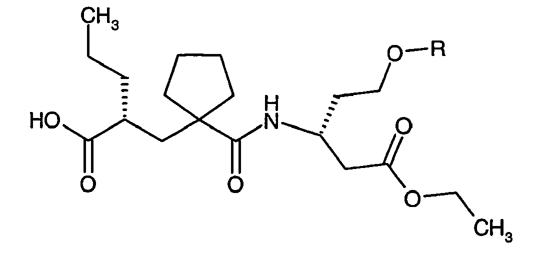 were prepared from the corresponding *tert*-butyl esters, following a similar procedure to that described in Example 28.

| Ex. No | R | Data |
|---|---|---|
| 29 | 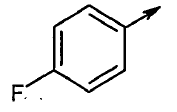 | ¹H-NMR (CDCl₃, 400MHz) δ: 0.85 (t, 3H), 1.26 (m, 6H), 1.48-1.75 (m, 8H), 1.95-2.22 (m, 5H), 2.37 (m, 1H), 2.62 (m, 2H), 3.99 (m, 2H), 4.18 (q, 2H), 4.45 (m, 1H), 6.77 (d, 1H), 6.80 (m, 2H), 6.98 (dd, 2H). LRMS : m/z (ES⁺) 488 [MNa⁺] |
| 30 | 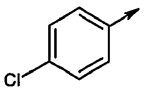 | ¹H-NMR (CDCl₃, 400MHz) δ: 0.85 (t, 3H), 1.28 (m, 6H), 1.50-1.77 (m, 8H), 1.92-2.14 (m, 5H), 2.34 (m, 1H), 2.64 (d, 2H), 4.00 (t, 2H), 4.18 (q, 2H), 4.42 (m, 1H), 6.80 (d, 2H), 6.96 (d, 1H), 7.22 (d, 2H). LRMS : m/z (ES⁺) 504 [MNa⁺] |

### Example 31

### (2R)-2-{[1-({[(1S)-2-(Benzyloxy)-1-(carboxymethyl)ethyl]amino}carbonyl)-cyclopentyl]methyl}pentanoic acid

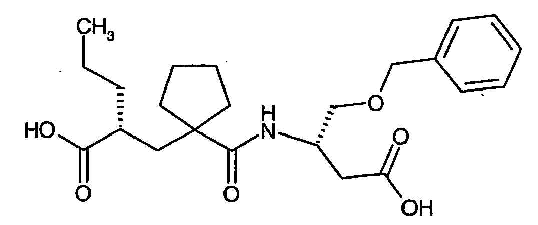

A solution of the ethyl ester from example 17 (70mg, 0.16mmol) in sodium hydroxide solution (1 ml, 1N) and methanol (5ml) was stirred at room temperature for 18 hours. The reaction was concentrated under reduced pressure to remove the methanol, and the residue was partitioned between ethyl acetate (10ml) and hydrochloric acid (2N, 10ml). The phases were separated, the aqueous layer extracted with ethyl acetate (4x10ml), the combined organic solutions washed with brine (10ml), dried (MgSO₄) and evaporated under reduced pressure to afford the title compound, 50mg; ¹H NMR (CDCl₃, 400MHz) δ: 0.92 (t, 3H), 1.23-2.39 (m, 15H), 2.74 (d, 2H), 3.60 (m, 2H), 4.52 (m, 3H), 6.66 (d, 1H), 7.35 (m, 5H); HRMS: m/z (ES⁺) 420.2374 [MH⁺]; C₂₃H₃₃NO₆ calculated 420.2381.

### Example 32

### (2R)-2-[(1-{[((1S)-2-Carboxy-1-{[(3-methoxybenzyl)oxy]methyl}ethyl)amino]carbonyl}-cyclopentyl)methyl]pentanoic acid

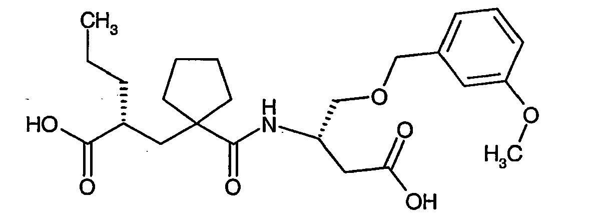

Sodium hydroxide solution (1ml, 1N, 1mmol) was added to a solution of the ethyl ester from example 18 (50mg, 0.11 mmol) in dioxan (3ml), and the reaction stirred at room temperature for 2 hours. The reaction was concentrated under reduced pressure, the residue dissolved in water, acidified using 2N hydrochloric acid, and extracted into ethyl acetate. The combined organic extracts were dried (MgSO₄) and evaporated under reduced pressure to afford the title compound, 30mg; ¹H NMR (CDCl₃, 400MHz) δ: 0.86 (t, 3H), 1.23-1.66 (m, 10H), 1.78 (m, 1H), 1.85 (m, 1H), 1.98 (dd, 1H), 2.14 (m, 1H), 2.39 (m, 1H), 2.72 (d, 2H), 3.58 (m, 2H), 3.80 (s, 3H), 4.46 (m, 3H), 6.58 (d, 1H), 6.84 (m, 3H), 7.23 (m, 1H); LRMS : m/z (ES⁻) 448 [M-H]⁻.

### Examples 33 to 40

The compounds of the following general structure: 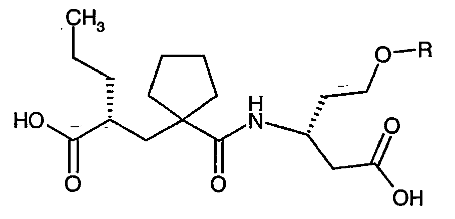 were prepared from the corresponding ethyl esters, following a similar procedure to that described in Example 32.

| Ex. No | R | Data |
|---|---|---|
| 33 | 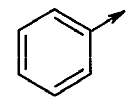 | ¹H-NMR (CDCl₃, 400MHz) δ: 0.84 (t, 3H), 1.30-1.74 (m, 9H), 1.90 (m, 3H), 2.17 (m, 4H), 2.37 (m, 1H), 2.78 (m, 2H), 4.02 (m, 2H), 4.56 (m, 1H), 6.59 (d, 1H), 6.90 (m, 2H), 6.99 (m, 1H), 7.24 (m, 2H). LRMS : m/z (ES⁻) 418 [M-H]⁻ |
| 34 | 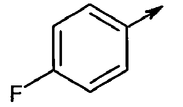 | ¹H-NMR (CDCl₃, 400MHz) δ: 0.84 (t, 3H), 1.24-1.70 (m, 9H), 1.86 (m, 3H), 2.16 (m, 4H), 2.34 (m, 1H), 2.70 (dd, 1H), 2.80 (dd, 1H), 3.99 (dd, 2H), 4.50 (m, 1H), 6.68 (d, 1H), 6.81 (m, 2H), 6.98 (m, 2H). LRMS : m/z (ES⁺) 460 [MNa⁺] |
| 35 | 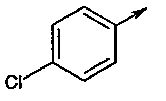 | ¹H-NMR (CDCl₃, 400MHz) δ: 0.86 (t, 3H), 1.21-1.74 (m, 9H), 1.86 (m, 3H), 2.16 (m, 4H), 2.27 (m, 1H), 2.70 (dd, 1H), 2.82 (dd, 1H), 3.98 (dd, 2H), 4.54 (m, 1H), 6.72 (d, 1H), 6.81 (d, 2H), 7.22 (d, 2H). LRMS : m/z (ES⁺) 454 [MH⁺] |
| 36 | 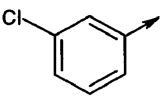 | ¹H-NMR (CDCl₃, 400MHz) δ: 0.85 (t, 3H), 1.22-1.77 (m, 9H), 1.84 (m, 3H), 2.17 (m, 4H), 2.29 (m, 1H), 2.70 (dd, 1H), 2.81 (dd, 1H), 4.00 (dd, 2H), 4.55 (m, 1H), 6.76 (m, 2H), 6.86 (s, 1H), 6.94 (d, 1H), 7.18 (dd, 1H). LRMS : m/z (ES⁻) 452 [M-H]⁻ |
| 37 | 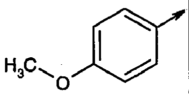 | ¹H-NMR (CDCl₃, 400MHz) δ: 0.84 (t, 3H), 1.20-1.99 (m, 13H), 2.16 (m, 3H), 2.34 (m, 1H), 2.74 (m, 2H), 3.77 (s, 3H), 4.02 (m, 2H), 4.40 (m, 1H), 6.76 (d, 1H), 6.80 (s, 4H). |
| 38 | 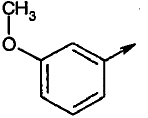 | ¹H-NMR (CDCl₃, 400MHz) δ: 0.85 (t, 3H), 1.22-1.74 (m, 10H), 1.96 (m, 3H), 2.17 (m, 3H), 2.32 (m, 1H), 2.70 (dd, 1H), 2.80 (dd, 1H), 3.79 (s, 3H), 4.00 (m, 2H), 4.50 (m, 1H), 6.46 (m, 3H), 6.78 (d, 1H), 7.18 (dd, 1H); HRMS : m/z (ES⁺) 450.2488 [MH⁺]; C₂₄H₃₅NO₇ gives 450.2487 |
| 39 | 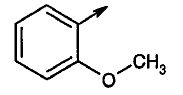 | ¹H-NMR (CDCl₃, 400MHz) δ: 0.83 (t, 3H), 1.22-1.39 (m, 3H), 1.41-1.65 (m, 7H), 1.76 (dd, 1H), 1.90-2.21 (m, 5H), 2.37 (m, 1H), 2.71 (dd, 1H), 2.79 (dd, 1H), 3.83 (s, 3H), 4.08 (m, 2H), 4.50 (m, 1H), 6.90 (m, 4H), 7.02 (d, 1H), 8.84 (br s, 2H); HRMS : m/z (ES⁺) 450.2490 [MH⁺] C₂₄H₃₅NO₇ gives 450.2487 |
| 40 | 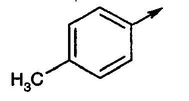 | ¹H-NMR (CDCl₃, 400MHz) δ: 0.83 (t, 3H), 1.22-1.72 (m, 10H), 1.86 (m, 4H), 2.17 (m, 4H), 2.30 (m, 4H), 2.75 (m, 2H), 4.00 4.50 (m, 1H), 6.78 (m, 3H), 7.05 (d, 2H); LRMS : m/z (ES⁺) 456 [MNa⁺] |

### Example 41

### (3S)-3-[({1-[(2R)-2-Carboxypentyl]cyclopentyl}carbonyl)amino]-5-(4-chlorophenoxy)pentanoic acid

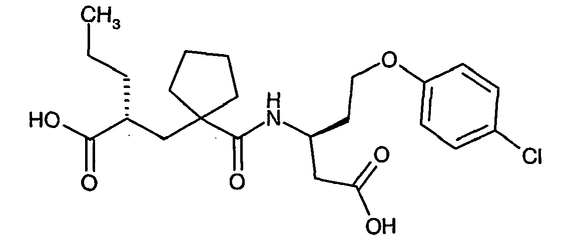

A mixture of the ethyl ester from preparation 86 (180mg, 0.37mmol) and sodium hydroxide solution (2N, 2ml) in dioxan (5ml) was stirred at 50°C for 5 hours, then at room temperature for a further 18 hours. The mixture was concentrated under reduced pressure, the residue acidifed using 2N hydrochloric acid, and extracted with ethyl acetate (2x20ml). The combined organic solutions were dried (MgSO₄), concentrated under reduced pressure and the residue purified by column chromatography on silica gel using an elution gradient of dichloromethane:methanol:acetic acid (95:5:0 to 90:10:0 to 90:10:1) to afford the title compound as a colourless oil, 150mg. ¹H-NMR (CDCl₃, 400MHz) δ: 0.84 (t, 3H), 1.26 (m, 3H), 1.40-1.80 (m, 6H), 1.97 (m, 2H), 2.10 (m, 5H), 2.37 (m, 1H), 2.61 (dd, 1H), 2.78 (dd, 1 H), 4.00 (m, 2H), 4.42 (m, 1H), 6.80 (d, 2H), 6.98 (d, 1H), 7.20 (d, 2H). LRMS : m/z (ES⁻) 452 [M-H⁻]

### Example 42

### (3S)-3-[({1-[(2R)-2-Carboxypentyl]cyclopentyl}carbonyl)amino]-5-(4-fluorophenoxy)-pentanoic acid

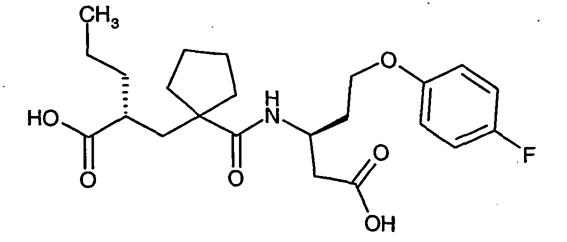

A mixture of the ester from preparation 87 (130mg, 0.28mmol) in sodium hydroxide solution (3ml, 2N) and dioxan (6ml) was stirred at 50°C for 3 hours. The reaction mixture was concentrated under reduced pressure, the residue acidifed using 2N hydrochloric acid and extracted with ethyl acetate (3x50ml). The crude product was purified by column chromatography on silica gel using an elution gradient of methanol:dichloromethane (5:95 to 10:90) to afford the title compound as a colourless oil, 58mg; ¹H-NMR (CDCl₃+1 drop TFAd, 400MHz) δ: 0.83 (t, 3H), 1.23 (m, 2H), 1.39 (m, 1H), 1.55-1.78 (m, 8H), 1.82 (m, 1H), 1.98 (m, 1H), 2.16 (m, 3H), 2.38 (m, 1H), 2.79 (m, 2H), 4.05 (t, 2H), 4.58 (m, 1H), 6.82 (m, 2H), 7.00 (m, 2H), 7.18 (d, 1H); LRMS : m/z (ES⁻) 436 [M-H]⁻; Microanalysis found: C, 59.87; H, 7.11; 3.02. C₂₃H₃₂FNO₆;0.4CH₂Cl₂ requires C, 59.61; H, 7.01; N, 2.97%.

### Examples 43 to 45

The following examples of general formula: 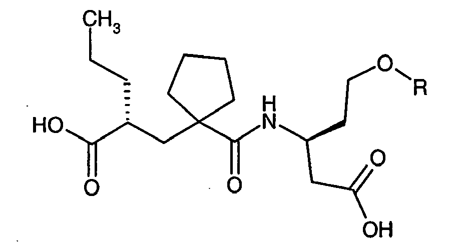 were prepared from the corresponding ethyl esters following a similar procedure to that described in Example 42.

| Ex. No | R | Data |
|---|---|---|
| 43 | 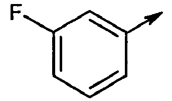 | ¹H-NMR (CDCl₃, 400MHz) δ: 0.82 (t, 3H), 1.24 (m, 3H), 1.40-1.80 (m, 8H), 1.98 (m, 2H), 2.16 (m, 3H), 2.37 (m, 1H), 2.62 (m, 1H), 2.79 (m, 1H), 4.02 (m, 2H), 4.43 (m, 1H), 6.62 (m, 3H), 7.01 (m, 1H), 7.20 (m, 1H); LRMS : m/z (ES⁺) 460 [MNa]⁺; [α]_{D} = -17.15 (c = 0.07, methanol) |
| 44^{a} | 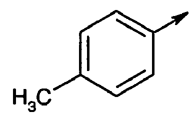 | ¹H-NMR (CDCl₃, 400MHz) δ: 0.84 (t, 3H), 1.26 (m, 3H), 1.42-1.70 (m, 7H), 1.78 (dd, 1H), 1.98 (m, 2H), 2.16 (m, 3H), 2.26 (s, 3H), 2.39 (m, 1H), 2.60 (dd, 1H), 2.80 (dd, 1H), 4.02 (m, 2H), 4.42 (m, 1H), 6.78 (d, 2H), 7.04 (m, 3H); LRMS : m/z (ES⁻) 432 [M-H]⁻ |
| 45^{a} | 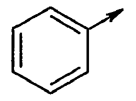 | ¹H-NMR (CDCl₃, 400MHz) δ: 0.84 (t, 3H), 1.26 (m, 3H), 1.42-1.80 (m, 8H), 1.98 (m, 3H), 2.18 (m, 2H), 2.38 (m, 1H), 2.62 (dd, 1H), 2.80 (dd, 1H), 4.04 (m, 2H), 4.42 (m, 1H), 6.85 (d, 2H), 6.97 (m, 1H), 7.04 (d, 1H), 7.25 (d, 2H); LRMS : m/z (ES⁺) 420 [MH⁺]; [α]_{D} = -19.20 (c = 0.10, methanol) |

| | | |
|---|---|---|
| a-product purified by reverse phase HPLC using acetonitrile:water:trifluoroacetic acid as eluant | | |

### Example 46

### (3R)-3-[({1-[(2S)-2-Carboxy-4-methoxybutyl]cyclopentyl}carbonyl)amino]-5-(4-chlorophenoxy)pentanoic acid

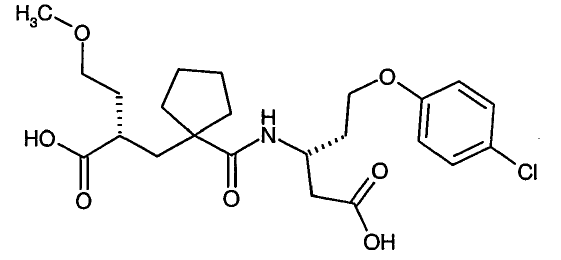

A solution of the ester from preparation 80 (295mg, 0.51 mmol) in trifluoroacetic acid (1ml) and dichloromethane (4ml) was stirred at room temperature for 4 hours. The solution was concentrated under reduced pressure and then azeotroped with toluene. The residue was partitioned between saturated sodium bicarbonate solution and dichloromethane, the layers separated and the aqueous extracted with dichloromethane. The pH of the aqueous solution was adjusted to 1 using hydrochloric acid (2N), and this was then extracted with dichloromethane (3x). These combined organic extracts were dried (MgSO₄) and evaporated under reduced pressure to afford the title compound as a colourless solid, 134mg; ¹H-NMR (CDCl₃, 400MHz) δ: 1.40-1.74 (m, 7H), 1.80-1.99 (m, 4H), 2.08 (m, 3H), 2.41 (m, 1H), 2.63 (dd, 1 H), 2.78 (dd, 1 H), 3.24 (s, 3H), 3.38 (m, 2H), 3.98 (s, 2H), 4.55 (m, 1 H), 6.62 (m, 1 H), 6.78 (d, 2H), 7.20 (d, 2H); LRMS : m/z (ES⁻) 468 [M-H⁻]; Microanalysis found: C, 57.76; H, 6.88; N, 3.03. C₂₃H₃₂ClNO₇;0.5H₂O requires C, 57.68; H, 6.94; N, 2.92%.

### Example 47

### Ethyl (3S)-3-[({1-[(2S)-2-Carboxy-4-methoxybutyl]cyclopentyl}carbonyl)amino]-5-(4-chlorophenoxy)pentanoate

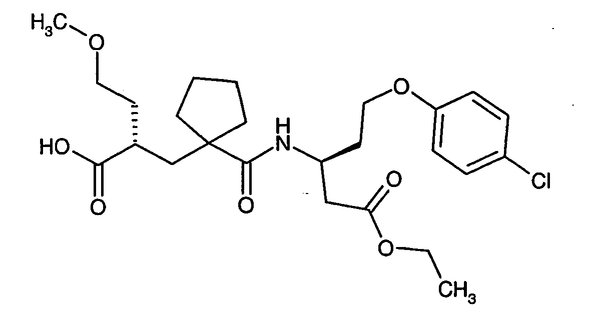

A solution of the ester from preparation 81 (300mg, 0.54mmol) and trifluoroacetic acid (5ml) in dichloromethane (5ml) was stirred at room temperature for 2 hours. The mixture was concentrated under reduced pressure and the residue azeotroped with toluene. The crude product was purified by column chromatography on reverse phase silica gel using an elution gradient of acetonitrile:water:trifluoroacetic acid (5:95:0.1 to 95:5:0) to afford the title compound as a colourless oil, 109mg; ¹H NMR (CDCl₃, 400MHz) δ: 1.28 (t, 3H), 1.54-1.75 (m, 8H), 1.98 (m, 3H), 2.06 (m, 3H), 2.50 (m, 1 H), 2.63 (m, 2H), 3.25 (s, 3H), 3.38 (m, 2H), 4.00 (t, 2H), 4.18 (q, 2H), 4.43 (m, 1H), 6.81 (m, 3H), 7.22 (d, 2H); LRMS : m/z (ES⁻) 496 [M-H]⁻.

### Example 48

### (3S)-3-[({1-[(2S)-2-Carboxy-4-methoxybutyl]cyclopentyl}carbonyl)amino]-5-(4-chlorophenoxy)pentanoic acid

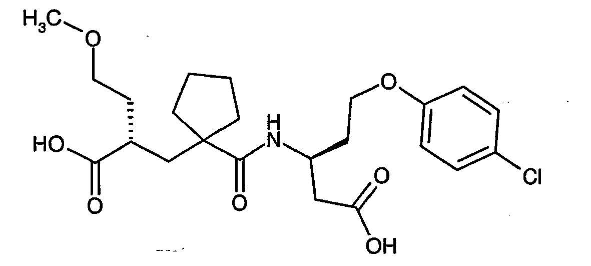

A solution of the ethyl ester from example 47 (100mg, 0.20mmol) and 2N sodium hydroxide solution (2ml) in dioxan (4ml) was stirred at 50°C for 3 hours, then concentrated under reduced pressure. The residue was suspended in 2N hydrochloric acid (2ml), extracted with ethyl acetate (3x20ml), and the combined organic solutions dried (MgSO₄) and evaporated under reduced pressure.The crude product was purified by column chromatography on silica gel using an elution gradient of ethyl acetate:methanol:acetic acid (90:10:0 to 90:10:1) to afford the title compound as a colourless oil, 50mg; ¹H NMR (CDCl₃, 400MHz) δ: 1.45-1.78 (m, 8H), 1.85-2.19 (m, 6H), 2.56 (m, 1 H), 2.62 (m, 2H), 3.37 (m, 4H), 3.46 (m, 1 H), 4.00 (t, 2H), 4.62 (m, 1 H), 6.64 (d, 1 H), 6.81 (d, 2H), 7.22 (d, 2H); LRMS : m/z (ES⁻) 468 [M-H]⁻.

### Example 49

### (2S)-2-({1-[({(1S)-3-Butoxy-1-[2-(4-chlorophenoxy)ethyl]-3-oxopropyl}amino)carbonyl]-cyclopentyl}methyl)-4-methoxybutanoic acid

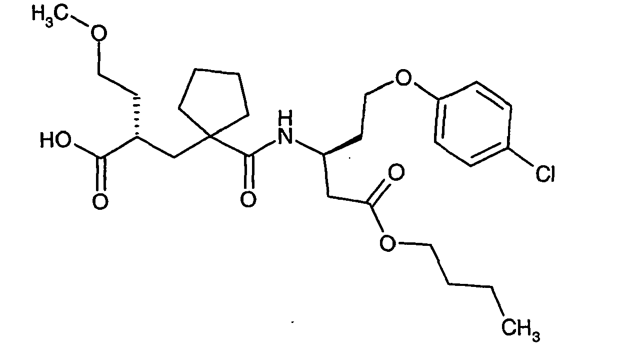

A solution of the ester from preparation 92 (278mg, 0.48mmol) and trifluoroacetic acid (3ml) in dichloromethane (5ml) was stirred at room temperature for 2 hours. The mixture was concentrated under reduced pressure and the residue azeotroped with toluene. The crude product was purified by column chromatography on silica gel using an elution gradient of ethyl acetate:pentane (30:70 to 50:50) to afford the title compound as a colourless oil, 229mg; ¹H NMR (CDCl₃, 400MHz) δ: 0.95 (t, 3H), 1.40 (m, 2H), 1.45-1.75 (m, 10H), 1.95 (m, 3H), 2.05 (m, 3H), 2.50 (m, 1H), 2.65 (m, 2H), 3.25 (s, 3H), 3.35 (m, 2H), 4.00 (t, 2H), 4.05 (t, 2H), 4.45 (m, 1 H), 6.80 (m, 3H), 7.20 (d, 2H); Microanalysis found: C, 60.39; H, 7.50; N, 2.49. C₂₇H₄₀ClNO₇;0.6H₂O requires C, 60.40; H, 7.74; N, 2.61 %; [α]_{D} = -14.8 (c = 0.17, methanol)

### Example 50

### (3S)-3-[({1-[(2R)-2-(Ethoxycarbonyl)pentyl]cyclopentyl}carbonyl)amino]-4-[(3-methoxybenzyl)oxy]butanoic acid

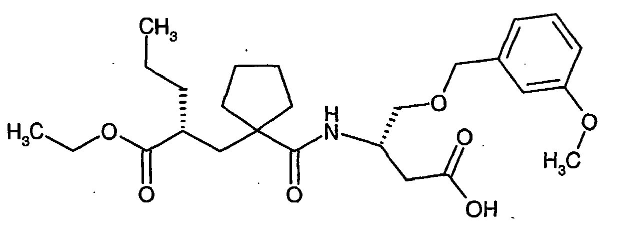

Pyrrolidine (39µl, 0.46mmol) and tetrakis(triphenylphosphine)palladium(0) (10mg) were added to a solution of the allyl ester from preparation 35 (60mg, 0.116mmol) in tetrahydrofuran (2ml), and the reaction stirred at room temperature for 2 hours. The mixture was partitioned between hydrochloric acid (1N) and ethyl acetate, the layers separated, and the organic phase dried (MgSO₄) and concentrated under reduced pressure. The residual orange oil was purified by column chromatography on silica gel using an elution gradient of dichloromethane:methanol:acetic acid (99:1:0 to 97:3:0.3) to afford the title compound as a colourless solid, 15mg; ¹H NMR (CDCl₃, 400MHz) δ: 0.84 (t, 3H), 1.22 (m, 5H), 1.30-1.63 (m, 8H), 1.75 (dd, 1 H), 1.84 (m, 1 H), 1.96-2.07 (m, 2H), 2.35 (m, 1 H), 2.70 (m, 2H), 3.56 (m, 1 H), 3.60 (m, 1 H), 3.80 (s, 3H), 4.09 (m, 2H), 4.40 (m, 1 H), 4.50 (s, 2H), 6.42 (d, 1 H), 6.83 (m, 3H), 7.24 (m, 1 H); LRMS : m/z (ES⁺) 500 [MNa⁺].

### Example 51

### (3S)-4-[(4-Chlorobenzyl)oxyl-3-[({1-[(2S)-2-(ethoxycarbonyl)-4-methoxybutyl]-cyclopentyl}carbonyl)amino]butanoic acid

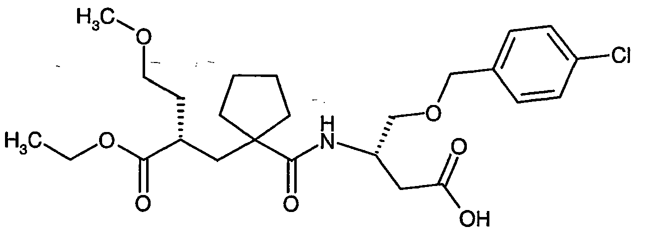

Pyrrolidine (53mg, 0.74mmol) and tetrakis(triphenylphosphine)paladium(0) (20mg) were added to a solution of the allyl ester from preparation 36 (100mg, 0.186mmol) in tetrahydrofuran (4ml), and the reaction stirred at room temperature for 3 hours. The mixture was diluted with hydrochloric acid (1N, 30ml), the aqueous solution extracted with dichloromethane (3x30ml), the combined organic solutions washed with water (50ml), and dried (MgSO₄) and concentrated under reduced pressure. The residual yellow oil was purified by column chromatography on silica gel using an elution gradient of dichloromethane:methanol (98:2 to 92:8) to afford the title compound as a colourless oil, 42mg; ¹H NMR (CDCl₃, 400MHz) δ: 1.22 (t, 3H), 1.41-1.72 (m, 7H), 1.82 (m, 3H), 1.95-2.05 (m, 2H), 2.46 (m, 1H), 2.64 (m, 2H), 3.28 (m, 4H), 3.38 (m, 1 H), 3.46-3.59 (m, 2H), 4.08 (m, 2H), 4.46 (s, 3H), 6.38 (d, 1 H), 7.22 (d, 2H), 7.30 (d, 2H); LRMS : m/z (APCI) 498 [MH⁺]

### Example 52

### Ethyl (3S)-3-[({1-[(2S)-2-carboxy-4-methoxybutyl]cyclopentyl}carbonyl)amino]-6-(4-chlorophenyl)hexanoate

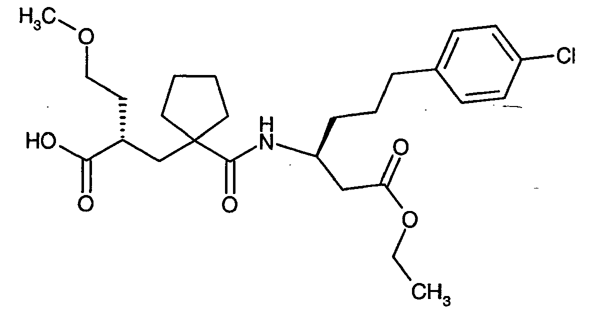

A solution of the diester from preparation 97 (200mg, 0.36mmol) in dichloromethane (15ml) and trifluoroacetic acid (6ml) was stirred at room temperature for 18 hours. The mixture was concentrated under reduced pressure and the residue partitioned between dichloromethane (75ml) and water (75ml). The layers were separated, the organic phase dried (MgSO₄) and evaporated under reduced pressure to afford the title compound, as a colourless oil, 180mg; ¹H NMR (CDCl₃, 400MHz) δ: 1.22 (t, 3H), 1.44-1.78 (m, 13H), 1.90-2.00 (m, 3H), 2.42-2.62 (m, 5H), 3.22 (s, 3H), 3.37 (m, 2H), 4.15 (q, 2H), 4.26 (m, 1 H), 6.80 (d, 1H), 7.08 (d, 2H), 7.22 (d, 2H).

### Example 53

### (3S)-3-[({1-[(2S)-2-carboxy-4-methoxybutyl]cyclopentyl}carbonyl)amino]-6-(4-chlorophenyl)hexanoic acid

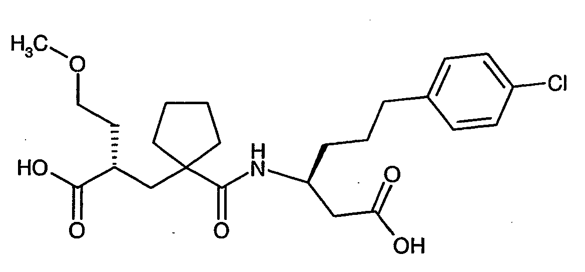

A solution of the ester from example 52 (60mg, 0.12mmol) in dioxan (4ml) and 1N sodium hydroxide (4ml) was stirred at room temperature for 5 hours. The mixture was concentrated under reduced pressure and the residue partitioned between water (20ml) and ethyl acetate (20ml). The layers were separated, the aqueous acidified to pH 1 using hydrochloric acid, and this soluton then re-extracted with ethyl acetate (2x20ml). These combined organic extracts were dried (MgSO₄) and evaporated under reduced pressure to afford the title compound, 49mg; ¹H NMR (CDCl₃, 400MHz) δ: 1.42-1.78 (m, 10H), 1.97 (m, 3H), 2.16 (m, 1 H), 2.40-2.70 (m, 6H), 3.37 (m, 5H), 3.44 (m, 1 H), 4.42 (m, 1 H), 6.38 (d, 1 H), 7.07 (d, 2H), 7.21 (d, 2H).

### Example 54

### (3S)-3-[({1-[(2S)-2-carboxy-4-methoxybutyl]cyclopentyl}carbonyl)amino]-6-(4-methoxyphenyl)hexanoic acid

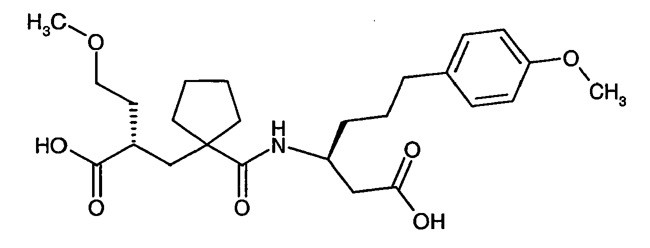

A solution of the ester from preparation 98 (17mg, 0.03mmol) in trifluoroacetic acid (0.5ml) and dichloromethane (2ml) was stirred at room temperature for 18 hours. The reaction was concentrated under reduced pressure and the residue partitioned between dichloromethane and water, and the layers separated. The organic phase was dried (MgSO₄) and evaporated under reduced pressure. The product was purified by Fraction-Lynx® reverse phase HPLC using a Phenomenex Luna C18 column and acetonitrile:water:trifluoroacetic acid (5:95:0.1 to 95.25:4.75:0.005) as eluant to give the title compound; ¹H NMR (CD₃OD, 400MHz) δ: 1.40-1.86 (m, 12H), 1.97-2.16 (m, 4H), 2.38-2.62 (m, 5H), 3.22 (s, 3H), 3.50 (m, 2H), 3.76 (s, 3H), 4.30 (m, 1H), 6.80 (d, 2H), 7.06 (d, 2H); LRMS: m/z (ES⁻) 462 [M-H]⁻.

### Preparation 1

### 1-[(2S)-2-(tert-Butoxycarbonyl)-4-methoxybutyl]cyclopentanecarboxylic acid

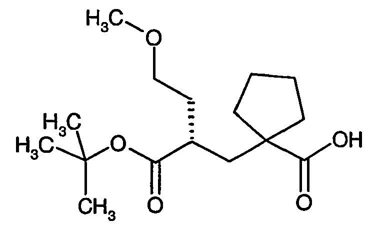

To a solution of 1-[2-(*tert*-butoxycarbonyl)-4-methoxybutyl]cyclopentane carboxylic acid (WO 0202513) (3.90 kg, 13.0 mol) in heptane (58.5 L, total solution weight 44.0 kg) was added (1*S*, 2*S*)-(+)-pseudoephedrine (2.13 kg, 12.9 mol) under an atmosphere of nitrogen at 20 °C. The suspension was then heated to 70 °C with stirring until a clear solution was obtained. The solution was then cooled to 40 °C and a sample of authentic crystallised title compound (0.8 g) was added to seed the crystallisation. The temperature of the mixture was maintained at 40 °C for 2 hours and then the slurry was cooled to 20 °C over 6 hours. The product was then collected by filtration and was washed with heptane (2 x 2.3 L) then dried under vacuum for 22 hours at 50 °C to give (1*S*, 2*S*)-1-hydroxy-*N*-methyl-1-phenyl-2-propanaminium 1-[(2*S*)-2-(*tert*-butoxy carbonyl)-4-methoxybutyl]cyclopentane carboxylate (3.20 kg, 6.87 mol, 53% yield as an 86:14 mixture of diastereoisomeric salts as measured by ¹H NMR). The product (3.20 kg, 6.87 mol) was then suspended in heptane (30 L) and heated to 70 °C until a clear solution was obtained. The resultant solution was then cooled to 58 °C and a sample of authentic crystallised title compound (1.0 g) was added to seed the crystallisation. The solution was then held at 58 °C for 1 hour and was then cooled to 20 °C over 6 hours. The slurry was then granulated at 20 °C for 12 hours. The product was collected by filtration and was washed with heptane (2 x 2 L). Drying in a vacuum oven at 50 °C for 22.5 hours gave (1*S*, 2*S*)-1-hydroxy-*N*-methyl-1-phenyl-2-propanaminium 1-[(2*S*)-2-(*tert-*butoxycarbonyl)-4-methoxybutyl]cyclopentane carboxylate as a white crystalline solid (2.35 kg, 5.0 mol, 73% yield). m.p. (heptane); 95 °C; ¹H-NMR (CDCl₃, 300 MHz), □: 1.08 (d, 3 H), 1.48 (s, 10 H), 1.56-1.74 (m, 4 H), 1.74-1.90 (m,2H), 1.90-2.03 (m,2H), 2.03-2.27 (m,2H), 2.4-2.53 (m, 1 H), 2.66 (s, 3 H), 3.08 (dq, 1 H), 3.24 (s, 3 H), 3.38 (q, 2 H), 4.58 (d, 1 H), 7.27-7.45 (m, 5 H), 7.70 (s, br, 3 H); Anal. found C, 67.06; H, 9.35; N, 3.04; C₂₆H₄₃NO₆ requires C, 67.07; H, 9.31; N, 3.01 %. The title compound was obtained by breaking the salt as follows. To a stirred suspension of (1 *S*, 2*S*)-1-hydroxy-*N*-methyl-1-phenyl-2-propanaminium 1-[(2*S*)-2-(*tert*-butoxycarbonyl)-4-methoxybutyl]cyclopentane carboxylate (210 g, 0.45 mol) in deionised water (1.26 L) and isopropyl acetate (1.47 L) was added aqueous hydrochloric acid (99.5 ml of a 5 M solution, 0.50 mol) until the pH of the aqueous layer was between pH 2 and 3. The layers were then separated, and the aqueous phase was extracted with isopropyl acetate (630 ml). The organic extracts were then combined and washed with saturated brine solution (420 ml). The organic phase was then concentrated by distillation at atmospheric pressure (to remove 1.4 L of isopropyl acetate) to the title compound as a solution in isopropyl acetate which was used directly in the next step. An aliquot can be taken and the solvent removed to give an analytical sample; ¹H NMR (CDCl₃ 300MHz) δ: 1.44 (s, 9 H), 1.48-1.59 (m,2H), 1.59-1.72 (m, 5 H), 1.72-1.93 (m,2H), 2.03-2.18 (m, 3 H), 2.35-2.46 (m, 1 H), 3.31 (s, 3 H), 3.38 (t,2H); LRMS (EI): m/z 244 [M-C₄H₈]⁺, 227 [M-C₄H₉O]⁺, 199 [M-C₄H₉O₂C]⁺; GC (injector program: initial temp. 0 °C, rate 150 °C/min; final temp. 230 °C; oven program: initial temp. 100 °C, rate 10 °C/min, final temp. 230 °C, final time 20 min; column, BP-21 25 m x 0.25mm ID x 0.25um FT; detection FID) Retention Time 16.0 min; HPLC (column: ChiralPak AD (25 x 0.46 cm); mobile phase: hexane/IPA/acetic acid (98/2/0.1 v/v/v); Rinsing mobile phase: hexane/IPA/DEA (80/20/.5 v/v/v); flow rate: 1.0 ml/min; temperature: ambient; injection volume: 20 µl; detection: ELSD) Run time: 20 mins followed by 10 mins rinse with hexane/IPA/DEA (80/20/.5 v/v/v), followed by 10 mins rinse with hexane/IPA/acetic acid (98/2/0.1 v/v/v); Retention Time: minor enantiomer 15.5 min (3.3%), major enantiomer 17.5 min (96.7%).

### Preparation 2

### tert-Butyl (2R)-2-{[1-({[(3S)-5-oxotetrahydro-3-furanyl]amino}carbonyl)-cyclopentyl]methyl}pentanoate

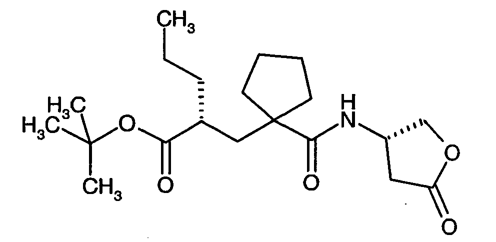

A mixture of 3(*S*)-amino-γ-butyrolactone hydrobromide (J.A.C.S; 1986; 108(16), 4943) (7g, 38mmol), 1-[(2*R*)-2-(*tert*-butyoxycarbonyl-pentyl)]-cyclopentanecarboxylic acid (WO 0202513) (11.4g, 40mmol), 1-(3-dimethylaminopropyl)-3-ethylcarbodiimide hydrochloride (7.7g, 40mmol), 1-hydroxybenzotriazole hydrate (5.4g, 40mmol) and 4-methylmorpholine (22ml, 200mmol) in dichloromethane (200ml) was stirred at room temperature for 18 hours. The mixture was washed with water, hydrochloric acid (2N), then dried (MgSO₄) and concentrated under reduced pressure. The crude product was purified by column chromatography on silica gel using an elution gradient of dichloromethane:methanol (99:1 to 98:2) to afford the title compound as a colourless oil, 12.6g; ¹H NMR (CDCl₃, 400MHz) δ: 0.85 (t, 3H), 1.27 (m, 3H), 1.40-1.68 (m, 17H), 1.88-2.14 (m, 3H), 2.19 (m, 1 H), 2.54 (dd, 1H), 2.84 (dd, 1 H), 4.19 (dd, 1 H), 4.54 (dd, 1H), 4.66 (m, 1 H), 6.43 (d, 1 H); LRMS : m/z (ES⁺) 390 [MNa⁺]; Microanalysis found: C, 62.28; H, 9.26; N, 3.54. C₂₀H₃₃NO₅ requires C, 62.31; H, 9.15; N, 3.63%.

### Preparation 3

### tert-Butyl (2R)-2-{[1-({[(3R)-5-oxotetrahydro-3-furanyl]amino}carbonyl)cyclopentyl]-methyl}pentanoate

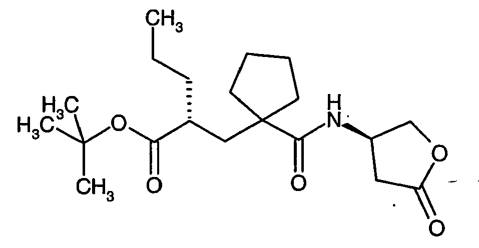

3(*R*)-Amino-γ-butyrolactone hydrobromide (US 5252747, compound 25) (6.2g, 34mmol) was added to a solution of 1-[(2*R*)-2-(*tert*-butyoxycarbonyl-pentyl)]-cyclopentanecarboxylic acid (WO 0202513, preparation 2) (9.9g, 34.8mmol), 1-(3-dimethylaminopropyl)-3-ethylcarbodiimide hydrochloride (8.65g, 44.3mmol), 1-hydroxybenzotriazole hydrate (6.22g, 41.8mmol) and 4-methylmorpholine (14g, 138mmol) in dichloromethane (200ml) and the reaction was stirred at room temperature for 72 hours. The mixture was concentrated under reduced pressure and the residue partitioned between ethyl acetate and brine. The organic phase was dried (MgSO₄) and concentrated under reduced pressure. The crude product was purified by column chromatography on silica gel using an elution gradient of dichloromethane:methanol (99:1 to 90:10). The product was triturated with a solution of ether:pentane (20:80) and the resulting precipitate filtered and dried to afford the title compound, 6.3g.
¹H NMR (CDCl₃, 400MHz) δ: 0.86 (t, 3H), 1.20-1.39 (m, 3H), 1.40-1.75 (m, 17H), 1.90 (m, 1H), 1.99 (dd, 1H), 2.06 (m, 1H), 2.20 (m, 1H), 2.44 (dd, 1H), 2.84 (dd, 1 H), 4.22 (dd, 1H), 4.56 (dd, 1H), 4.66 (m, 1H), 6.22 (d, 1H); LRMS : m/z (ES⁺) 390 [MNa⁺]; [α]_{D} = +21.9 (c = 0.104, methanol).

### Preparation 4

### tert-Butyl (2S)-4-methoxy-2-{[1-({[(3S)-5-oxotetrahydro-3-furanyl]amino}carbonyl)-cyclopentyl]methyl}butanoate

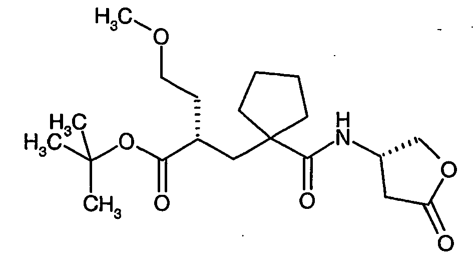

The title compound was obtained as a colourless oil in 82% yield, from the acid from preparation 1 and 3-(*S*)-amino-γ-butyrolactone hydrobromide (J.A.C.S; 1986; 108(16), 4943) following a similar procedure to that described in preparation 2; ¹H-NMR (CDCl₃, 400MHz) δ: 1.41 (m, 11H), 1.50-1.80 (m, 7H), 2.01 (m, 3H), 2.35 (m, 1 H), 2.55 (m, 1H), 2.82 (m, 1H), 3.26 (s, 3H), 3.35 (m, 2H), 4.10 (dd, 1 H), 4.52 (m, 1H), 4.65 (m, 1H), 6.46 (br d, 1H); LRMS : m/z (APCI) 384 [MH⁺]; Microanalysis found: C, 61.30; H, 8.55; N, 3.60. C₂₀H₃₃NO₆;0.1CH₂Cl₂ requires C, 61.60; H, 8.40; N, 3.57%.

### Preparation 5

### tert-Butyl (2S)-4-methoxy-2-{[1-({[(3R)-5-oxotetrahydro-3-furanyl]amino}carbonyl)-cyclopentyl]methyl}butanoate

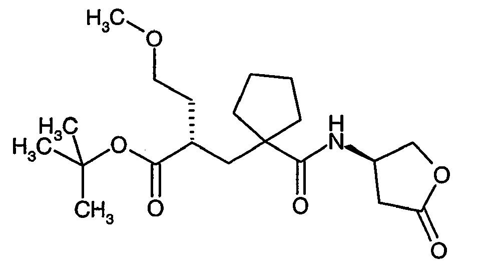

The title compound was obtained as a colourless oil from 3(*R*)-amino-γ-butyrolactone hydrobromide (US 5252747, compound 25) and the acid from preparation 1, following a similar procedure to that described in preparation 4, except the compound was purified by column chromatography on silica gel using an elution gradient of ethyl acetate:pentane (50:50 to 75:25); ¹H-NMR (CDCl₃, 400MHz) δ: 1.42 (m, 11H), 1.58-1.82 (m, 7H), 1.92-2.08 (m, 3H), 2.35 (m, 1H), 2.46 (m, 1H), 2.82 (dd, 1H), 3.34 (m, 5H), 4.22 (dd, 1H), 4.54 (m, 1H), 4.65 (m, 1H), 6.44 (br d, 1H); LRMS : m/z (ES⁺) 406 [MNa⁺].

### Preparation 6

### (3S)-3-[({1-[(2R)-2-(tert-Butoxycarbonyl)pentyl]cyclopentyl}carbonyl)amino]-4-hydroxybutanoic acid

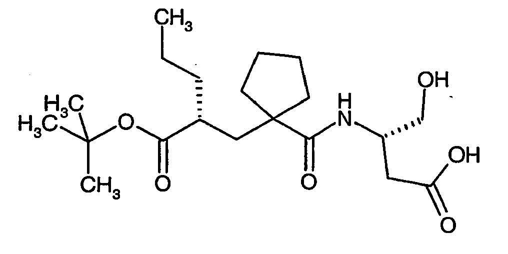

Sodium hydroxide solution (60ml, 1 M, 60mmol) was added to a solution of the lactone from preparation 2 (12.5g, 34mmol) in methanol (200ml), and the solution stirred at room temperature for 18 hours. The mixture was concentrated under reduced pressure and the residue diluted with water. The aqueous solution was washed with ethyl acetate, acidifed with hydrochloric acid (2N), and this solution extracted with ethyl acetate. The combined organic extracts were dried (MgSO₄), and evaporated under reduced pressure to give a colourless oil. This was triturated with ether, the solid filtered and dried, to afford the title compound as a white solid, 7.55g; ¹H NMR (CDCl₃, 400MHz) δ: 0.90 (t, 3H), 1.23-1.40 (m, 3H), 1.42-1.76 (m, 16H), 1.92-2.16 (m, 4H), 2.24 (m, 1H), 2.75 (m, 2H), 3.78 (d, 2H), 4.21 (m, 1H), 6.62 (d, 1 H); LRMS : m/z (ES⁺) 386 [MH⁺]

### Preparation 7

### (3R)-3-[({1-[(2R)-2-(tert-Butoxycarbonyl)pentyl]cyclopentyl}carbonyl)amino]-4-hydroxybutanoic acid

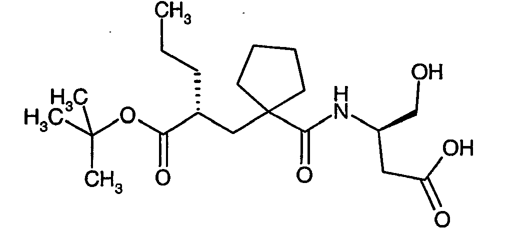

The title compound was obtained from the lactone from preparation 3, following a similar procedure to that described in preparation 6; ¹H NMR (CDCl₃, 400MHz) δ: 0.86 (t, 3H), 1.20-1.38 (m, 3H), 1.40-1.79 (m, 16H), 1.90 (m, 2H), 2.14 (m, 2H), 2.21 (m, 1 H), 2.62 (d, 2H), 3.68 (dd, 1 H), 3.79 (dd, 1 H), 4.32 (m, 1 H), 6.60 (d, 1H).

### Preparation 8

### (3S)-3-[({1-[(2S)-2-(tert-Butoxycarbonyl)-4-methoxybutyl]cyclopentyl}carbonyl)amino]-4-hydroxybutanoic acid

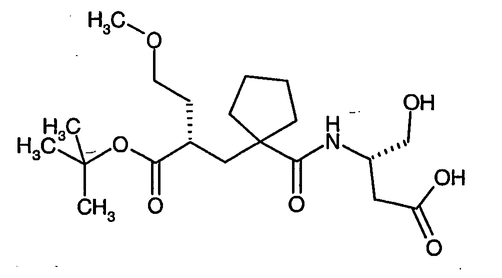

The title compound was obtained from the compound from preparation 4, following a similar procedure to that described in preparation 6; ¹H-NMR (CDCl₃, 400MHz) δ: 1.44 (m, 11 H), 1.58-1.82 (m, 7H), 2.00 (m, 3H), 2.38 (m, 1H), 2.65 (m, 2H), 3.34 (s, 3H), 3.42 (m, 2H), 3.72 (m, 2H), 4.26 (m, 1H), 6.60 (br d, 1 H); LRMS : m/z (APCI) 402 [MH⁺] Microanalysis found: C, 59.71; H, 8.81; N, 3.47. C₂₀H₃₅NO₇ requires C, 59.85; H, 8.73; N, 3.49%.

### Preparation 9

### (3S)-3-[({1-[(2R)-2-(tert-Butoxycarbonyl)-4-methoxybutyl]cyclopentyl}carbonyl)amino]-4-hydroxybutanoic acid

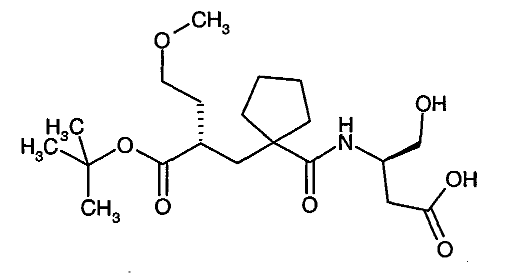

The title compound was obtained as a colourless oil in 64% yield from the lactone from preparation 5, following a similar procedure to that described in preparation 6; ¹H-NMR (CDCl₃, 400MHz) δ: 1.42 (m, 11 H), 1.58-1.82 (m, 7H), 1.92-2.03 (m, 3H), 2.38 (m, 1H), 2.62 (m, 2H), 3.30 (m, 4H), 3.42 (m, 1 H), 3.64 (dd, 1H), 3.78 (dd, 1 H), 4.38 (m, 1H), 6.54 (d, 1H); LRMS : m/z (ES⁺) 424 [MNa⁺].

### Preparation 10

### (3S)-3-[({1-[(2R)-2-(tert-Butoxycarbonyl)pentyl]cyclopentyl}carbonyl)amino]-4-[(3-methoxybenzyl)oxy]butanoic acid

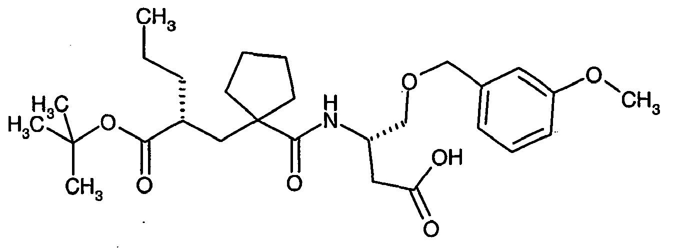

Sodium hydride (60% dispersion in mineral oil, 935mg, 24mmol) was added to a cooled (-15°C) solution of the alcohol from preparation 6 (1 g, 2.6mmol) in tetrahydrofuran (20ml), and the mixture stirred for 1 hour. 3-Methoxybenzyl bromide (862mg, 4.29mmol) and imidazole (52mg, 0.79mmol) were added and the reaction stirred for a further hour at -15°C, and then allowed to warm to room temperature, and stirred for a further 3 hours. The reaction was quenched by the addition of water, tetrahydrofuran was removed *in vacuo* and the residue acidified with 1N hydrochloric acid. This aqueous mixture was extracted with ethyl acetate (3x), the combined organic solutions were dried (MgSO₄) and evaporated under reduced pressure. The crude product was purified by column chromatography on silica gel using an elution gradient of dichloromethane:methanol (99:1 to 96:4) to afford the title compound as a clear oil; ¹H NMR (CDCl₃, 400MHz) δ: 0.84 (t, 3H), 1.20-1.37 (m, 3H), 1.44 (m, 12H), 1.56-1.75 (m, 5H), 1.85-2.03 (m, 3H), 2.20 (m, 1 H), 2.70 (m, 2H), 3.54 (m, 1H), 3.60 (m, 1H), 3.80 (m, 3H), 4.42 (m, 1 H), 4.48 (d, 2H), 6.42 (bd, 1H), 6.64 (m, 3H), 7.23 (m, 1H); LRMS : m/z (ES⁺) 528 [MNa⁺].

### Preparations 11 to 14

The following preparations of general formula: were prepared from the alcohol from preparation 6 and the corresponding benzyl bromides, following a similar procedure to that described in preparation 10.

| Prep. No | R | Data |
|---|---|---|
| 11 | 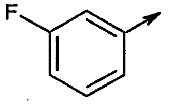 | ¹H NMR (CDCl₃, 400MHz) δ: 0.81 (t, 3H), 1.18-1.70 (m, 20H), 1.88 (m, 2H), 2.00 (m, 1H), 2.19 (m, 1 H), 2.65 (m, 2H), 3.52 (dd, 1 H), 3.59 (dd, 1H), 4.40 (m, 1H), 4.45 (s, 2H), 6.40 (d, 1H), 6.74-7.04 (m, 3H), 7.24 (m, 1 H). LRMS : m/z (ES⁺) 494 [MH⁺] |
| 12 | 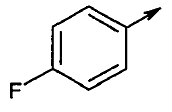 | ¹H NMR (CDCl₃, 400MHz) δ: 0.84 (t, 3H), 1.20-1.77 (m, 20H), 1.98 (m, 3H), 2.21 (m, 1 H), 2.65 (m, 2H), 3.58 (m, 2H), 4.46 (m, 3H), 6.43 (d, 1H), 7.02 (d, 2H), 7.24 (d, 2H). LRMS : m/z (ES⁺) 494.57 [MH⁺] |
| 13^{a} | 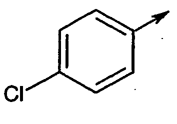 | ¹H NMR (CDCl₃, 400MHz) δ: 0.82 (t, 3H), 1.18-1.70 (m, 19H), 1.82-2.02 (m, 3H), 2.18 (m, 1 H), 2.64 (d, 2H), 3.55 (m, 3H), 4.42 (m, 3H), 6.40 (d, 1H), 7.24 (m, 4H); LRMS : m/z (ES⁺) 532 [MNa⁺]; [α]_{D} = -17.8 (c = 0.1, methanol) |
| 14 | 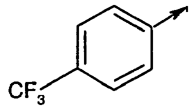 | ¹H NMR (CDCl₃, 400MHz) δ: 0.83 (t, 3H), 1.18-174 (m, 20H), 1.84-2.03 (m, 3H), 2.20 (m, 1H), 2.70 (d, 2H), 3.58 (m, 1H), 3.62 (m, 1H), 4.44 (m, 1H), 4.58 (s, 2H), 6.46 (d, 1H), 7.41 (d, 2H), 7.60 (d, 2H). LRMS : m/z (ES⁺) 566 [MNa⁺] |

| | | |
|---|---|---|
| a= no imidazole was added to the reaction | | |

### Preparation 15

### (3R)-3-[({1-[(2R)-2-(tert-Butoxycarbonyl)pentyl]cyclopentyl}carbonyl)amino]-4-[(4-chlorobenzyl)oxy]butanoic acid

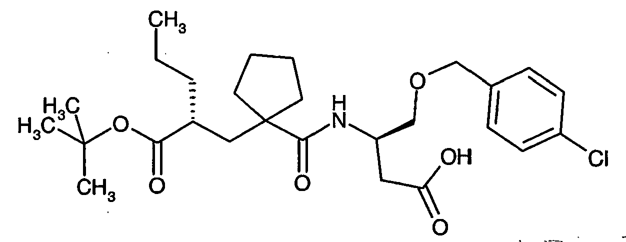

The title compound was obtained in 13% yield from the alcohol from preparation 7 and 4-chlorobenzyl bromide, following a similar procedure to that described in preparation 10; ¹H NMR (CDCl₃, 400MHz) δ: 0.83 (t, 3H), 1.20-1.38 (m, 3H), 1.40-1.75 (m, 17H), 1.84-2.02 (m, 3H), 2.20 (m, 1 H), 2.64 (m, 2H), 3.58 (m, 2H), 4.40-4.56 (m, 3H), 6.45 (d, 1H), 7.23 (d, 2H), 7.30 (d, 2H); LRMS : m/z (ES⁺) 532 [MNa⁺].

### Preparation 16

### (3R)-3-[({1-[(2R)-2-(tert-Butoxycarbonyl)pentyl]cyclopentyl}carbonyl)amino]-4-[(3-methoxybenzyl)oxy]butanoic acid

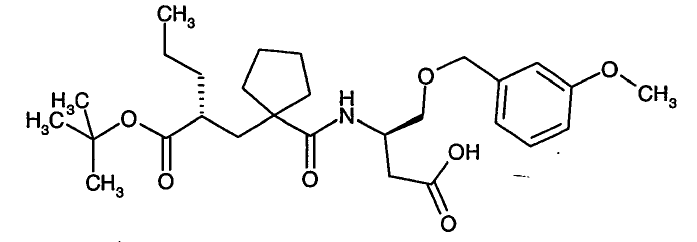

The title compound was obtained in 10% yield from the alcohol from preparation 7 and 3-methoxybenzyl bromide, following a similar procedure to that described in preparation 10; ¹H NMR (CDCl₃, 400MHz) δ: 0.83 (t, 3H), 1.20-1.36 (m, 3H), 1.40-1.74 (m, 17H), 1.88-2.02 (m, 3H), 2.20 (m, 1H), 2.70 (m, 2H), 3.59 (m, 2H), 3.80 (s, 3H), 4.40 (m, 1H), 4.50 (s, 2H), 6.45 (d, 1H), 6.84 (m, 3H), 7.24 (m, 1 H); LRMS : m/z (ES⁺) 528 [MNa⁺].

### Preparation 17

### (3S)-3-[({1-[(2S)-2-(tert-Butoxycarbonyl)-4-methoxybutyl]cyclopentyl}carbonyl)amino]-4-[(3-methoxybenzyl)oxy]butanoic acid

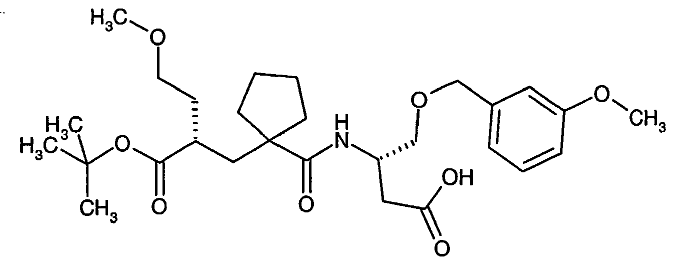

Imidazole (13mg, 0.19mmol) and sodium hydride (60% dispersion in mineral oil, 400mg, 10mmol) were added to a cooled (-15°C) solution of the alcohol from preparation 8 (400mg, 1.0mmol) in tetrahydrofuran (20ml), and the mixture stirred for 45 minutes. A solution of 3-methoxybenzyl bromide (221 mg, 1.1mmol) was added and the reaction stirred for a further 2 hours at -15°C, and then allowed to warm to room temperature. Stirring was continued for a further 3 hours, then the flask cooled in an ice-bath. The reaction was quenched by the addition of water, diluted with 1N hydrochloric acid, and the mixture extracted with ethyl acetate. The combined organic solutions were dried (MgSO₄) and evaporated under reduced pressure. The crude product was purified by column chromatography on silica gel using an elution gradient of dichloromethane:methanol (98:2 to 92:8) to afford the title compound as a colourless oil, 66mg; ¹H NMR (CDCl₃, 400MHz, rotamers) δ: 1.40 (s, 9H),1.45-2.05 (m, 13H), 2.35 (m, 1H), 2.62 (m, 2H), 3.30 (s, 3H), 3.32-3.60 (m, 3H), 3.78 (s, 3H), 4.45 (s, 2H), 4.47 (m, 1H), 6.20, 6.40 (2xd, 1H), 6.83 (m, 3H), 7.22 (d, 1 H); LRMS : m/z (APCI) 522 [MH⁺].

### Preparations 18 to 22

The following preparations of general formula: 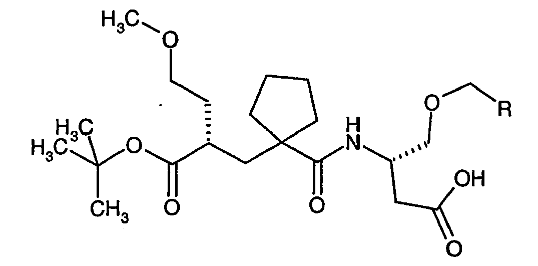 were prepared from the alcohol from preparation 8 and the corresponding benzyl bromides, following a similar procedure to that described in preparation 17.

| Prep. No | R | Data |
|---|---|---|
| 18 | 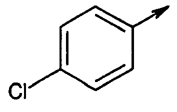 | ¹H NMR (CDCl₃, 400MHz, rotamers) δ: 1.37-1.90 (m, 20H), 2.00 (m, 1 H), 2.38 (m, 1H), 2.62 (m, 2H), 3.32 (m, 4H), 3.40-3.59 (m, 3H), 4.48 (s, 2H), 4.58 (m, 1 H), 6.02, 6.26 (2xd, 1 H), 7.22 (d, 2H), 7.32 (d, 2H); LRMS : m/z (APCI) 526 [MH⁺] |
| 19 | 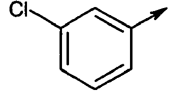 | ¹H NMR (CDCl₃, 400MHz) δ: 1.41 (s, 9H), 1.43-1.80 (m, 8H), 1.86 (m, 3H), 2.01 (m, 1H), 2.38 (m, 1H), 2.70 (d, 2H), 3.35 (m, 4H), 3.40 (m, 1H), 3.61 (m, 2H), 4.59 (m, 3H), 6.36 (d, 1H), 7.23 (m, 2H), 7.37 (m, 1 H), 7.40 (m, 1 H); LRMS : m/z (APCI) 526 [MH⁺] |
| 20 | 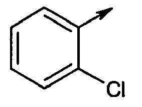 | ¹H NMR (CDCl₃, 400MHz) δ: 1.42 (s, 9H), 1.43-1.78 (m, 8H), 1.83 (m, 3H), 2.00 (m, 1H), 2.38 (m, 1H), 2.64 (d, 2H), 3.30 (m, 4H), 3.40 (m, 1H), 3.54 (m, 2H), 4.46 (d, 2H), 4.56 (m, 1H), 6.24 (d, 1H), 7.17 (m, 1 H), 7.23 (m, 3H); LRMS : m/z (APCI) 526 [MH⁺] |
| 21 | 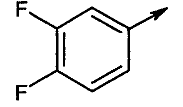 | ¹H NMR (CDCl₃, 400MHz) δ: 1.42 (s, 9H), 1.45-1.80 (m, 9H), 1.85 (m, 3H), 2.01 (m, 1H), 2.39 (m, 1H), 2.65 (d, 2H), 3.35 (s, 3H), 3.40 (m, 1H), 3.57 (m, 2H), 4.45 (s, 2H), 4.57 (m, 1H), 6.28 (d, 1H), 7.00 (m, 1H), 7.15 (m, 2H); LRMS : m/z (ES⁻) 526 [M-H]⁻ |
| 22 | 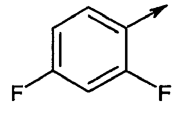 | ¹H NMR (CDCl₃, 400MHz, rotamers) δ: 1.41-1.88 (m, 21 H), 2.00 (m, 1 H), 2.38 (m, 1 H), 2.62 (d, 2H), 3.34, 3.40 (2xm, 4H), 3.56 (m, 2H), 4.56 (m, 3H), 6.00, 6.23 (2xd, 1 H), 6.81 (m, 2H), 7.32 (m, 1 H); LRMS : m/z (ES⁻) 526 [M-H]⁻ |

### Preparation 23

### (3R)-3-[({1-[(2S)-2-(tert-Butoxycarbonyl)-4-methoxybutyl]cyclopentyl}carbonyl)amino]-4-[(4-chlorobenzyl)oxy]butanoic acid

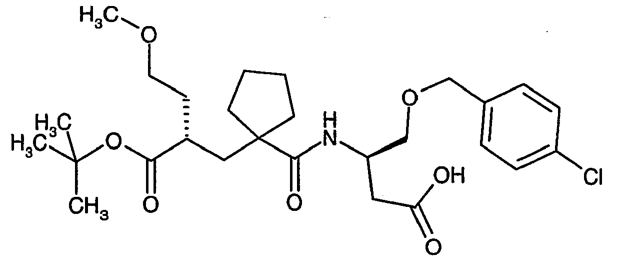

The title compound was obtained as a colourless oil in 30% yield from the alcohol from preparation 9 and 4-chlorobenzyl bromide, following the procedure described in preparation 17; ¹H NMR (CDCl₃, 400MHz) (rotamers) δ: 1.36 (m, 1 H), 1.41-1.88 (m, 18H), 2.01 (m, 1 H), 2.18 (m, 1 H), 2.40 (m, 1 H), 2.62 (m, 2H), 3.34 (m, 4H), 3.50 (m, 3H), 4.47 (s, 2H), 4.59 (m, 1 H), 6.00, 6.24 (2xd, 1 H), 7.22 (d, 2H), 7.30 (d, 2H); LRMS : m/z (ES⁺) 548 [MNa⁺].

### Preparation 24

### tert-Butyl (2R)-2-[(1-{[((1S)-3-ethoxy-1-{[(3-methoxybenzyl)oxy]methyl}-3-oxopropyl)-amino]carbonyl}cyclopentyl)methyl]pentanoate

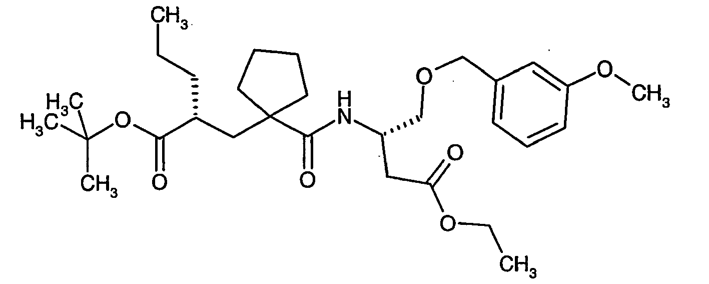

Potassium carbonate (61 mg, 0.45mmol) and iodoethane (40µl, 0.49mmol) were added to a solution of the acid from preparation 10 (275mg, 0.45mmol) in N,N-dimethylformamide (5ml), and the reaction stirred at room temperature for 18 hours. The mixture was diluted with ethyl acetate (20ml), washed with water (x4) and brine, then dried (MgSO₄) and concentrated under reduced pressure. The residue was purified by column chromatography on silica gel using an elution gradient of dichloromethane:methanol (100:0 to 99:1). The residue was azeotroped with xylene, ethyl acetate and dichloromethane to afford the title compound as a colourless oil, 250mg; ¹H NMR (CDCl₃, 400MHz) δ: 0.84 (t, 3H), 1.20-1.27 (m, 6H), 1.44 (m, 11H), 1.57-1.76 (m, 6H), 1.97 (m, 3H), 2.21 (m, 1H), 2.63 (m, 2H), 3.48 (m, 1H), 3.59 (m, 1H), 3.80 (s, 3H), 4.10 (q, 2H), 4.44 (m, 3H), 6.56 (br d, 1H), 6.84 (m, 3H), 7.24 (m, 1H); LRMS : m/z (ES⁺) 556 [MNa⁺].

### Preparation 25

### tert-Butyl (2R)-2-[(1-{[((1S)-1-{[(4-chlorobenzyl)oxy]methyl}-3-ethoxy-3-oxopropyl)amino]-carbonyl}cyclopentyl)methyl]pentanoate

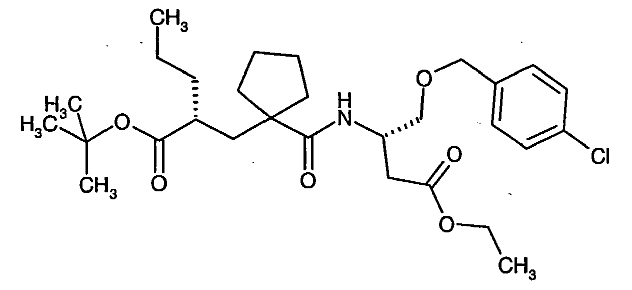

A mixture of the acid from preparation 13 (190mg, 0.37mmol), iodoethane (13mg, 0.84mmol) and potassium carbonate (158.5mg, 1.15mmol) in N,N-dimethylformamide (3ml) was stirred at room temperature for 72 hours. The mixture was poured into water (100ml), and extracted with ethyl acetate (3x100ml). The combined organic extracts were washed with brine (3x100ml), dried (MgSO₄) and evaporated under reduced pressure to afford the title compound as a pale yellow oil, 190mg; ¹H NMR (CDCl₃, 400MHz) δ: 0.82 (t, 3H), 1.18-1.78 (m, 23H), 1.98 (m, 3H), 2.20 (m, 1H), 2.61 (m, 2H), 3.50 (dd, 1H), 3.58 (dd, 1H), 4.10 (q, 2H), 4.42 (m, 3H), 6.54 (d, 1H), 7.24 (d, 2H), 7.30 (d, 2H); HRMS : m/z 538.2942 [MH⁺] C₂₉H₄₄ClNO₆ requires 538.2930; [α]_{D} = -15.8 (c =0.1, methanol).

### Preparation 26

### tert-Butyl (2R)-2-[(1-{[((1S)-3-(allyloxy)-1-{[(3-methoxybenzyl)oxy]methyl}-3-oxopropyl)amino]carbonyl}cyclopentyl)methyl]pentanoate

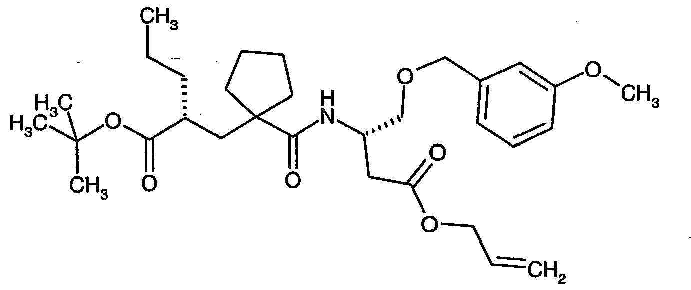

Potassium carbonate (38mg, 0.27mmol) and allyl bromide (27µl, 0.28mmol) were added to a solution of the acid from preparation 10 (140mg, 0.28mmol) in N,N-dimethylformamide (3ml), and the reaction stirred at room temperature for 18 hours. The mixture was partitioned between ethyl acetate and water, the layers separated, and the organic phase washed with water, dried (MgSO₄) and evaporated under reduced pressure to afford the title compound as a colourless oil, 120mg; ¹H NMR (CDCl₃, 400MHz) δ: 0.83 (t, 3H), 1.20-1.37 (m, 3H), 1.44 (m, 11H), 1.58-1.75 (m, 6H), 1.96 (m, 3H), 2.20 (m, 1H), 2.68 (m, 2H), 3.50 (m, 1H), 3.59 (m, 1H), 3.80 (s, 3H), 4.45 (m, 3H), 4.56 (m, 2H), 5.20-5.34 (m, 2H), 5.87 (m, 1H), 6.52 (m, 1H), 6.83 (m, 3H), 7.24 (m, 1H); LRMS : m/z (ES⁺) 568 [MNa⁺].

### Preparation 27

### tert-Butyl (2S)-2-[(1-{[((1S)-1-{[(4-chlorobenzyl)oxy]methyl}-3-ethoxy-3-oxopropyl)amino]-carbonyl}cyclopentyl)methyl]-4-methoxybutanoate

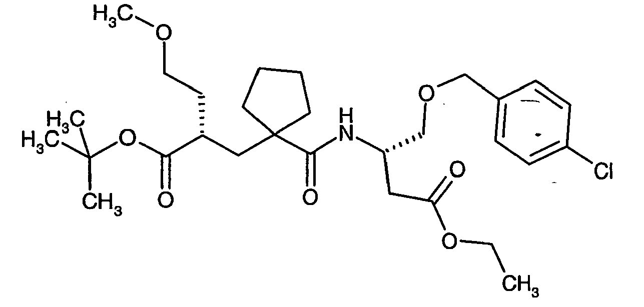

A mixture of the acid from preparation 18 (30mg, 0.06mmol), iodoethane (19mg, 0.12mmol) and potassium carbonate (24mg, 0.17mmol) in N,N-dimethylformamide (2ml) was stirred at room temperature for 18 hours. TLC showed starting material remaining, so additional iodoethane (19mg, 0.12mmol) was added and the reaction stirred at room temperature for a further 3 hours. The reaction was poured into water (30ml), potassium carbonate (50mg) added, and this mixture extracted with ethyl acetate (3x20ml). The combined organic extracts were washed with brine (20ml), dried (MgSO₄) and evaporated under reduced pressure to afford the title compound as a pale yellow oil, 31mg; ¹H NMR (CDCl₃, 400MHz) δ: 1.22 (t, 3H), 1.40-1.82 (m, 18H), 1.97 (m, 3H), 2.38 (m, 1 H), 2.61 (m, 2H), 3.26 (m, 5H), 3.54 (m, 2H), 4.08 (m, 2H), 4.43 (m, 3H), 6.57 (m, 1H), 7.21 (d, 2H), 7.29 (d, 2H); HRMS : m/z (ESI) 554.2887 [MH⁺]; C₂₉H₄₄ClNO₇ requires 554.2879 [MH⁺].

### Preparation 28

### tert-Butyl (2S)-2-[(1-{[((1S)-1-{[(3-chlorobenzyl)oxy]methyl}-3-ethoxy-3-oxopropyl)amino]-carbonyl}cyclopentyl)methyl]-4-methoxybutanoate

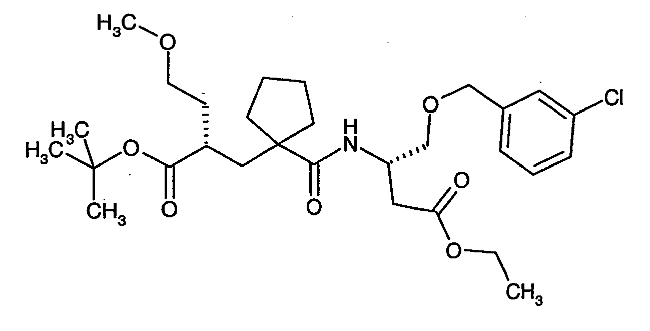

The title compound was obtained as a pale yellow oil, from the acid from preparation 19 and iodoethane, following the procedure described in preparation 27; ¹H NMR (CDCl₃, 400MHz, rotamers) δ: 1.22 (t, 3H), 1.40-1.80 (m, 18H), 1.97 (m, 3H), 2.38, 2.54 (2xm, 1 H), 2.63 (m, 2H), 3.30 (m, 5H), 3.62 (m, 2H), 4.10 (m, 2H), 4.48 (m, 1H), 4.60 (m, 2H), 6.60, 6.70 (2xm, 1H), 7.21 (m, 2H), 7.36 (m, 1H), 7.40 (m, 1H); LRMS : m/z (APCI) 554 [MH⁺].

### Preparation 29

### tert-Butyl (2S)-2-[(1-{[((1S)-1-{[(2-chlorobenzyl)oxy]methyl}-3-ethoxy-3-oxopropyl)amino]-carbonyl}cyclopentyl)methyl]-4-methoxybutanoate

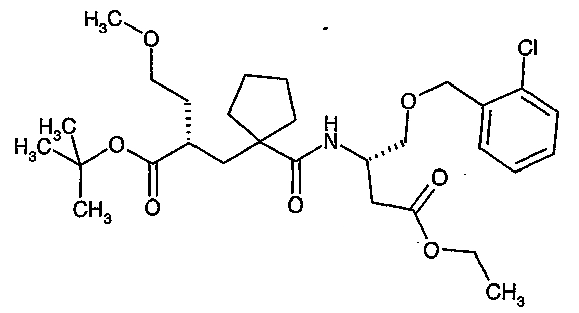

The title compound was obtained as a pale yellow oil, from the acid from preparation 20 and iodoethane, following the procedure described in preparation 27; ¹H NMR (CDCl₃, 400MHz) δ: 1.22 (t, 3H), 1.40 (m, 10H), 1.60 (m, 5H), 1.78 (m, 3H), 1.98 (m, 3H), 2.37 (m, 1 H), 2.63 (m, 2H), 3.30 (m, 5H), 3.58 (m, 2H), 4.10 (q, 2H), 4.44 (m, 3H), 6.58 (d, 1 H), 7.17-7.39 (m, 4H); HRMS : m/z (ESI) 554.2888 [MH⁺] C₂₉H₄₄ClNO₇ requires 554.2879 [MH⁺].

### Preparation 30

### tert-Butyl (2S)-2-[(1-{[((1S)-1-{[(3,4-difluorobenzyl)oxy]methyl}-3-ethoxy-3-oxopropyl}-amino]carbonyl}cyclopentyl)methyl]-4-methoxybutanoate

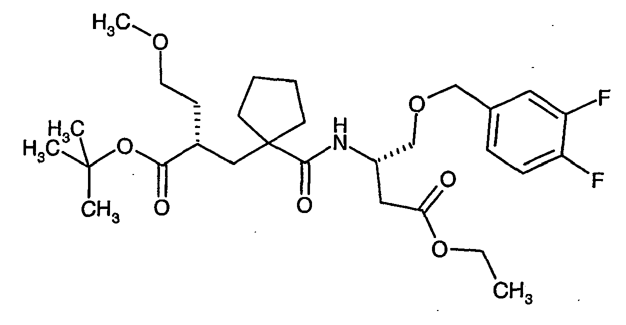

A mixture of the acid from preparation 21 (58mg, 0.11 mmol), iodoethane (21 mg, 0.13mmol) and potassium carbonate (36mg, 0.26mmol) in N,N-dimethylformamide (2ml) was stirred at room temperature for 18 hours. The mixture was diluted with water and extracted with ethyl acetate (3x). The combined organic extracts were washed with brine, dried (MgSO₄) and evaporated under reduced pressure to afford the title compound, 39mg; ¹H NMR (CDCl₃, 400MHz) δ: 1.23 (t, 3H), 1.40-1.82 (m, 18H), 1.98 (m, 3H), 2.37 (m, 1H), 2.63 (m, 2H), 3.24 (s, 3H), 3.32 (m, 2H), 3.50 (m, 1H), 3.58 (m, 1H), 4.10 (q, 2H), 4.42 (m, 3H), 6.58 (d, 1H), 7.00 (m, 1H), 7.13 (m, 2H); LRMS : m/z (APCI) 578 [MNa⁺].

### Preparation 31

### tert-Butyl (2S)-2-[(1-{[((1S)-1-{[(2,4-difluorobenzyl)oxy]methyl}-3-ethoxy-3-oxopropyl)-amino]carbonyl}cyclopentyl)methyl]-4-methoxybutanoate

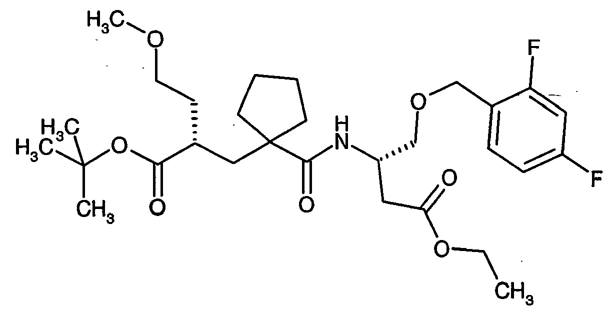

A mixture of the acid from preparation 22 (53mg, 0.1mmol), iodoethane (24mg, 0.15mmol) and potassium carbonate (28mg, 0.2mmol) in N,N-dimethylformamide (2ml) was stirred at room temperature for 18 hours. The reaction was diluted with water and extracted with dichloromethane. The combined organic solutions were dried (MgSO₄) and concentrated under reduced pressure. The residue was purified by column chromatography on silica gel using dichloromethane:methanol (99:1 to 97.5:2.5) to afford the title compound as a colourless oil, 42mg; ¹H NMR (CDCl₃, 400MHz) δ: 1.22 (t, 3H), 1.40-1.80 (m, 18H), 1.98 (m, 3H), 2.35 (m, 1 H), 2.63 (m, 2H), 3.26 (s, 3H), 3.32 (m, 2H), 3.50 (m, 1H), 3.60 (m, 1H), 4.08 (q, 2H), 4.43 (m, 1 H), 4.52 (s, 2H), 6.58 (d, 1H), 6.78-6.88 (m, 2H), 7.36 (m, 1H); LRMS : m/z (ES⁺) 578 [MNa⁺].

### Preparation 32

### tert-Butyl (2S)-2-[(1-{[((1S)-3-(allyloxy)-1-{[(4-chlorobenzyl)oxy]methyl}-3-oxopropyl)-amino]carbonyl}cyclopentyl)methyl]-4-methoxybutanoate

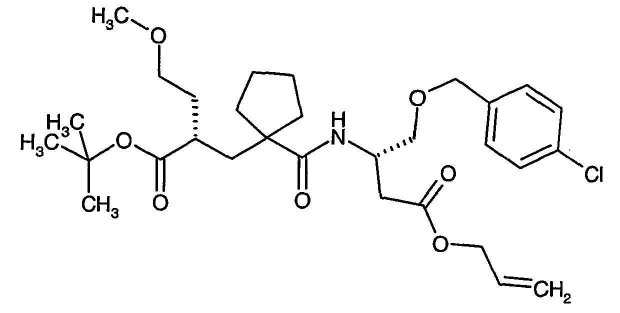

The title compound was obtained as a pale yellow oil in 86% yield, from the acid from preparation 18 and allyl bromide, following the procedure described in preparation 26. ¹H NMR (CDCl₃, 400MHz) δ: 1.40-1.82 (m, 18H), 1.98 (m, 3H), 2.38 (m, 1H), 2.68 (m, 2H), 3.27 (m, 5H), 3.50 (m, 1 H), 3.58 (m, 1 H), 4.44 (s, 3H), 4.57 (d, 2H), 5.22 (d, 1 H), 5.30 (d, 1 H), 5.85 (m, 1 H), 6.58 (d, 1 H), 7.22 (d, 2H), 7.30 (d, 2H); LRMS : m/z (APCI) 566 [MH⁺]; [α]_{D} = -18.8 (c = 0.1, methanol); Microanalysis found: C, 63.44; H, 7.91; N, 2.43. C₃₀H₄₄ClNO₇ requires C, 63.70; H, 7.78; N, 2.47%.

### Preparation 33

### (2R)-2-[(1-{[((1S)-3-(Allyloxy)-1-{[(3-methoxybenzyl)oxy]methyl}-3-oxopropyl)amino]-carbonyl}cyclopentyl)methyl]pentanoic acid

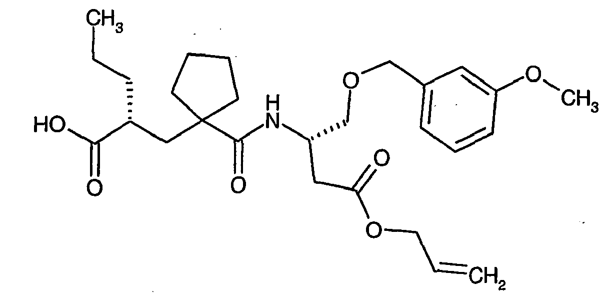

Trifluoroacetic acid (1 ml) was added to a solution of the ester from preparation 26 (120mg, 0.20mmol) in dichloromethane (5ml) and the reaction stirred at room temperature for 3 hours. The mixture was concentrated under reduced pressure and the residue azeotroped with dichloromethane and ethyl acetate to afford the title compound, 110mg; ¹H NMR (CDCl₃, 400MHz) δ: 0.85 (t, 3H), 1.50 (m, 4H), 1.50-1.76 (m, 7H), 1.92-2.04 (m, 3H), 2.38 (m, 1 H), 2.64 (m, 2H), 3.58 (m, 2H), 3.80 (s, 3H), 4.45 (m, 3H), 4.57 (d, 2H), 5.22 (d, 1 H), 5.30 (d, 1 H), 5.86 (m, 1 H), 6.75 (d, 1 H), 6.84 (m, 3H), 7.26 (m, 1 H); LRMS : m/z (ES⁺) 490 [MH⁺].

### Preparation 34

### (2S)-2-[(1-{[((1S)-3-(Allyloxy)-1-{[(4-chlorobenzyl)oxy]methyl}-3-oxopropyl)amino]-carbonyl}cyclopentyl)methyl]-4-methoxybutanoic acid

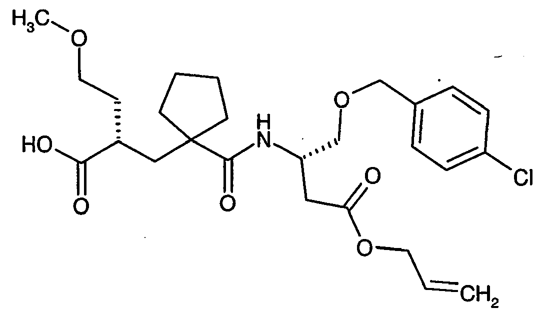

The title compound was obtained as an orange oil, from the ester from preparation 32, following the procedure described in preparation 33; ¹H NMR (CDCl₃, 400MHz) δ: 1.55-1.74 (m, 8H), 1.97 (m, 3H), 2.10 (m, 1 H), 2.56 (m, 1 H), 2.62 (m, 2H), 3.26 (s, 3H), 3.38 (m, 2H), 3.57 (m, 2H), 4.43 (m, 3H), 4.56 (m, 2H), 5.21 (d, 1H) 5.27 (d, 1H), 5.84 (m, 1H), 6.64 (d, 1H), 7.21 (m, 2H), 7.30 (d, 2H); LRMS : m/z (ES⁺) 510 [MH⁺]; [α]_{D} = -16.67° (c = 0.06, methanol); Microanalysis found: C, 59.06; H, 6.96; N, 2.56. C₂₆H₃₆ClNO₇;H₂O requires C, 59.20; H, 7.21; N, 2.65%.

### Preparation 35

### Ethyl (2R)-2-[(1-{[((1S)-3-(allyloxy)-1-{[(3-methoxybenzyl)oxy]methyl}-3-oxopropyl)-amino]carbonyl}cyclopentyl)methyl]pentanoate

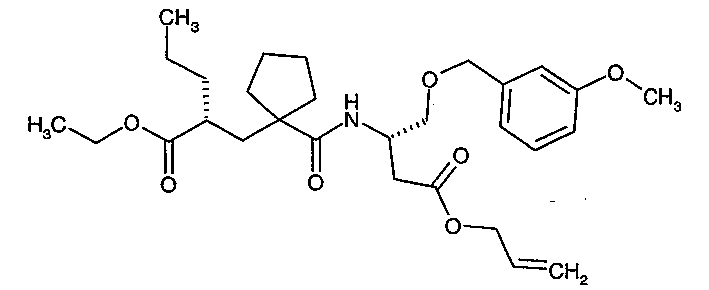

The title compound was obtained as a pale yellow solid, from the acid from preparation 33 and iodoethane, following the procedure described in preparation 26; ¹H NMR (CDCl₃, 400MHz) δ: 0.82 (t, 3H), 1.21 (m, 6H), 1.30-1.65 (m, 7H), 1.78 (dd, 1H), 1.84-2.04 (m, 3H), 2.35 (m, 1H), 2.66 (m, 2H), 3.50 (m, 1 H), 3.59 (m, 1H), 3.79 (s, 3H), 4.08 (m, 2H), 4.44 (m, 3H), 4.56 (d, 2H), 5.21 (d, 1 H), 5.32 (d, 1 H), 5.87 (m, 1H), 6.50 (d, 1H), 6.82 (m, 3H), 7.22 (m, 1 H); LRMS : m/z (ES⁺) 540 [MNa⁺].

### Preparation 36

### Ethyl (2S)-2-[(1-{[((1S)-3-(allyloxy)-1-{[(4-chlorobenzyl)oxy]methyl}-3-oxopropyl)amino]-carbonyl}cyclopentyl)methyl]-4-methoxybutanoate

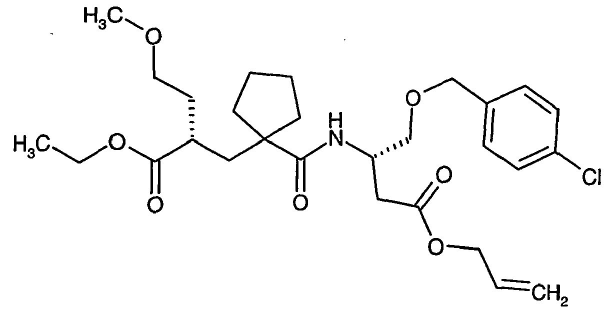

A mixture of the acid from preparation 34 (280mg, 0.55mmol), iodoethane (200mg, 1.29mmol) and potassium carbonate (228mg, 1.65mmol) in N,N-dimethylformamide (3ml) was stirred at room temperature for 18 hours. The reaction was diluted with water (50ml) and extracted with ethyl acetate (3x50ml). The combined organic solutions were washed with brine (2x50ml), dried (MgSO₄) and evaporated under reduced pressure, to afford the title compound as a pale yellow oil, 241 mg; ¹H NMR (CDCl₃, 400MHz) δ: 1.22 (t, 3H), 1.40-1.70 (m, 7H), 1.78-1.95 (m, 3H), 2.00 (m, 2H), 2.44 (m, 1 H), 2.68 (m, 2H), 3.28 (m, 5H), 3.50 (dd, 1 H), 3.59 (dd, 1 H), 4.10 (m, 2H), 4.44 (s, 3H), 4.56 (d, 2H), 5.22 (d, 1 H), 5.30 (d, 1 H), 5.88 (m, 1 H), 6.57 (d, 1 H), 7.22 (d, 2H), 7.31 (d, 2H); Microanalysis found: C, 62.12; H, 7.58; N, 2.68. C₂₈H₄₀ClNO₇ requires C, 62.45; H, 7.43; N, 2.60%.

### Preparation 37

### Sodium (3R)-3-[(tert-butoxycarbonyl)amino]-4-hydroxybutanoate

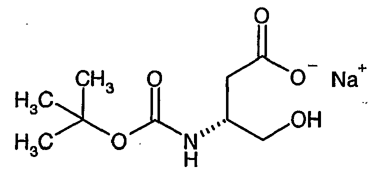

A solution of *N*^{α}-(*tert*-butyloxycarbonyl)-D-aspartic anhydride (J. Med. Chem. 1996; 3842) (19g, 88mmol) in tetrahydrofuran (150ml) was added dropwise to an ice-cooled suspension of sodium borohydride (3.4g, 88mmol) in tetrahydrofuran (75ml), and once addition was complete, the mixture was stirred for a further 2 hours. Acetic acid (30ml) was added dropwise and the mixture then evaporated under reduced pressure. The residue was partitioned between water (150ml) and ether (150ml) and 2N hydrochloric acid (20ml) carefully added. The layers were separated, the aqueous phase further extracted with ether (2x150ml), and the combined organic solutions dried (MgSO₄) and evaporated under reduced pressure. The product was dissolved in 2N sodium hydroxide (50ml), and the solution stirred for 1 hour. The mixture was washed with ether (2x50ml), acidified with 2N hydrochloric acid (50ml) and extracted with dichloromethane (4x100ml). The combined dichloromethane extracts were dried (MgSO₄) and evaporated under reduced pressure. The acid product (7.8g, 35.6mmol) was dissolved in methanol (100ml) sodium hydroxide (1.42g, 35.6mmol) added, and the reaction stirred at room temperature for 1 hour. The mixture was evaporated under reduced pressure the residue azeotroped with toluene and then triturated with diisopropyl ether to afford the title compound as a white solid, 8.6g; ¹H NMR (DMSOd₆, 400MHz) δ: 1.38 (s, 9H), 2.17 (m, 2H), 3.21-3.50 (m, 3H), 6.74 (d, 1H).

### Preparation 38

### Ethyl (3S)-4-(benzyloxy)-3-[(tert-butoxycarbonyl)amino]butanoate

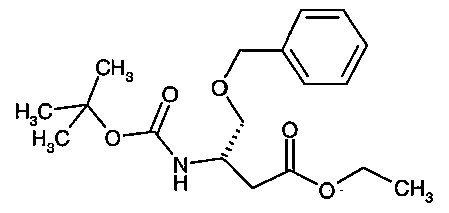

A solution of cesium carbonate (3.53g, 10.9mmol) in water (22ml) was added to an ice-cooled solution of (3*S*)-4-(benzyloxy)-3-[(*tert*-butoxycarbonyl)amino]butanoic acid (3.36g, 10.9mmol) in methanol (70ml) and water (7ml), and the reaction stirred for 20 minutes. The mixture was concentrated under reduced pressure and azeotroped with N,N-dimethylformamide and dichloromethane. The residual oil was dissolved in N,N-dimethylformamide (70ml), ethyl iodide (1.64g, 10.9mmol) added, and the reaction stirred at room temperature for 18 hours. The mixture was concentrated under reduced pressure, suspended in ethyl acetate (300ml), washed with water (4x), brine, then dried (MgSO₄) and evaporated under reduced pressure. The residual oil was purified by column chromatography on silica gel using hexane:ethyl acetate (85:15) to afford the title compound as a pale yellow oil, 2.86g; ¹H NMR (CDCl₃, 400MHz) δ: 1.26 (t, 3H), 1.45 (s, 9H), 2.65 (d, 2H), 3.58 (m, 2H), 4.17 (m, 3H), 4.55 (s, 2H), 5.20 (bs, 1 H), 7.36 (m, 5H).

### Preparation 39

### (3R)-3-[(tert-Butoxycarbonyl)amino]-4-[(4-cyanobenzyl)oxy]butanoic acid

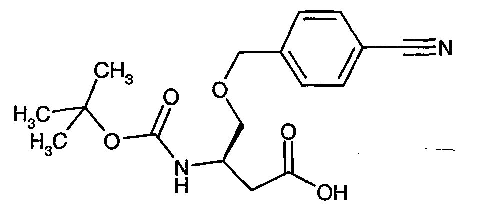

Sodium hydride (650mg, 60% in mineral oil, 16.25mmol), followed by imidazole (34mg, 0.49mmol) were added portionwise to a cooled (-15°C) suspension of the acid from preparation 37 (600mg, 2.49mmol) in tetrahydrofuran (20ml), and the mixture then stirred for a further hour. 4-Cyanobenzyl bromide (540mg, 2.74mmol) was added, the mixture stirred at -15°C for 2 hours and then allowed to warm to room temperature and stirred for a further 18 hours. The reaction mixture was cooled in an ice-bath, water added carefully, followed by 1 M hydrochloric acid, and the mixture extracted with ethyl acetate (3x100ml). The combined organic extracts were washed with brine (150ml), dried (MgSO₄) and evaporated under reduced pressure. The residual oil was purified by column chromatography on silica gel using dichloromethane:methanol (94:6) as eluant to afford the title compound as a yellow oil, 442mg; ¹H NMR (CDCl₃, 400MHz) δ: 1.42 (s, 9H), 2.68 (d, 2H), 3.59 (m, 2H), 4.18 (m, 1 H), 4.59 (s, 2H), 5.17 (m, 1H), 7.41 (d, 2H), 7.62 (d, 2H); HRMS : m/z (ESI⁺) 357.1414 [MNa⁺] C₁₇H₂₂N₂O₅ requires C, 357.1421.

### Preparation 40

### 5-(Bromomethyl)-2-methylpyridine hydrobromide

A solution of (6-methylpyridin-3-yl)methanol (J. Med. Chem. 43; 18; 2000; 3386) (492mg, 4mmol) and thionyl bromide (4.16g, 20mmol) in dichloromethane (20ml) was stirred at room temperature for 3 hours. The reaction was concentrated under reduced pressure and the residue azeotroped with dichloromethane. The residual red oil was triturated well with ether to afford the title compound as an orange powder, 1.39g; ¹H NMR (DMSOd₆, 400MHz) δ: 2.64 (s, 3H), 4.81 (s, 2H), 7.81 (d, 1H), 8.42 (d, 1 H), 8.84 (s, 1H).

### Preparation 41

### Ethyl (3R)-3-[(tert-butoxycarbonyl)amino]-4-[(4-cyanobenzyl)oxy]butanoate

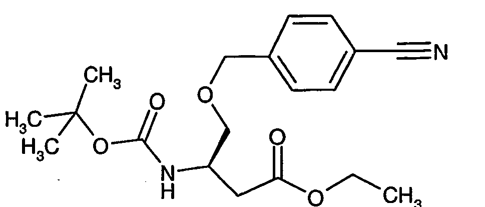

A mixture of the acid from preparation 39 (410mg, 1.13mmol), potassium carbonate (468mg, 3.40mmol) and iodoethane (529mg, 3.40mmol) in N,N-dimethylformamide (5ml) was stirred at room temperature for 6 hours. The mixture was evaporated under reduced pressure, the residue suspended in brine (100ml) and extracted with ethyl acetate (3x80ml). The combined organic extracts were washed with brine (150ml), dried (MgSO₄) and evaporated under reduced pressure to afford the title compound as a yellow oil, 385mg; ¹H NMR (CDCl₃, 400MHz) δ: 1.22 (t, 3H), 1.42 (s, 9H), 2.62 (d, 2H), 3.58 (m, 2H), 4.05-4.22 (m, 3H), 4.58 (m, 2H), 5.18 (m, 1 H), 7.40 (d, 2H), 7.62 (d, 2H). HRMS : m/z (ESI⁺) 385.1726 [MNa⁺] C₁₉H₂₆N₂O₅ requires 385.1726.

### Preparation 42

### (3R)-3-[(tert-Butoxycarbonyl)amino]-4-[(6-methylpyridin-3-yl)methoxy]butanoic acid

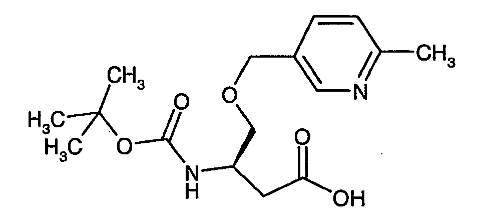

Sodium hydride (800mg, 60% dispersion in mineral oil, 20mmol) and imidazole (54mg, 0.8mmol) were added portionwise to a cooled (-15°C) solution of the acid from preparation 37 (960mg, 4mmol) in tetrahydrofuran (40ml) and the solution stirred for 1 hour. A suspension of the bromide from preparation 40 (1.28g, 4.8mmol) and N-methyl morpholine (535mg, 5.3mmol) in tetrahydrofuran (10ml) was added so as to maintain the temperature below -15°C, and the reaction then stirred for a further 2 hours. The reaction was allowed to warm to room temperature and stirred for a further 18 hours. The mixture was then basified to pH 11, washed with ethyl acetate, carefully acidifed to pH 5 and extracted with ethyl acetate. These combined organic solutions were washed with brine, dried (MgSO₄) and evaporated under reduced pressure. The crude product was purified by column chromatography on silica gel using dichloromethane:methanol (94:6) as eluant, and the product triturated with ether to afford the title compound, 177mg; ¹H NMR (CDCl₃, 400MHz) δ: 1.42 (s, 9H), 2.62-2.80 (m, 5H), 3.64 (m, 2H), 4.18 (m, 1 H), 4.60 (s, 2H), 5.50 (d, 1 H), 7.40 (d, 1H), 7.83 (d, 1H), 8.62 (s, 1H); LRMS : m/z (ES⁻) 323 [M-H]⁻.

### Preparation 43

### Ethyl (3S)-3-amino-4-(benzyloxy)butanoate hydrochloride

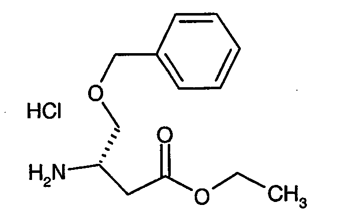

Hydrogen chloride was bubbled through an ice-cooled solution of the protected amine from preparation 38 (2.8g, 8.3mmol) in ethyl acetate (40ml) for 15 minutes. The solution was evaporated under reduced pressure and azeotroped with diethyl ether to afford the title compound as an oil, 2.0g; ¹H NMR (CDCl₃, 400MHz) δ: 1.20 (t, 3H), 2.84 (dd, 1 H), 3.00 (dd, 1 H), 3.58 (m, 2H), 3.90 (m, 1 H), 4.10 (q, 2H), 4.58 (s, 2H), 7.30 (m, 5H), 8.60 (bs, 2H).

### Preparation 44

### Ethyl (3R)-3-amino-4-(4-cyanobenzyloxy)butanoate

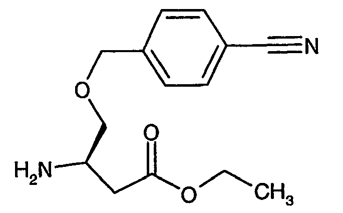

A solution of the protected amine from preparation 41 (375mg, 1 mmol) and trifluoroacetic acid (2ml) in dichloromethane (8ml) was stirred at room temperature for 3 hours. The reaction was concentrated under reduced pressure and the residue azeotroped with dichloromethane. The product was partitioned between ethyl acetate and saturated sodium bicarbonate solution, the layers separated, and the organic phase washed with brine, dried (MgSO₄) and evaporated under reduced pressure to give a yellow oil, 260mg. The crude product was purified by column chromatography on silica gel using dichloromethane:methanol (97.5:2.5) to afford the title compound as a colourless oil, 180mg; ¹H NMR (CDCl₃, 400MHz) δ: 1.22 (t, 3H), 2.65 (d, 2H), 3.57-3.70 (m, 3H), 4.16 (m, 2H), 4.60 (m, 2H), 7.43 (d, 2H), 7.63 (d, 2H); LRMS : m/z (ESI⁺) 285 [MNa⁺].

### Preparation 45

### Ethyl (3R)-3-amino-4-[(6-methylpyridin-3-yl)methoxy]butanoate dihydrochloride

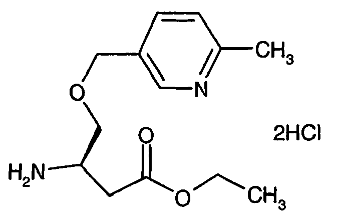

A solution of the protected amine from preparation 42 (175mg, mmol) in ethanolic hydrogen chloride (6ml) was heated under reflux for 2 hours. The solution was concentrated under reduced pressure, the residue azeotroped with toluene and dried *in vacuo* to give the title compound as a colourless gum, 176mg; ¹H NMR (CD₃OD, 400MHz) δ: 1.27 (t, 3H), 2.80 (m, 5H), 3.79 (m, 1H), 3.86 (m, 2H), 4.20 (q, 2H), 4.82 (s, 2H), 7.97 (d, 1H), 8.54 (d, 1H), 8.78 (s, 1H); LRMS : m/z (ES⁺) 275 [MNa⁺].

### Preparation 46

### tert-Butyl (2R)-2-({1-[({(1S)-1-[(benzyloxy)methyl]-3-ethoxy-3-oxopropyl}amino)carbonyl]-cyclopentyl}methyl)pentanoate

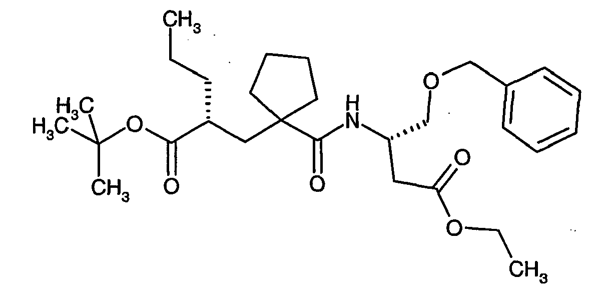

A solution of 1-[(2*R*)-2-(*tert*-butyoxycarbonyl-pentyl)]cyclopentanecarboxylic acid (WO 0202513, preparation 2) (156mg, 0.55mmol) in dichloromethane (1ml) was added to a solution of the amine from preparation 43 (150mg, 0.55mmol), 1-hydroxybenzotriazole hydrate (74mg, 0.55mmol), 1-(3-dimethylaminopropyl)-3-ethylcarbodiimide hydrochloride (105mg, 0.55mmol) and N-methyl morpholine (120µl, 1.1mmol) in dichloromethane (5ml), and the reaction stirred at room temperature for 20 hours. The mixture was diluted with dichloromethane (20ml), washed with 1N hydrochloric acid (10ml), then water (10ml), dried (MgSO₄) and evaporated under reduced pressure. The crude product was purified by column chromatography on silica gel using dichloromethane:methanol (100:0 to 95:5) to afford the title compound, 110mg; ¹H NMR (CDCl₃, 300MHz) δ: 0.86 (t, 3H), 1.20-1.78 (m, 22H), 1.96-2.30 (m, 5H), 2.63 (m, 2H), 3.56 (dd, 1H), 3.60 (dd, 1H), 4.10 (q, 2H), 4.50 (m, 3H), 6.58 (d, 1H), 7.37 (m, 5H); LRMS : m/z (ES⁺) 526 [MNa⁺].

### Preparation 47

### tert-Butyl (2R)-2-[(1-{[((1R)-1-{[(4-cyanobenzyl)oxy]methyl}-3-ethoxy-3-oxopropyl)amino]-carbonyl}cyclopentyl)methyl]pentanoate

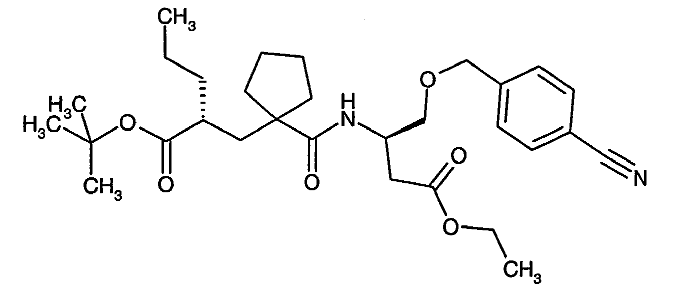

The amine from preparation 44 (160mg, 0.61 mmol), was added to a solution of 1-[(2R)-2-(*tert*-butyoxycarbonyl-pentyl)]-cyclopentanecarboxylic acid (WO 0202513, preparation 2) (191mg, 0.67mmol), 1-hydroxybenzotriazole hydrate (108mg, 0.80mmol), 1-(3-dimethylaminopropyl)-3-ethyloarbodiimide hydrochloride (141 mg, 0.74mmol) and N-methyl morpholine (169mg, 1.7mmol) in dichloromethane (6ml), and the reaction stirred at room temperature for 18 hours. The reaction was diluted with ethyl acetate, washed with water (2x), then brine, dried (MgSO₄) and evaporated under reduced pressure. The residual gum was purified by column chromatography on silica gel using an elution gradient of dichloromethane:methanol (100:0 to 99:1) to afford the title compound as a viscous oil, 240mg; ¹H NMR (CDCl₃, 400MHz) δ: 0.83 (t, 3H), 1.22 (m, 6H), 1.40-1.77 (m, 17H), 1.97 (m, 3H), 2.20 (m, 1 H), 2.62 (m, 2H), 3.60 (m, 2H), 4.10 (q, 2H), 4.50 (m, 1 H), 4.60 (s, 2H), 6.59 (d, 1 H), 7.41 (d, 2H), 7.62 (d, 2H); LRMS : m/z (ES⁺) 551 [MNa⁺].

### Preparation 48

### tert-Butyl (2R)-2-[(1-{[((1R)-3-ethoxy-1-{[(6-methyl-3-pyridinyl)methoxy]methyl}-3-oxopropyl)amino]carbonyl}cyclopentyl)methyl]pentanoate

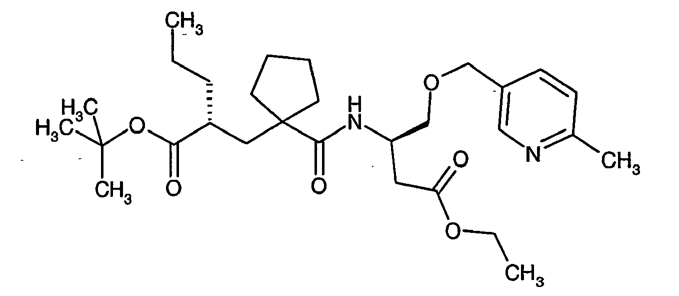

The title compound was obtained as a colourless oil in 67% yield from 1-[(2*R*)-2-(*tert-*butyoxycarbonyl-pentyl)]-cyclopentanecarboxylic acid (WO 0202513, preparation 2) and the amine from preparation 45, following the procedure described in preparation 47.
¹H NMR (CDCl₃, 400MHz) δ: 0.83 (t, 3H), 1.24 (m, 6H), 1.40-1.78 (m, 17H), 1.98 (m, 3H), 2.21 (m, 1H), 2.60 (m, 5H), 3.55 (m, 1 H), 3.61 (m, 1H), 4.08 (q, 2H), 4.50 (m, 3H), 6.55 (d, 1H), 7.19 (d, 1H), 7.62 (d, 1H), 8.83 (s, 1H); LRMS : m/z (ES⁺) 519 [MH⁺].

### Preparation 49

### (3S)-4-(Benzyloxy)-3-[({1-[(2R)-2-(tert-butoxycarbonyl)pentyl]cyclopentyl}-carbonyl)amino]butanoic acid

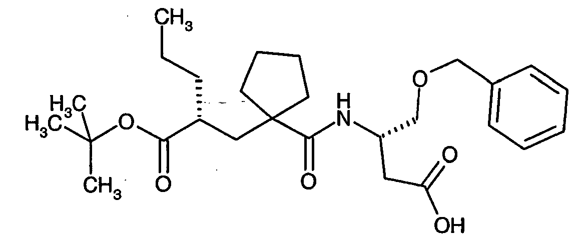

The title compound was prepared, from the alcohol from preparation 6 and benzyl bromide, following the procedure described for preparation 10; ¹H NMR (CDCl₃, 400MHz) δ: 0.83 (t, 3H), 1.22-1.74 (m, 20H), 1.90-2.02 (m, 3H), 2.21 (m, 1H), 2.67 (m, 2H), 3.56 (dd, 1 H), 3.60 (dd, 1H), 4.40-4.58 (m, 3H), 6.48 (d, 1H), 7.17-7.40 (m, 5H); LRMS : m/z (ES⁻) 474 [M-H⁻].

### Preparation 50

### tert-Butyl (2R)-2-{[1-({[(1S)-1-[(benzyloxy)methyl]-3-(2-butoxyethoxy)-3-oxopropyl]amino}carbonyl)cyclopentyl]methyl}pentanoate

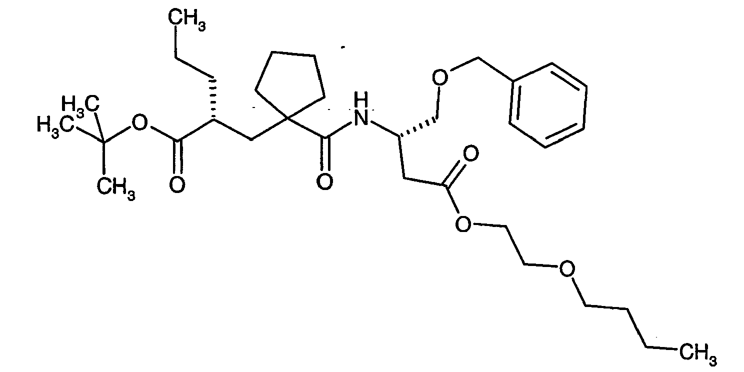

4-Dimethylaminopyridine (30mg) and 1-(3-dimethylaminopropyl)-3-ethylcarbodiimide hydrochloride (48mg, 0.25mmol) were added to a solution of the acid from preparation 49 (120mg, 0.25mmol) and 2-butoxyethanol (30mg, 0.25mmol) in dichloromethane (5ml), and the reaction stirred at room temperature for 18 hours. The mixture was evaporated under reduced pressure and the residue purified by column chromatography on silica gel using an elution gradient of dichloromethane:methanol (100:0 to 98:2) to afford the title compound, 100mg; ¹H NMR (CDCl₃, 400MHz) δ: 0.82 (t, 3H), 0.94 (t, 3H), 1.21-1.76 (m, 24H), 1.98 (m, 3H), 2.20 (m, 1H), 2.68 (m, 2H), 3.41 (d, 2H), 3.52 (m, 1H), 3.60 (m, 3H), 4.19 (m, 2H), 4.44 (m, 3H), 6.50 (d, 1H), 7.30 (m, 5H).

### Preparation 51

### tert-Butyl (2R)-2-({1-[({(1S)-1-[(benzyloxy)methyl]-3-oxo-3-[2-oxo-2-(1-piperidinyl)ethoxy]propyl}amino)carbonyl]cyclopentyl}methyl)pentanoate

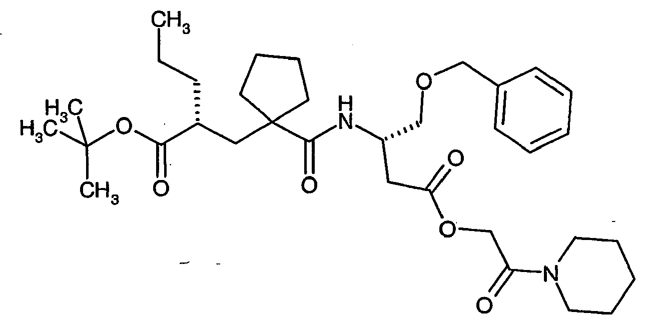

1-(Bromoacetyl)piperidine (EP 580402) (58mg, 0.28mmol) was added to a suspension of the acid from preparation 49 (120mg, 0.25mmol) and potassium carbonate (35mg, 0.25mmol) in N,N-dimethylformamide (3ml), and the reaction stirred at room temperature for 18 hours. The mixture was concentrated under reduced pressure and the residue purified by column chromatography on silica gel using an elution gradient of dichloromethane:methanol (100:0 to 98.5:1.5) to afford the title compound, 100mg; ¹H NMR (CDCl₃, 400MHz) δ: 0.82 (t, 3H), 1.20-1.68 (m, 25H), 1.78 (m, 1H), 2.00 (m, 3H), 2.22 (m, 1 H), 2.78 (dd, 2H), 3.25 (m, 2H), 3.56 (m, 2H), 3.60 (m, 2H), 4.58 (m, 3H), 4.62 (d, 1H), 4.78 (d, 1H), 6.98 (d, 1H), 7.26 (m, 5H); LRMS : m/z (ES⁺) 623 [MNa⁺].

### Preparation 52

### (2R)-2-[(1-{[((1R)-1-{[(4-Cyanobenzyl)oxy]methyl}-3-ethoxy-3-oxopropyl)amino]carbonyl}-cyclopentyl)methyl]pentanoic acid

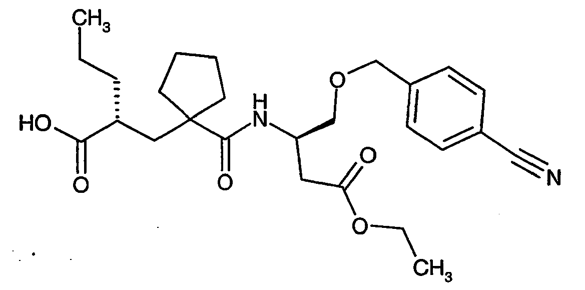

A solution of the ester from preparation 47 (220mg, 0.42mmol) and trifluoroacetic acid (2ml) in dichloromethane (8ml) was stirred at room temperature for 3 hours. The mixture was concentrated under reduced pressure and the residue azeotroped with dichloromethane. The product was suspended in ethyl acetate, washed with water and brine, then dried (MgSO₄) and evaporated under reduced pressure to afford the title compound as a colourless gum, 189mg; ¹H NMR (CDCl₃, 400MHz) δ: 0.84 (t, 3H), 1.26 (m, 6H), 1.46-1.78 (m, 8H), 1.98 (m, 1 H), 2.02 (m, 2H), 2.38 (m, 1H), 2.62 (m, 2H), 3.60 (m, 2H), 4.10 (q, 2H), 4.44-4.62 (m, 3H), 6.70 (d, 1H), 7.41 (d, 2H), 7.62 (d, 2H); LRMS : m/z (ES⁺) 495 [MNa⁺].

### Preparation 53

### (2R)-2-[(1-{[((1R)-3-Ethoxy-1-{[(6-methyl-3-pyridinyl)methoxy]methyl}-3-oxopropyl)-amino]carbonyl}cyclopentyl)methyl]pentanoic acid

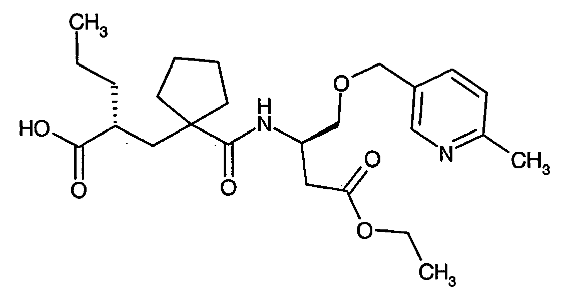

A solution of the ester from preparation 48 (94mg, 0.18mmol) and trifluoroacetic acid (1ml) in dichloromethane (4ml) was stirred at room temperature for 18 hours. The mixture was concentrated under reduced pressure and the residue azeotroped with dichloromethane. The product was dissolved in water, the pH adjusted to 6 and the mixture extracted with ethyl acetate. The combined organic extracts were dried (MgSO₄) and evaporated under reduced pressure. The crude product was purified by column chromatography on silica gel using dichloromethane:methanol (90:10) as eluant to afford the title compound as a colourless gum, 63mg; HRMS : m/z (ES⁺) 463.2801 [MH⁺] C₂₅H₃₈N₂O₆ requires 463.2803.

### Preparation 54 -

### Ethyl 5-{[tert-bulyl(dimethyl)silyl]oxy}-2-pentenoate

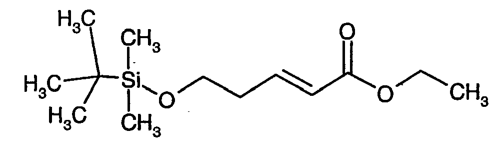

Methyl sulphoxide (1.9ml, 26.8mmol) was added to a cooled (-78°C) solution of oxalyl chloride (2.16ml, 24.7mmol) in dichloromethane (100ml), and after 10 minutes, a solution of 3-(*tert*-butyl-dimethyl-silanyloxy)-propan-1-ol (3g, 15.8mmol) in dichloromethane (25ml) was added dropwise. Once addition was complete, the solution was stirred for a further 15 minutes. Triethylamine (8ml, 57.5mmol) was added, followed by dropwise addition of a solution of (ethoxycarbonylmethylene)triphenylphosphorane (10g, 29.9mmol) in dichloromethane (50ml), and the reaction mixture allowed to warm to room temperature. The mixture was washed with water (4x), dried (MgSO₄) and concentrated under reduced pressure. The residue was pre-adsorbed onto silica gel and purified by column chromatography using diethyl ether:pentane (2.5:97.5) as eluant to afford the title compound, 1.3g; ¹H NMR (CDCl₃, 400MHz) δ: 0.02 (s, 6H), 0.87 (s, 9H), 1.26 (t, 3H), 2.40 (m, 2H), 3.74 (t, 2H), 4.18 (q, 2H), 5.84 (d, 1H), 6.95 (m, 1H).

### Preparation 55

### Ethyl (3R)-3-{benzyl[(1R)-1-phenylethyl]amino}-5-{[tert-butyl(dimethyl)silyl]oxy}-pentanoate

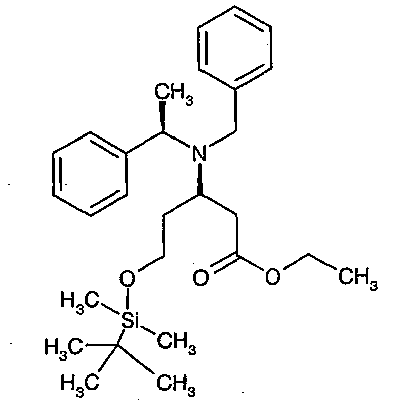

n-Butyl lithium (21.4ml, 53.5mmol) was added to a cooled (-78°C) solution of (*R*)-(+)-*N-*benzyl-α-methylbenzylamine (12.0g, 57.06mmol) in tetrahydrofuran (100ml), and the solution stirred for 1 hour. A solution of the compound from preparation 54 (9.2g, 35.7mmol) in tetrahydrofuran (50ml) was added, and the reaction stirred for a further 2 hours at -78°C. Saturated ammonium chloride solution (40ml) was added, and the mixture then allowed to warm to room temperature. The mixture was concentrated under reduced pressure, the residue partitioned between ether and water and the layers separated. The organic phase was dried (MgSO₄) and evaporated under reduced pressure. The residual yellow oil was purified by column chromatography on silica gel using diethyl ether:pentane (1.5:98.5) as eluant to afford the title compound as a pale yellow oil, 8.6g; ¹H NMR (CDCl₃, 400MHz) δ: 0.07 (s, 6H), 0.88 (s, 9H), 1.18 (t, 3H), 1.38 (d, 3H), 1.55 (m, 1 H), 1.80 (m, 1H), 2.02 (m, 2H), 3.58 (m, 1H), 3.76 (m, 3H), 3.83 (m, 1 H), 3.94-4.06 (m, 2H), 7.20-7.42 (m, 10H); LRMS : m/z (ES⁺) 470 [MH⁺].

### Preparation 56

### Ethyl (3S)-3-{benzyl[(1S)-1-phenylethyl]amino}-5-{[tert-butyl(dimethyl)silyl]oxy}-pentanoate

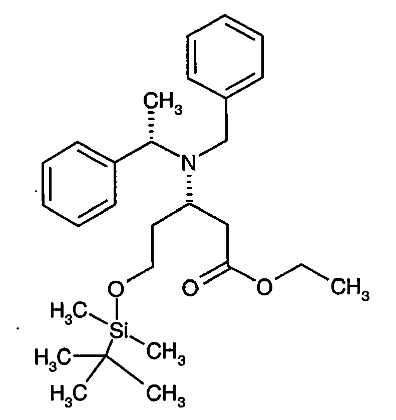

The title compound was obtained as a colourless oil in 56% yield from (*S*)-(-)-*N*-benzyl-α-methylbenzylamine and the compound from preparation 54, following the procedure described in preparation 55; ¹H NMR (CDCl₃, 400MHz) δ: 0.05 (s, 6H), 0.88 (s, 9H), 1.18 (t, 3H), 1.38 (d, 3H), 1.55 (m, 1 H), 1.80 (m, 1 H), 2.02 (m, 2H), 3.58 (m, 2H), 3.70-3.88 (m, 4H), 3.99 (m, 2H), 7.19-7.37 (m, 8H), 7.40 (d, 2H); [α]_{D} = -4.6 (c = 0.096 in methanol).

### Preparation 57

### Ethyl (3R)-3-amino-5-{[tert-butyl(dimethyl)silyl]oxy}pentanoate

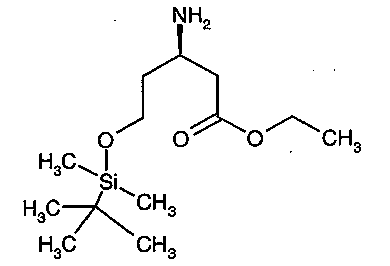

A mixture of the compound from preparation 55 (8.6g, 18.34mmol), and 10% palladium on charcoal (2.5g) in acetic acid (100ml) was hydrogenated at 6 atm and room temperature for 72 hours. The mixture was filtered through Arbocel® and the filtrate evaporated under reduced pressure. The residue was purified by column chromatography on silica gel using an elution gradient of dichloromethane:methanol:0.88 ammonia (98:2:0.2 to 95:5:0.5) to give an orange oil. The product was dissolved in ethyl acetate, the solution washed with sodium carbonate solution, dried (MgSO₄) and evaporated under reduced pressure. The residual oil was further purified by column chromatography on silica gel using an elution gradient of dichloromethane:methanol:0.88 ammonia (99:1:0.1 to 97:3:0.3) to afford the title compound, 3g; ¹H NMR (CDCl₃, 400MHz) δ: 0.05 (s, 6H), 0.88 (s, 9H), 1.25 (t, 3H), 1.56-1.68 (m, 2H), 2.34 (dd, 1H), 2.50 (dd, 1H), 3.39 (m, 1H), 3.77 (m, 2H), 4.15 (q, 2H).

### Preparation 58

### Ethyl (3S)-3-amino-5-{[tert-butyl(dimethyl)silyl]oxy}pentanoate

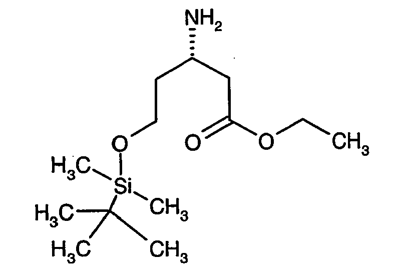

A mixture of the compound from preparation 56 (10.0g, 21.3mmol), and 10% palladium on charcoal (2.9g) in acetic acid (150ml) was hydrogenated at 6 atm and room temperature for 42 hours. The mixture was filtered through Arbocel®, and the filtrate concentrated under reduced pressure. The residue was partitioned between ethyl acetate and saturated aqueous sodium bicarbonate solution and the layers separated. The aqueous phase was further extracted with ethyl acetate (200ml), and the combined organic solutions dried (MgSO₄) and evaporated under reduced pressure. The crude product was purified by column chromatography on silica gel using an elution gradient of dichloromethane:methanol:0.88 ammonia (95:5:0.5 to 90:10:1) to afford the title compound as a yellow oil, 4.27g; ¹H NMR (CDCl₃, 400MHz) δ: 0.05 (s, 6H), 0.88 (s, 9H), 1.25 (t, 3H), 1.64 (m, 2H), 2.40 (dd, 1H), 2.56 (m, 1H), 3.42 (m, 1H), 3.77 (m, 2H), 4.15 (q, 2H); LRMS : m/z (ES⁺) 275.6 [MH⁺]

### Preparation 59

### Ethyl (3R)-3-[({1-[(2R)-2-(tert-butoxycarbonyl)pentyl]cyclopentyl}carbonyl)amino]-5-{[tert-butyl(dimethyl)silyl]oxy}pentanoate

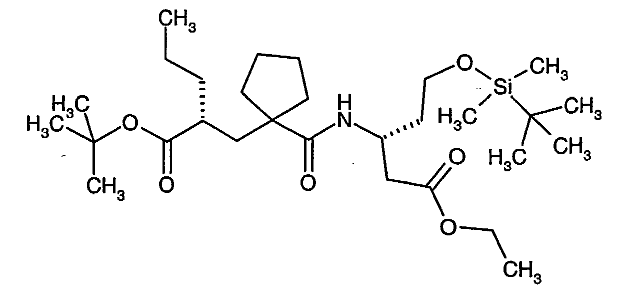

A mixture of the amine from preparation 57 (1.5g, 5.45mmol), 1-[(2*S*)-2-(*tert-*butyoxycarbonyl-pentyl)]-cyclopentanecarboxylic acid (WO 0202513, preparation 2) (1.7g, 6.0mmol), 1-(3-dimethylaminopropyl)-3-ethylcarbodiimide hydrochloride (1.15g, 6.00mmol), 1-hydroxybenzotriazole hydrate (811mg, 6.0mmol) and N-methylmorpholine (1.15ml, 10.45mmol) in dichloromethane (30ml) was stirred at room temperature for 18 hours. The reaction was washed with water, dried (MgSO₄) and concentrated under reduced pressure. The residual yellow oil was purified by column chromatography on silica gel using dichloromethane:methanol:0.88 ammonia (99.5:0.5:0.05) as eluant to afford the title compound as a colourless oil, 2.3g; ¹H NMR (CDCl₃, 400MHz) δ: 0.05 (s, 6H), 0.90 (m, 15H), 1.24-1.55 (m, 15H), 1.58-1.74 (m, 6H), 1.80 (m, 2H), 1.98 (m, 3H), 2.21 (m, 1H), 2.57 (dd, 1H), 2.70 (dd, 1H), 3.70 (m, 1H), 4.16 (q, 2H), 4.37 (m, 1H), 6.65 (d, 1 H); LRMS : m/z (ES⁺) 542 [MH⁺].

### Preparation 60

### Ethyl (3S)-3-[({1-[(2R)-2-(tert-butoxycarbonyl)pentyl]cyclopentyl}carbonyl)amino]-5-{[tert-butyl(dimethyl)silyl]oxy}pentanoate

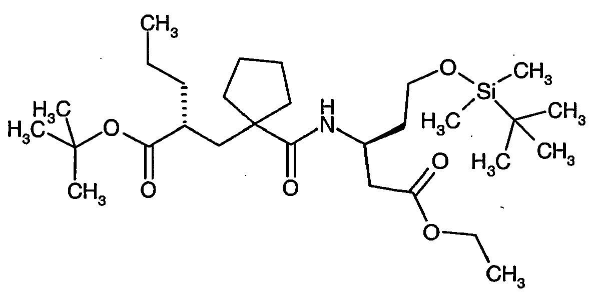

The title compound was obtained as a colourless oil in 56% yield from 1-[(2*S*)-2-(*tert-*butyoxycarbonyl-pentyl)]-cyclopentanecarboxylic acid (WO 0202513, preparation 2) and the amine from preparation 58, following a similar procedure to that described in preparation 59, except the compound was purified by column chromatography on silica gel using pentane:ethyl acetate (90:10) and re-columned using an elution gradient of toluene:ethyl acetate (94:4 to 88:12); ¹H NMR (CDCl₃, 400MHz) δ: 0.05 (s, 6H), 0.86 (m, 12H), 1.26 (t, 3H), 1.43 (m, 13H), 1.59-1.85 (m, 8H), 1.98 (m, 3H), 2.20 (m, 1H), 2.56 (dd, 1H), 2.63 (dd, 1 H), 3.70 (m, 1H), 4.14 (q, 2H), 4.37 (m, 1H), 6.60 (d, 1H).
LRMS : m/z (ES⁺) 542 [MH⁺]; [α]_{D} = -19.88 (c = 0.156 in methanol).

### Preparation 61

### tert-Butyl (3R)-3-[({1-[(2S)-2-(tert-butoxycarbonyl)-4-methoxybutyl]cyclopentyl}carbonyl)-amino]-5-{[tert-butyl(dimethyl)silyl]oxy}pentanoate

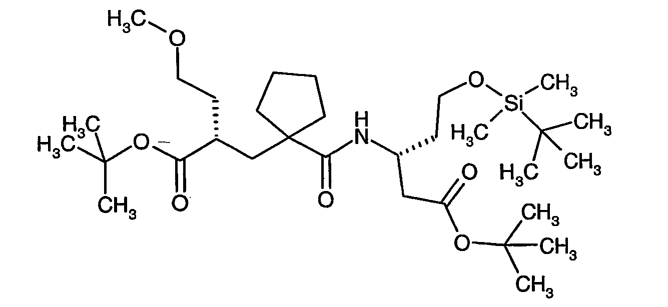

The title compound was obtained as a viscous oil in 84% yield, from (*R*) *tert*-butyl-3-amino-5-(*tert*-butyl-dimethylsilyloxy)-pentanoate (J. Org. Chem. 57(7); 1992; 2120) and the acid from preparation 1, following the procedure described in preparation 59; ¹H NMR (CDCl₃, 400MHz) δ: 0.03 (s, 6H), 0.90 (s, 9H), 1.42 (2xs, 18H), 1.61 (m, 7H), 1.80 (m, 4H), 1.98 (m, 3H), 2.35 (m, 1H), 2.44 (dd, 1H), 2.60 (dd, 1H), 3.26 (s, 3H), 3.36 (m, 2H), 3.68 (m, 2H), 4.26 (m, 1H), 6.65 (d, 1 H); LRMS : m/z (ES⁺) 608 [MNa⁺]; Microanalysis found: C, 63.55; H, 10.15; N, 2.39. C₃₁H₅₉NO₇Si requires C, 63.36; H, 10.05; N, 2.57%.

### Preparation 62

### Ethyl (3S)-3-[({1-[(2S)-2-(tert-butoxycarbonyl)-4-methoxybutyl]cyclopentyl}carbonyl)-amino]-5-{[tert-butyl(dimethyl)silyl]oxy}pentanoate

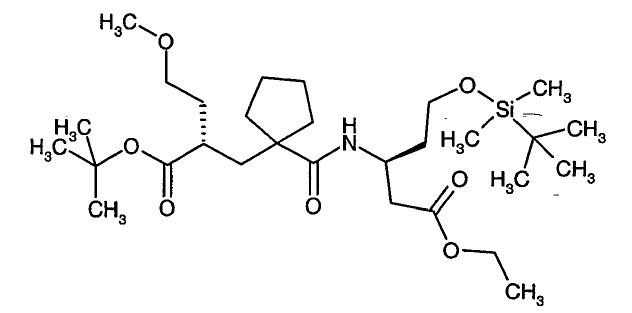

The title compound was obtained as a colourless oil in 87% yield from the acid from preparation 1 and the amine from preparation 58, following a similar procedure to that described in preparation 59, except the compound was purified by column chromatography on silica gel using pentane:ethyl acetate (90:10 to 75:25); ¹H NMR (CDCl₃, 400MHz) δ: 0.05 (s, 6H), 0.90 (s, 9H), 1.25 (t, 3H), 1.40 (s, 9H), 1.45 (m, 1H), 1.66 (m, 6H), 1.80 (m, 4H), 1.95 (m, 3H), 2.20 (m, 1H), 2.55 (m, 1H), 2.65 (m, 1H), 3.25 (s, 3H), 3.30 (m, 2H), 3.70 (m, 2H), 4.10 (q, 2H), 4.35 (m, 1H), 6.60 (d, 1H);

### Preparation 63

### Ethyl (3R)-3-[({1-[(2R)-2-(tert-butoxycarbonyl)pentyl]cyclopentyl}carbonyl)amino]-5-hydroxypentanoate

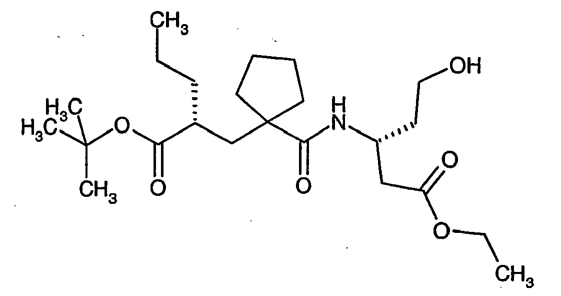

A mixture of the protected alcohol from preparation 59 (1g, 1.85mmol), and acetic acid (10ml) in water (5ml) and tetrahydrofuran (5ml) was stirred at room temperature for 2 hours. The reaction was concentrated under reduced pressure, the residue dissolved in ethyl acetate, washed with saturated sodium carbonate solution, dried (MgSO₄) and concentrated under reduced pressure. The residue was purified by column chromatography on silica gel using dichloromethane:methanol:0.88 ammonia (99:1:0.1) to afford the title compound as a colourless oil, 550mg; LRMS : m/z (ES⁺) 450 [MNa⁺].

### Preparation 64

### Ethyl (3S)-3-[({1-[(2R)-2-(tert-butoxycarbonyl)pentyl]cyclopentyl}carbonyl)amino]-5-hydroxypentanoate

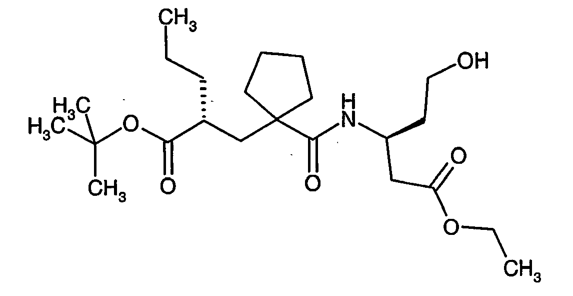

The title compound was obtained in 91% yield from the compound from preparation 60, following a similar procedure to that described in preparation 63; ¹H NMR (CDCl₃, 400MHz) δ: 0.87 (m, 3H), 1.28 (m, 5H), 1.40-1.84 (m, 19H), 1.98 (m, 3H), 2.22 (m, 1H), 2.59 (m, 2H), 2.98 (m, 1 H), 3.60 (m, 2H), 4.18 (m, 2H), 4.40 (m, 1H), 6.94 (d, 1H); LRMS : m/z (ES⁺) 450 [MNa⁺].

### Preparation 65

### tert-Butyl (3R)-3-[({1-[(2S)-2-(tert-butoxycarbonyl)-4-methoxybutyl]cyclopentyl}-carbonyl)amino]-5-hydroxypentanoate

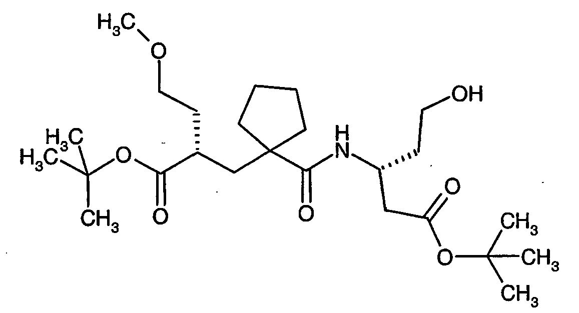

The title compound was obtained as a viscous oil, from the compound from preparation 61, following a similar procedure to that described in preparation 63, but without chromatography; ¹H NMR (CDCl₃, 400MHz) δ: 1.43 (2xs, 18H), 1.46-1.83 (m, 9H), 1.95-2.08 (m, 3H), 2.38 (m, 1 H), 2.44 (dd, 1H), 2.62 (dd, 1H), 2.72 (m, 2H), 3.28 (s, 3H), 3.35 (q, 2H), 3.50 (m, 1H), 3.60 (m, 1H), 4.36 (m, 1H), 7.15 (d, 1H).

### Preparation 66

### Ethyl- (3S)-3-[({1-[(2S)-2-(tert-butoxycarbonyl)-4-methoxybutyl]cyclopentyl}-carbonyl)amino]-5-hydroxypentanoate

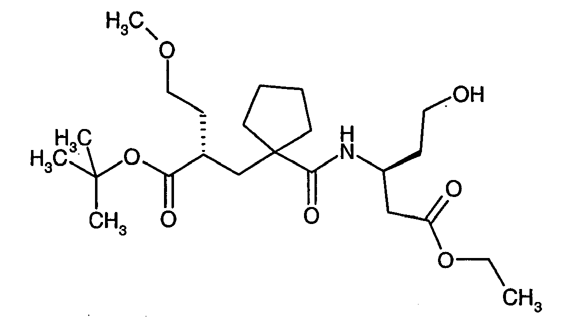

The title compound was obtained as a colourless oil, from the compound from preparation 62, following a similar procedure to that described in preparation 63, but without chromatography; ¹H NMR (CDCl₃, 400MHz) δ: 1.25 (t, 3H), 1.42 (s, 9H), 1.50-2.01 (m, 14H), 2.38 (m, 1H), 2.56 (dd, 1H), 2.62 (dd, 1 H), 3.30 (s, 3H), 3.37 (m, 2H), 3.62 (m, 2H), 4.15 (q, 2H), 4.41 (m, 1H), 6.96 (d, 1 H); LRMS : m/z (ES⁺) 466 [MNa⁺].

### Preparation 67

### Ethyl (3R)-3-[({1-[(2R)-2-(tert-butoxycarbonyl)pentyl]cyclohentyl}carbonyl)amino]-5-phenoxypentanoate

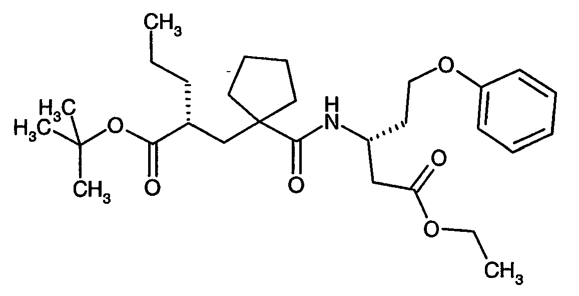

Diisopropyl azodicarboxylate (60µl. 0.30mmol) was added to a solution of triphenylphosphine (79mg, 0.30mmol), phenol (28mg, 0.30mmol) and the alcohol from preparation 63 (100mg, 0.23mmol) in tetrahydrofuran (5ml), and the mixture stirred at room temperature for 18 hours. The mixture was concentrated under reduced pressure, the residue dissolved in dichloromethane and washed with saturated sodium carbonate solution. The organic solution was then dried (MgSO₄) and concentrated under reduced pressure. The residue was purified by column chromatography on silica gel using an elution gradient of ethyl acetate:pentane (87.5:12.5 to 80:20) to afford the title compound as a colourless residue, 60mg; ¹H NMR (CDCl₃, 400MHz) δ : 0.84 (t, 3H), 1.24 (m, 6H), 1.40-1.78 (m, 17H), 1.99 (m, 3H), 2.14 (m, 2H), 2.22 (m, 1H), 2.62 (dd, 1 H), 2.78 (dd, 1 H), 4.02 (m, 2H), 4.19 (q, 2H), 4.44 (m, 1 H), 6.78 (d, 1H), 6.90 (d, 2H), 6.98 (m, 1H), 7.25 (m, 2H); LRMS : m/z (ES⁺) 526 [MNa⁺].

### Preparations 68 to 74

The following compounds of general formula: 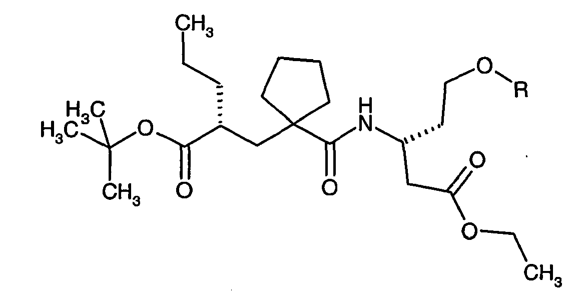 were prepared from the alcohol from preparation 63 and the corresponding alcohols, following a similar procedure to that described in preparation 67.

| Prep. No | R | Data |
|---|---|---|
| 68 | 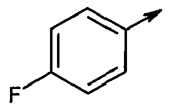 | ¹H NMR (CDCl₃, 400MHz) δ: 0.83 (t, 3H), 1.25 (m, 7H), 1.40-1.77 (m, 15H), 1.98 (m, 3H), 2.05 (m, 3H), 2.21 (m, 1H), 2.60 (dd, 1H), 2.72 (dd, 1H), 3.99 (m, 2H), 4.16 (q, 2H), 4.43 (m, 1H), 6.68 (d, 1H), 6.82 (m, 2H), 6.97 (m 2H);LRMS : m/z (ES⁺) 544 [MNa⁺] |
| 69 | 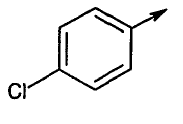 | ¹H NMR (CDCl₃, 400MHz) δ: 0.83 (t, 3H), 1.25 (m, 7H), 1.40-1.75 (m, 16H), 1.98 (m, 3H), 2.05 (m, 2H), 2.20 (m, 1H), 2.60 (dd, 1H), 2.68 (dd, 1 H), 4.00 (m, 2H), 4.16 (q, 2H), 4.43 (m, 1H), 6.68 (d, 1 H), 6.80 (d, 2H), 7.20 (d, 2H);LRMS : m/z (ES⁺) 560 [MNa⁺]; Microanalysis found: C 64.49; H, 8.22; N, 2.57. C₂₉H₄₄CINO₆ requires C, 64.73; H, 8.24; N, 2.60%. |
| 70 | 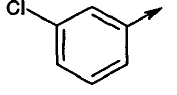 | ¹H NMR (CDCl₃, 400MHz) δ: 0.83 (t, 3H), 1.18-2.10 (m, 28H), 2.22 (m, 1 H), 2.57 (dd, 1H), 2.70 (dd, 1 H), 3.46 (m, 1 H), 3.60 (m, 1 H), 4.18 (q, 2H), 4.38 (m, 1 H), 7.03 (d, 1 H), 7.40-7.74 (m, 4H); LRMS : m/z (ES⁺) 538.4 [MH⁺] |
| 71a | 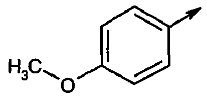 | ¹H NMR (CDCl₃, 400MHz) δ: 0.84 (t, 3H), 1.23 (m, 7H), 1.40-1.77 (m, 16H), 1.98 (m, 3H), 2.04 (m, 2H), 2.20 (m, 1 H), 2.58 (dd, 1 H), 2.72 (dd, 1 H), 3.77 (s, 3H), 4.00 (m, 2H), 4.17 (q, 2H), 4.42 (m, 1 H), 6.68 (d, 1H), 6.81 (s, 4H). |
| 72 | 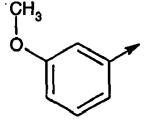 | ¹H NMR (CDCl₃, 400MHz) δ: 0.82 (t, 3H), 1.20-1.78 (m, 23H), 1.98 (m, 3H), 2.05 (m, 2H), 2.20 (m, 1 H), 2.60 (dd, 1 H), 2.74 (dd, 1 H), 3.79 (s, 3H), 4.00 (m, 2H), 4.16 (q, 2H), 4.42 (m, 1 H), 6.43 (m, 3H), 6.72 (d, 1 H), 7.18 (dd, 1 H); LRMS : m/z (APCI⁺) 534 [MH⁺] |
| 73 | 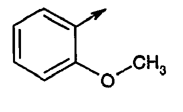 | ¹H NMR (CDCl₃, 400MHz) δ: 0.81 (t, 3H), 1.22-1.62 (m, 22H), 1.75 (dd, 1 H), 1.90-2.02 (m, 3H), 2.10-2.24 (m, 3H), 2.59 (dd, 1 H), 2.77 (dd, 1 H), 3.84 (s, 3H), 4.01-4.18 (m, 4H), 4.50 (m, 1 H), 6.79 (d, 1 H), 6.84-6.98 (m, 4H); LRMS : m/z (ES⁺) 556 [MNa⁺] |
| 74 | 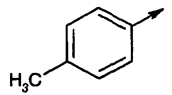 | LRMS : m/z (ES⁺) 540 [MNa⁺] |

| | | |
|---|---|---|
| a = the product was further triturated with pentane to remove impurities | | |

### Preparations 75 to 79

The following compounds of general formula: 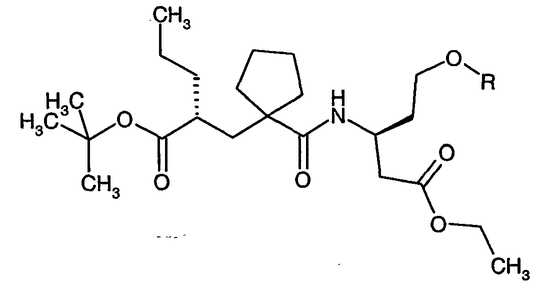 were prepared from the alcohol from preparation 64 and the corresponding alcohols, following a similar procedure to that described in preparation 67.

| Prep. No | R | Data |
|---|---|---|
| 75 | 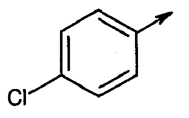 | ¹H-NMR (CDCl₃, 400MHz) δ: 0.81 (t, 3H), 1.15-1.34 (m, 7H), 1.40-1.78 (m, 15H), 1.84-2.20 (m, 7H), 2.60 (m, 2H), 4.00 (m, 2H), 4.16 (q, 2H), 4.43 (m, 1 H), 6.62 (d, 1 H), 6.80 (d, 2H), 7.20 (d, 2H); HRMS : m/z (ES⁺) 538.2924 [MH⁺] |
| 76 | 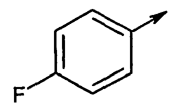 | ¹H-NMR (CDCl₃, 400MHz) δ: 0.81 (t, 3H), 1.18-1.38 (m, 7H), 1.40-1.78 (m, 17H), 1.86-2.14 (m, 4H), 2.20 (m, 1 H), 2.60 (m, 2H), 4.00 (t, 2H), 4.18 (q, 2H), 4.45 (m, 1 H), 6.60 (d, 1 H), 6.80 (dd, 2H), 7.20 (dd, 2H). LRMS : m/z (ES⁺) 544 [MNa⁺] |
| 77^{a} | 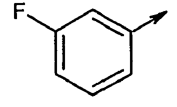 | ¹H-NMR (CDCl₃, 400MHz) δ: 0.80 (t, 3H), 1.15-1.30 (m, 7H), 1.38-1.78 (m, 17H), 1.82-2.20 (m, 5H), 2.60 (m, 2H), 4.00 (t, 2H), 4.16 (q, 2H), 4.42 (m, 1 H), 6.60 (m, 4H), 7.18 (m, 1H). LRMS : m/z (ES⁺) 544 [MNa⁺] |
| 78^{a} | 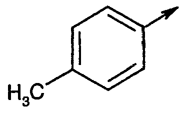 | ¹H-NMR (CDCl₃, 400MHz) δ: 0.82 (t, 3H), 1.22 (m, 6H), 1.40-1.68 (m, 16H), 1.74 (dd, 1 H), 1.86-2.00 (m, 3H), 2.08 (m, 2H), 2.19 (m, 1 H), 2.24 (s, 3H), 2.58 (dd, 1 H), 2.64 (dd, 1 H), 4.00 (t, 2H), 4.16 (q, 2H), 4.44 (m, 1 H), 6.64 (d, 1 H), 6.78 (d, 2H), 7.03 (d, 2H). LRMS : m/z (ES⁺) 540 [MNa⁺] |
| 79^{a} | 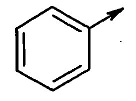 | ¹H-NMR (CDCl₃, 400MHz) δ: 0.82 (t, 3H), 1.16-1.36 (m, 6H), 1.40-1.68 (m, 16H), 1.75 (dd, 1H), 1.86-2.21 (m, 6H), 2.59 (dd, 1H), 2.65 (dd, 1 H), 4.00 (t, 2H), 4.16 (q, 2H), 4.46 (m, 1H), 6.68 (m, 1H), 6.84 (d, 2H), 6.95 (m, 1H), 7.22 (d, 2H). LRMS : m/z (ES⁺) 526 [MNa⁺] |

| | | |
|---|---|---|
| a= dichloromethane was used instead of ethyl acetate in the work-up | | |

### Preparation 80

### tert-Butyl (3R)-3-[({1-[(2S)-2-(tert-butoxycarbonyl)-4-methoxybutyl]cyclopentyl}-carbonyl)amino]-5-(4-chlorophenoxy)pentanoate

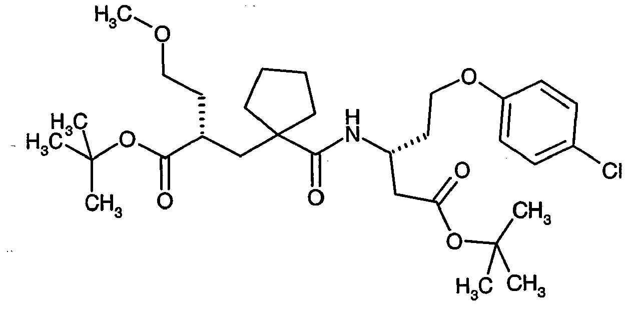

The title compound was obtained as an oil from the alcohol from preparation 65 and p-chlorophenol, following the procedure described in preparation 67; ¹H-NMR (CDCl₃, 400MHz) δ: 1.24 (2xs, 18H), 1.55-1.80 (m, 9H), 1.98 (m, 3H), 2.03 (m, 2H), 2.37 (m, 1 H), 2.53 (dd, 1 H), 2.60 (dd, 1 H), 3.26 (m, 5H), 4.00 (m, 2H), 4.39 (m, 1 H), 6.70 (d, 1 H), 6.80 (d, 2H), 7.20 (d, 2H); LRMS : m/z (ES⁺) 604 [MNa⁺].

### Preparation 81

### Ethyl (3S)-3-[({1-[(2S)-2-(tert-butoxvcarbonyl)-4-methoxybutyl]cyclopentyl}-carbonyl)amino]-5-(4-chlorophenoxy)aentanoate

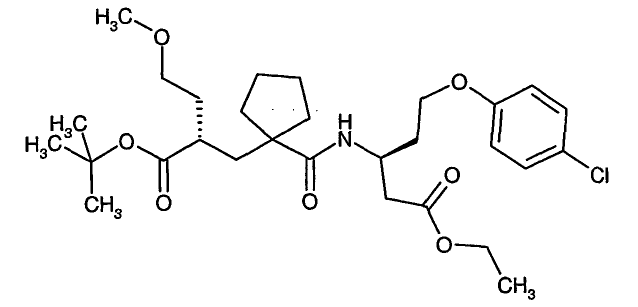

The title compound was obtained as an oil from the alcohol from preparation 66 and p-chlorophenol, following the procedure described in preparation 67, except the crude product was purified by column chromatography on silica gel using an elution gradient of toluene:methanol (99:1 to 98:2) and re-columned using an elution gradient of ether:pentane (25:75 to 80:20); ¹H-NMR (CDCl₃, 400MHz) δ: 1.25 (t, 3H), 1.40-1.70 (m, 16H), 1.80 (m, 2H), 1.98 (m, 3H), 2.08 (m, 2H), 2.30 (m, 1 H), 2.62 (m, 2H), 3.25 (m, 5H), 4.01 (m, 2H), 4.18 (q, 2H), 4.44 (m, 1H), 6.63 (d, 1 H), 6.82 (d, 2H), 7.20 (d, 2H); LRMS : m/z (APCI⁺) 554 [MH⁺]; [α]_{D} = -9.67 (c = 0.12, methanol).

### Preparation 82

### (2R)-2-({1-[({(1R)-1-[2-(3-Chlorophenoxy)ethyl]-3-ethoxy-3-oxopropyl}amino)carbonyl]-cyclopentyl}methyl)pentanoic acid

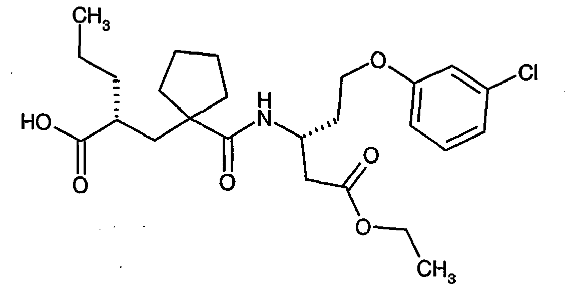

A solution of the ester from preparation 69 (45mg, 0.08mmol) and trifluoroacetic acid (0.5ml) in dichloromethane (2ml) was stirred at room temperature for 18 hours. The reaction was concentrated under reduced pressure, and the residue azeotroped with toluene to afford the title compound, 40mg; LRMS : m/z (ES⁺) 482 [MH]⁺.

### Preparations 83 to 85

The following compounds of general formula: 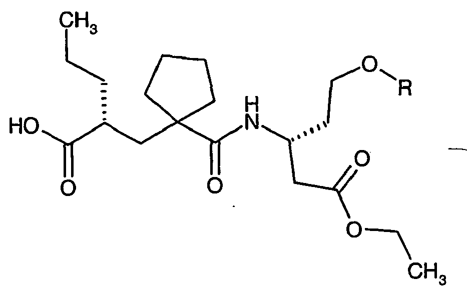 were prepared as colourless oils, from the corresponding *tert*-butyl esters following a similar procedure to that described in preparation 82.

| Prep. no | R | Data |
|---|---|---|
| 83 | 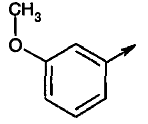 | LRMS : m/z (ES⁻) 476 [M-H]⁻ |
| 84 | 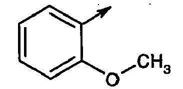 | LRMS : m/z (ES⁻) 476 [M-H]⁻ |
| 85^{a} | 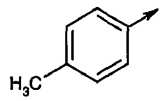 | |

| | | |
|---|---|---|
| a = The product was additionally dissolved in ethyl acetate, washed with water, dried (MgSO₄) and evaporated under reduced pressure. | | |

### Preparation 86

### (2R)-2-({1-[({(1S)-1-[2-(4-Chlorophenoxy)ethyl]-3-ethoxy-3-oxopropyl}amino)carbonyl]-cyclopentyl}methyl)pentanoic acid

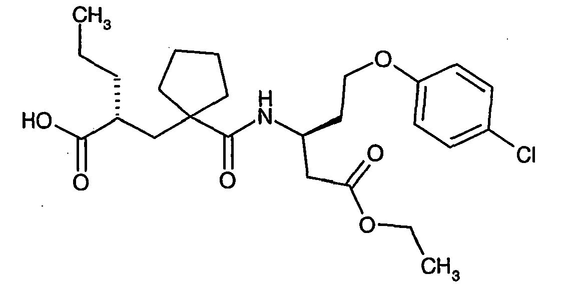

A solution of the ester from preparation 81 (390mg, 0.72mmol) in dichloromethane (4ml) and trifluoroacetic acid (2ml) was stirred at room temperature for 3 hours. The mixture was concentrated under reduced pressure and the residue purified by column chromatography on silica gel using an elution gradient of dichloromethane:methanol (99:1 to 95:5) to afford the title compound as a colourless oil, 249mg; ¹H NMR (CDCl₃, 400MHz) δ: 0.84 (t, 3H), 1.19-1.39 (m, 6H), 1.44-1.80 (m, 7H), 1.93-2.14 (m, 6H), 2.30 (m, 1 H), 2.62 (m, 2H), 3.99 (t, 2H), 4.16 (q, 2H), 4.44 (m, 1H), 6.76 (d, 1 H), 6.80 (d, 2H), 7.20 (d, 2H); LRMS : m/z (ES⁺) 482 [MH⁺].

### Preparations 87 to 90

The following compounds of general formula: 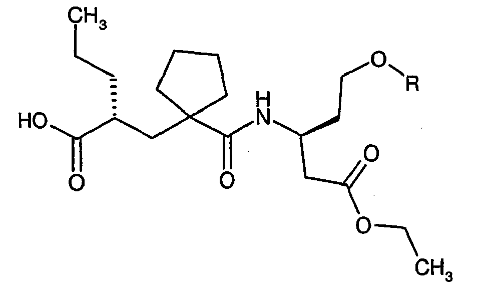 were prepared from the corresponding esters, following the procedure described in preparation 86.

| Prep. no | R | Data |
|---|---|---|
| 87 | 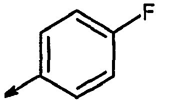 | ¹H NMR (CDCl₃, 400MHz) δ: 0.82 (t, 3H), 1.22-1.39 (m, 7H), 1.46-1.70 (m, 6H), 1.78 (dd, 1 H), 1.96-2.12 (m, 5H), 2.32 (m, 1 H), 2.62 (m, 2H), 4.00 (t, 2H), 4.16 (q, 2H), 4.44 (m, 1 H), 6.76 (d, 1H), 6.80 (dd, 2H), 6.97 (dd, 1 H); LRMS : m/z (ES⁺) 488 [MNa⁺] |
| 88 | 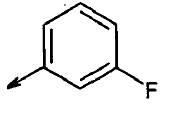 | ¹H NMR (CDCl₃, 400MHz) δ: 0.82 (t, 3H), 1.20-1.39 (m, 7H), 1.43-1.70 (m, 6H), 1.78 (dd, 1 H), 1.96-2.12 (m, 5H), 2.30 (m, 1 H), 2.62 (m, 2H), 4.00 (t, 2H), 4.16 (q, 2H), 4.44 (m, 1H), 6.00 (m, 3H), 6.74 (d, 1 H), 7.19 (dd, 1 H); LRMS : m/z (ES⁺) 488 [MNa⁺]; [α_{D}] = -19.27 (c = 0.136, methanol) |
| 89 | 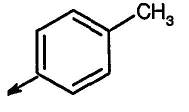 | ¹H NMR (CDCl₃, 400MHz) δ: 0.84 (t, 3H), 1.28 (m, 7H), 1.48-1.78 (m, 7H), 1.96-2.12 (m, 5H), 2.26 (s, 3H), 2.36 (m, 1 H), 2.62 (m, 2H), 4.00 (t, 2H), 4.16 (q, 2H), 4.44 (m, 1H), 6.79 (m, 3H), 7.04 (d, 2H). LRMS : m/z (ES⁻) 460 [M-H]⁻ |
| 90^{a} | 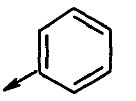 | ¹H NMR (CDCl₃, 400MHz) δ: 0.82 (t, 3H), 1.25 (m, 6H), 1.43-1.80 (m, 8H), 1.96-2.10 (m, 5H), 2.37 (m, 1 H), 2.63 (m, 2H), 4.02 (t, 2H), 4.16 (q, 2H), 4.44 (m, 1 H), 6.78 (d, 1 H), 6.85 (d, 2H), 6.95 (dd, 1 H), 7.25 (m, 2H); LRMS : m/z (ES⁻) 446 [M-H]⁻ |

| | | |
|---|---|---|
| a = the product was additionally purified by reverse phase HPLC using acetonitrile:water:trifluoroacetic acid as gradient eluant. | | |

### Preparation 91

### (3S)-3-[({1-[(2S)-2-(tert-Butoxycarbonyl)-4-methoxybutyl]cyclopentyl}carbonyl)amino]-5-(4-chlorophenoxy)pentanoic acid

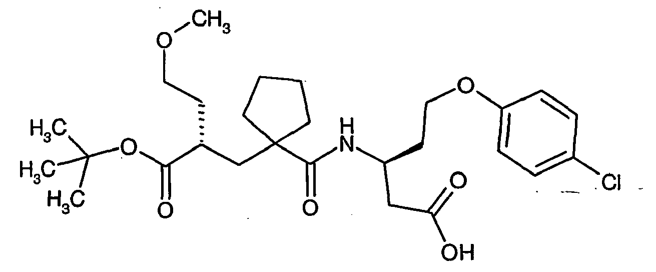

Sodium hydroxide solution (3ml, 2N) was added to a solution of the ester from preparation 81 (500mg, 0.90mmol) in tetrahydrofuran (3ml) and the reaction stirred under reflux for 5 hours. The cooled mixture was partitioned between ethyl acetate (30ml) and 2M hydrochloric acid (10ml), and the layers separated. The aqueous phase was extracted with further ethyl acetate (30ml), and the combined organic solutions washed with brine (20ml), dried (MgSO₄) and evaporated under reduced pressure. The crude product was purified by column chromatography on silica gel using an elution gradient of dichloromethane:methanol (96:4 to 90:10) to afford the title compound as a colourless oil, 298mg; ¹H NMR (CDCl₃, 400MHz) δ: 1.30-1.65 (m, 15H), 1.75 (m, 2H), 1.88 (m, 1 H), 2.00-2.16 (m, 5H), 2.40 (m, 1 H), 2.58 (dd, 1 H), 2.75 (dd, 1 H), 3.30 (m, 1 H), 3.38 (s, 3H), 3.45 (m, 1 H), 4.00 (m, 2H), 4.58 (m, 1 H), 6.08 (d, 1 H), 6.80 (d, 2H), 7.20 (d, 2H); LRMS : m/z (APCI⁺) 526 [MH⁺].

### Preparation 92

### Butyl (3S)-3-[({1-[(2S)-2-(tert-butoxycarbonyl)-4-methoxybutyl]cyclopentyl}-carbonyl)amino]-5-(4-chlorophenoxy)pentanoate

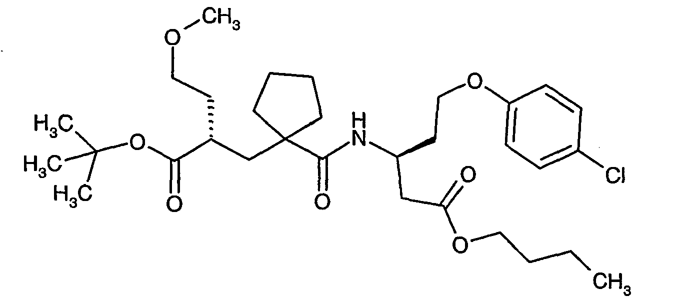

A mixture of the acid from preparation 91 (280mg, 0.53mmol), potassium carbonate (74mg, 0.53mmol) and n-butyl iodide (67µl, 0.59mmol) in N,N-dimethylformamide (2ml) was stirred at room temperature for 18 hours. The mixture was concentrated under reduced pressure and the residue partitioned between ether (50ml) and water (25ml) and the layers separated. The organic phase was washed with 2N sodium hydroxide solution (15ml), then brine (20ml), dried (MgSO₄) and evaporated under reduced pressure. The residual orange oil was dissolved in a minimum volume of dichloromethane and the solution filtered through silica gel washing through with dichloromethane:methanol solution (25ml, 97.5:2.5), and the filtrate evaporated under reduced pressure to afford the title compound as a colourless oil, 289mg; ¹H NMR (CDCl₃, 400MHz) δ: 0.94 (t, 3H), 1.36-1.58 (m, 19H), 1.61 (m, 4H), 1.79 (m, 1H), 1.96-2.03 (m, 2H), 2.08 (m, 1 H), 2.30 (m, 1 H), 2.62 (m, 2H), 3.23 (m, 5H), 4.01 (t, 2H), 4.09 (t, 2H), 4.45 (m, 1 H), 6.66 (d, 1 H), 6.81 (d, 2H), 7.20 (d, 2H); LRMS : m/z (ES⁺) 604 [MNa⁺].

### Preparation 93

### tert-Butyl (3S)-6-(4-chlorophenyl)-3-[(benzyloxycarbonyl)amino]hexanoate

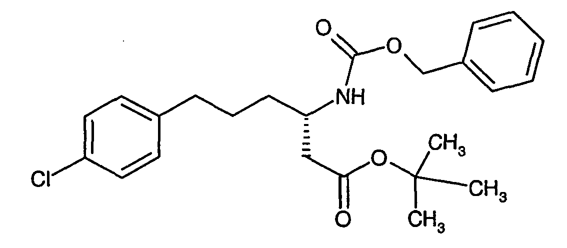

Triethylamine (1.2ml, 8.9mmol) followed by diphenylphosphoryl azide (1.6ml, 7.4mmol) was added to a solution of 4-*tert*-butyl hydrogen 2-(4-chlorophenylpropyl)succinate (WO 9504033 intermediate 2f) (2.4g, 7.4mmol) in toluene (250ml), and the solution stirred at room temperature for 30 minutes, then under reflux for a further 3 hours. The mixture was cooled to 50°C, benzyl alcohol (2.3ml, 22.3mmol) added, and the reaction heated under reflux for 18 hours. The cooled mixture was concentrated under reduced pressure and the residue dissolved in ethyl acetate (125ml). This solution was washed with saturated ammonium chloride solution (100ml), brine (100ml) and water (100ml), then dried (MgSO₄) and concentrated under reduced pressure. The product was purified by column chromatography on silica gel using ethyl acetate :pentane (6:94) as eluant to afford the title compound as a colourless oil 1.59g; ¹H NMR (CDCl₃, 400MHz) δ: 1.41 (s, 9H), 1.46-1.70 (m, 4H), 2.40 (m, 2H), 2.58 (m, 2H), 3.98 (m, 1 H), 5.10 (s, 2H), 5.20 (d, 1 H), 7.05 (d, 2H), 7.22 (m, 3H), 7.32 (m, 4H); LRMS : m/z (ES⁺) 454 [MNa⁺].

### Preparation 94

### (3S)-3-Amino-6-(4-chlorophenyl)hexanoic acid hydrobromide

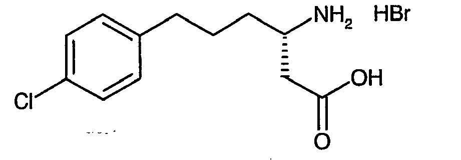

Hydrogen bromide in acetic acid (30wt.%, 50ml) was added to a solution of the protected amino acid from preparation 93 (1.5g, 3.47mmol) in acetic acid (100ml), and the reaction stirred at room temperature for 5 hours. The mixture was concentrated under reduced pressure and the residue azeotroped with dichloromethane to afford the title compound as an orange powder, 1.13g; ¹H NMR (CD₃OD, 400MHz) δ: 1.68 (m, 4H), 2.55-2.78 (m, 4H), 3.57 (m, 1 H), 7.18 (d, 2H), 7.25 (d, 2H); LRMS : m/z (ES⁺) 242 [MH⁺].

### Preparation 95

### tert-Butyl (3S)-3-amino-5-(4-methoxyphenyl)hexanoate

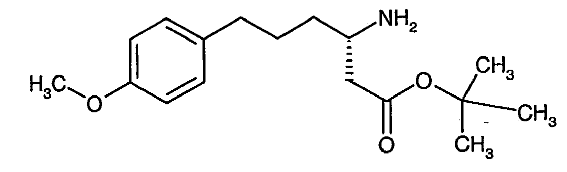

*tert*-Butyl (3*S*)-3-{[(benzyloxy)carbonyl]amino}-5-(4-methoxyphenyl)hexanoate was prepared as a colourless oil in 32% yield from 4-*tert*-butyl hydrogen 2-(4-methoxyphenylpropyl)succinate (WO 9504033 intermediate 4), following the procedure described in preparation 93. A mixture of this compound (110mg, 0.26mmol), 10% palladium on charcoal (20mg) and ethanol (10ml) was hydrogenated at room temperature for 18 hours. The reaction was filtered, and the filtrate concentrated under reduced pressure. The residue was purified by column chromatography on silica gel twice using dichloromethane:methanol:0.88 ammonia (98:2:0.2) to afford the title compound; ¹H NMR (CDCl₃, 400MHz) δ: 1.42 (s, 9H), 1.60-1.80 (m, 4H), 2.20 (dd, 1 H), 2.39 (dd, 1 H), 2.58 (t, 2H), 3.18 (m, 1 H), 3.79 (s, 3H), 6.82 (d, 2H), 7.07 (d, 2H); LRMS : m/z (ES⁺) 294 [MH⁺].

### Preparation 96

### Ethyl (3S)-3-amino-6-(4-chlorophenyl)hexanoate hydrochloride

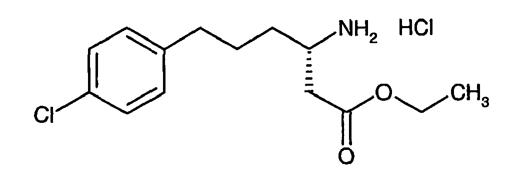

Ethanolic hydrogen chloride (45ml) was added to a solution of the acid from preparation 94 (1.06g, 3.29mmol) in ethanol (45ml), and the reaction stirred at room temperature for 6 hours. The mixture was evaporated under reduced pressure to afford the title compound as an orange oil, 1.1g; ¹H NMR (CD₃OD, 400MHz) δ: 1.24 (t, 3H), 1.68 (m, 4H), 2.58-2.80 (m, 4H), 3.58 (m, 1H), 4.19 (q, 2H), 7.20 (d, 2H), 7.25 (d, 2H).

### Preparation 97

### Ethyl (3S)-3-[({1-[(2S)-2-(tert-butoxycarbonyl)-4-methoxybutyl]cyclopentyl}-carbonyl)amino]-6-(4-chlorophenyl)hexanoate

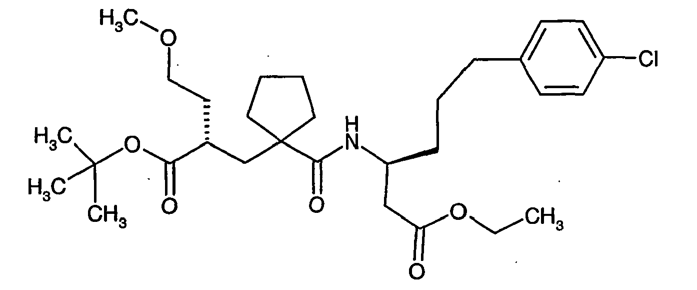

1-Hydroxybenzotriazole hydrate (486mg, 3.60mmol), 1-(3-dimethylaminopropyl)-3-ethylcarbodiimide hydrochloride (690mg, 3.60mmol) and N-methyl morpholine (1.32g, 13.08mmol) were added to a solution of the amine from preparation 96 (1.0g, 3.27mmol), and the acid from preparation 1 (980mg, 3.27mmol) in dichloromethane (50ml), and the reaction stirred at room temperature for 18 hours. The reaction was diluted with dichloromethane (100ml), washed with brine (2x100ml), dried (MgSO₄) and evaporated under reduced pressure. The residual gum was purified by column chromatography on silica gel using pentane:ethyl acetate (85:15) to give the title compound, 1.31g; ¹H NMR (CDCl₃, 400MHz) δ: 1.24 (t, 3H), 1.44-1.70 (m, 21 H), 1.79 (m, 2H), 1.98 (m, 3H), 2.36 (m, 2H), 2.60 (m, 2H), 3.24 (m, 5H), 4.12 (q, 2H), 4.26 (m, 1 H), 6.42 (d, 1H), 7.10 (d, 2H), 7.21 (d, 2H).

### Preparation 98

### tert-butyl (3S)-3-[({1-[(2S)-2-(tert-butoxycarbonyl)-4-methoxybutyl]cyclopentyl}-carbonyl)amino]-6-(4-methoxyphenyl)hexanoate

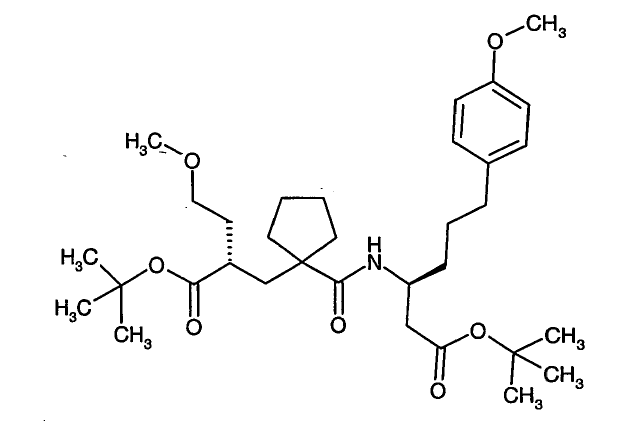

The title compound was obtained in 67% yield from the amine from preparation 95 and the acid from preparation 1, following the procedure described in preparation 97; ¹H NMR (CDCl₃, 400MHz) δ: 1.42 (2xs, 18H), 1.44-1.70 (m, 12H), 1.80 (m, 2H), 1.98 (m, 2H), 2.37 (m, 1H), 2.40 (m, 2H), 2.58 (m, 2H), 3.22 (s, 3H), 3.27 (m, 2H), 3.78 (s, 3H), 4.23 (m, 1H), 6.44 (d, 1H), 6.80 (d, 2H), 7.08 (d, 2H); LRMS : m/z (ES⁺) 598 [MNa⁺].

### Preparation 99

### tert-Butyl (2R)-2-[(1-{[((1R)-1-{[(4-chlorobenzyl)oxy]methyl}-3-ethoxy-3-oxopropyl)-amino]carbonyl}cyclopentyhl)methyl]pentanoate

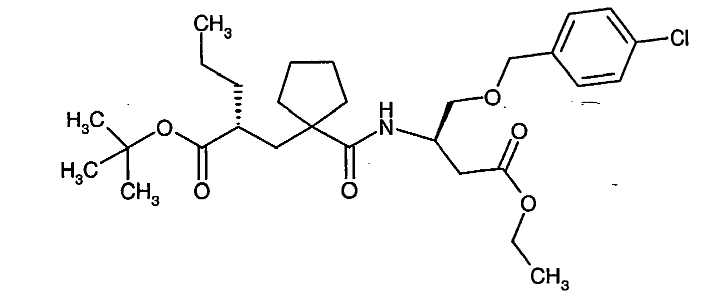

The title compound was prepared as a colourless oil in 77% yield, from the acid from preparation 15 and ethyl iodide, following the procedure described in preparation 24.
¹H NMR (CDCl₃, 400MHz) δ: 0.83 (t, 3H), 1.19-1.78 (m, 23H), 2.98 (m, 3H), 2.21 (m, 1 H), 2.61 (m, 2H), 3.58 (m, 2H), 4.06 (t, 2H), 4.45 (s, 3H), 6.50 (m, 1H), 7.25 (m, 4H). LRMS : m/z (ES⁺) 560 [MNa⁺].

### Biological Assays

IC₅₀ values of the compounds of the invention against NEP and ACE were determined using methods described in published patent application EP1097719-A1, paragraphs [0368] to [0376]. The IC₅₀ values presented below were determined using NEP (EC.3.4.24.11) from human kidney.

The compounds of the invention are potent inhibitors of NEP and are selective against ACE.

The title compounds of all diacid examples showed an IC₅₀ against NEP of less than 250 nM.

The title compounds of Examples 1-5, 7, 10-16, 31-42, 44, 46, 48, 53 and 54 showed an IC₅₀ against NEP of less than or equal to 50 nM and a selectivity over ACE of greater than 300 fold.

In particular, the title compound of Example 16 showed an IC₅₀ against NEP of 8.2 nM; the title compound of Example 41 showed an IC₅₀ against NEP of 6.3 nM; the title compound of Example 48 showed an IC₅₀ against NEP of 2.4 nM; the title compound of Example 53 showed an IC₅₀ against NEP of 0.7 nM; and the title compound of Example 54 showed an IC₅₀ against NEP of 4.7 nM. The title compounds of all these Examples were greater than 300 fold selective against ACE.

## Claims

1. A compound of formula (I), a pharmaceutically acceptable salt or solvate thereof 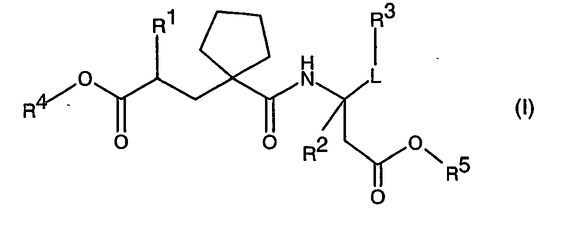 wherein
R¹ is C₁-C₆alkyl, C₁-C₆alkoxyC₁-C₃alkyl or C₁-C₆alkoxyC₁-C₆alkoxyC₁-C₃alkyl;
R² is hydrogen or C₁-C₆alkyl;
L is a three atom linkage selected from -CH₂-X-CH₂- and -CH₂-CH₂-X- where the right hand side of the linkage is attached to R³ and where X is oxygen, sulfur or methylene;
R³ is phenyl or aromatic heterocyclyl, either of which may be independently substituted by one or more groups selected from: C₁-C₆alkyl, halo, haloC₁-C₆alkyl, C₁-C₆alkoxy, haloC₁-C₆alkoxy, C₁-C₆alkylthio, haloC₁-C₆alkylthio and nitrile; and
R⁴ and R⁵ are either both hydrogen, or one of R⁴ and R⁵ is hydrogen and the other is a biolabile ester-forming group that in the body of a patient is replaced by hydrogen.

2. A compound according to claim 1 wherein R¹ is C₁-C₆alkyl or C₁-C₆alkoxyC₁₋C₃alkyl.

3. A compound according to claim 2 wherein R¹ is propyl or methoxyethyl.

4. A compound according to any preceding claim wherein R² is hydrogen.

5. A compound according to any preceding claim wherein L is a three atom linkage selected from -CH₂-O-CH₂-, -CH₂-CH₂-O- and CH₂-CH₂-CH₂-, where the right hand side of the linkage is attached to R³.

6. A compound according to claim 5 wherein L is -CH₂-CH₂-O- or CH₂-CH₂-CH₂-.

7. A compound according to any preceding claim wherein R³ is phenyl which may be independently substituted by one or more groups selected from: C₁-C₆alkyl, halo, haloC₁-C₆alkyl, C₁-C₆alkoxy, haloC₁-C₆alkoxy, C₁-C₆alkylthio, haloC₁₋C₆alkylthio and nitrile.

8. A compound according to claim 7 wherein R³ is phenyl which may be independently substituted by one or more groups selected from: C₁-C₆alkyl, halo, haloC₁-C₆alkyl, C₁-C₆alkoxy and halo C₁-C₆alkoxy.

9. A compound according to claim 8 wherein R³ is 4-fluorophenyl, 4-chlorophenyl, 4-methoxyphenyl or 4-methylphenyl.

10. A compound according to any preceding claim wherein the biolabile ester-forming groups are C₁-C₆alkyl, carbocyclyl or heterocyclyl each of which may be substituted.

11. A compound according to claim 10 wherein the biolabile ester-forming groups are: i) C₁-C₆alkyl optionally substituted by hydroxy, oxo, halo, haloC₁-C₆alkyl, C₁₋C₆alkoxy, haloC₁-C₆alkoxy, C₁-C₆alkylthio, haloC₁-C₆alkylthio, nitrile, carbocyclyl, heterocyclyl, carbocyclyloxy, heterocyclyloxy, C₁-C₇alkylcarbonyloxy, carbocyclylcarbonyloxy, heterocyclylcarbonyloxy, alkylcarbonylamino, and alkylaminocarbonyl, wherein any carbocyclyl or heterocyclyl group is optionally substituted by C₁-C₆alkyl, halo, haloC₁-C₆alkyl, C₁-C₆alkoxy, haloC₁-C₆alkoxy, C₁₋C₆alkylthio, haloC₁-C₆alkylthio or nitrile; or ii) carbocyclyl or heterocyclyl optionally substituted by C₁-C₆alkyl, halo, haloC₁-C₆alkyl, C₁-C₆alkoxy, haloC₁-C₆alkoxy, C₁₋C₆alkylthio, haloC₁-C₆alkylthio or nitrile.

12. A compound according to claim 11 wherein any carbocyclic group is phenyl and any heterocyclic group is aromatic.

13. A compound according to claim 11 wherein biolabile ester-forming groups are selected from the list: ethyl, propyl, butyl, isobutyl, cyclopentyl, benzyl, 1-(2,2-diethylbutyryloxy)ethyl, 2-ethylpropionyloxymethyl, 1-(2-ethylpropionyloxy)ethyl, 1-(2,4-dimethylbenzoyloxy)ethyl, 1-benzoyloxy)benzyl, 1-(benzoyloxy)ethyl, 2-methyl-1-propionyloxypropyl, 2,4,6-trimethylbenzoyloxymethyl, 1-(2,4,6-trimethylbenzyloxy)ethyl, pivaloyloxymethyl, phenethyl, phenpropyl, 2,2,2-trifluororethyl, 1-naphthyl, 2-naphthyl, 2,4-dimethylphenyl, 4-t-butylphenyl, 5-(4-methyl-1,3-dioxalynyl-2-onyl)methyl, N,N-diethylaminocarbonylmethyl and 5-indanyl.

14. A compound according to any preceding claim wherein R⁴ and R⁵ are both hydrogen.

15. A compound according to any preceding claim wherein the compound is of formula (Ia)

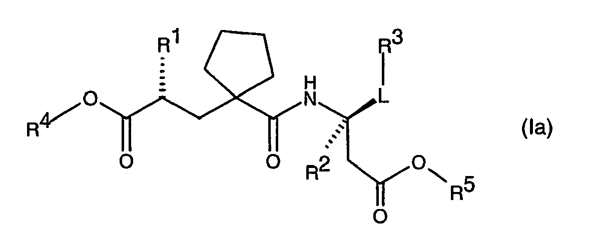

16. A compound according to claim 15 wherein
R¹ is C₁-C₆alkyl or C₁-C₆alkoxyC₁-C₃alkyl;
R² is hydrogen;
L is a three atom linkage selected from: -CH₂-O-CH₂-, -CH₂-CH₂-O- and CH₂-CH₂-CH₂-, where the right hand side of the linkage is attached to R³;
R³ is phenyl which may be independently substituted by one or more groups selected from: C₁-C₆alkyl, halo, haloC₁-C₆alkyl, C₁-C₆alkoxy, haloC₁-C₆alkoxy, C₁-C₆alkylthio, haloC₁-C₆alkylthio and nitrile; and
R⁴ and R⁵ are either both hydrogen, or one of R⁴ and R⁵ is hydrogen and the other is a biolabile ester-forming group that in the body of a patient is replaced by hydrogen.

17. A compound according to claim 16 wherein
R¹ is propyl or methoxyethyl;
R² is hydrogen;
L is -CH₂-CH₂-O- or CH₂-CH₂-CH₂-, where the right hand side of the linkage is attached to R³;
R³ is 4-fluorophenyl, 4-chlorophenyl, 4-methoxyphenyl or 4-methylphenyl; and
R⁴ and R⁵ are both hydrogen.

18. A compound according to claim 1 selected from:
(2*S*)-2-[(1-{[((1*R*)-2-carboxy-1-{[(4-chlorobenzyl)oxy]methyl}ethyl)-amino]carbonyl}cyclopentyl)methyl]-4-methoxybutanoic acid (Example 16);
(3*S*)-3-[({1-[(2*R*)-2-carboxypentyl]cyclopentyl}carbonyl)amino]-5-(4-chlorophenoxy)pentanoic acid (Example 41);
Ethyl (3*S*)-3-[({1-[(2*S*)-2-Carboxy-4-methoxybutyl]cyclopentyl}carbonyl)amino]-5-(4-chlorophenoxy)pentanoate (Example 47);
(3*S*)-3-[({1-[(2*S*)-2-carboxy-4-methoxybutyl]cyclopentyl}carbonyl)amino]-5-(4-chlorophenoxy)pentanoic acid (Example 48);
(2*S*)-2-({1-[({(1*S*)-3-butoxy-1-[2-(4-chlorophenoxy)ethyl]-3-oxopropyl}amino)carbonyl]cyclopentyl}methyl)-4-methoxybutanoic acid (Example 49);
(3*S*)-3-[({1-[(2*S*)-2-carboxy-4-methoxybutyl]cyclopentyl)carbonyl)amino]-6-(4-chlorophenyl)hexanoic acid (Example 53);
Ethyl (3*S*)-3-[({1-[(*2S*)-2-carboxy-4-methoxybutyl]cyclopentyl}carbonyl)amino]-6-(4-chlorophenyl)hexanoate (Example 52); and
(3*S*)-3-[({1-[(2*S*)-2-carboxy-4-methoxybutyl]cyclopentyl}carbonyl)amino]-6-(4-methoxyphenyl)hexanoic acid (Example 54).

19. The use of a compound defined in any preceding claim, a pharmaceutically acceptable salt, solvate, polymorph or prodrug thereof, in the manufacture of a medicament for treating or preventing a condition for which a beneficial response is obtained by the inhibition of neutral endopeptidase.

20. The use according to claim 19 wherein the condition is a cardiovascular disease or condition.

21. The use according to claim 20 wherein the condition is hypertension.

22. The use according to claim 19 wherein the condition is female sexual dysfunction or male erectile dysfunction.

23. A compound defined in any one of claims 1 to 18, a pharmaceutically acceptable salt, solvate, polymorph or prodrug thereof, for use as a medicament.

24. A pharmaceutical composition including a compound defined in any one of claims 1 to 18, a pharmaceutically acceptable salt, solvate, polymorph or prodrug thereof together with a pharmaceutically acceptable excipient, diluent or carrier.

25. A combination of a compound defined in any one of claims 1 to 18 and one or more active ingredients selected from the list:
a) angiotensin receptor blockers (ARB), such as losartan, valsartan, telmisartan, candesartan, irbesartan, eprosartan and olmesartan;
b) calcium channel blockers (CCB) such as amlodipine;
c) statins, such as atorvastatin;
d) PDES inhibitors, such as sildenafil, tadalafil, vardenafil, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and; the pyrazolo[4,3-d]pyrimidin-4-ones disclosed In WO00/27848 particularly N-[[3-(4,7-dihydro-1-methyl-7-oxo-3-propyl-1H-pyrazolo[4,3-d]-pyrimidin-5-yl)-4-propxyphenyl]sulfonyl]-1-methyl2-pyrrolidinepropanamide [DA-8159 (Example 68 of WO00/27848)];
e) beta blockers, such as atenolol or carvedilol;
f) ACE inhibitors, such as quinapril, enalapril and lisinopril;
g) alpha-blockers such as doxazosin;
h) selective aldosterone receptor antagonists (SARA), such as eplerenone or spironolactone;
i) imidazoline I₁ agonists, such as rilmenidine; and
j) endothelin receptor antagonists and endothelin converting enzyme inhibitors.

## Patentansprüche

1. Verbindung der Formel (I), ein pharmazeutisch annehmbares Salz oder Solvat davon 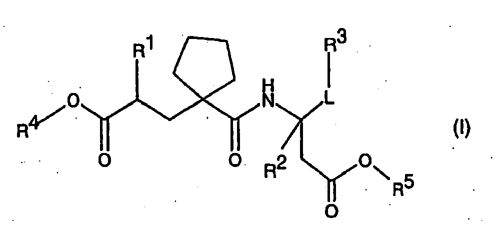 worin
R¹ für C₁-C₆-Alkyl, C₁-C₆-Alkoxy-C₁-C₃-alkyl oder C₁-C₆₋Alkoxy-C₁-C₆-alkoxy-C₁-C₃-alkyl steht;
R² für Wasserstoff oder C₁-C₆-Alkyl steht;
L für eine dreiatomige Verknüpfung, ausgewählt aus -CH₂-X-CH₂- und -CH₂-CH₂-X-, steht, wobei die rechte Seite der Verknüpfung am R³ gebunden ist und wobei X für Sauerstoff, Schwefel oder Methylen steht;
R³ für Phenyl oder aromatisches Heterocyclyl steht, die jeweils unabhängig voneinander substituiert sein können mit einer oder mehreren Gruppen, ausgewählt aus: C₁-C₆-Alkyl, Halogen, Halogen-C₁-C₆-alkyl, C₁-C₆₋Alkoxy, Halogen-C₁-C₆-alkoxy, C₁-C₆-Alkylthio, Halogen-C₁-C₆-alkylthio und Nitril; und
R⁴ und R⁵ entweder beide für Wasserstoff stehen oder eines von R⁴ und R⁵ für Wasserstoff steht und das andere für eine biolabile esterbildende Gruppe steht, die im Körper eines Patienten durch Wasserstoff ersetzt wird.

2. Verbindung nach Anspruch 1, wobei R¹ für C₁-C₆-Alkyl oder C₁-C₆-Alkoxy-C₁-C₃-alkyl steht.

3. Verbindung nach Anspruch 2, wobei R¹ für Propyl oder Methoxyethyl steht.

4. Verbindung nach einem der voranstehenden Ansprüche, wobei R² für Wasserstoff steht.

5. Verbindung nach einem der voranstehenden Ansprüche, wobei L für eine dreiatomige Verknüpfung, ausgewählt aus -CH₂-O-CH₂-, -CH₂-CH₂-O- und -CH₂-CH₂-CH₂-, steht, wobei die rechte Seite der Verknüpfung an R³ gebunden ist.

6. Verbindung nach Anspruch 5, wobei L für -CH₂-CH₂-O- oder -CH₂-CH₂-CH₂- steht.

7. Verbindung nach einem der voranstehenden Ansprüche, wobei R³ für Phenyl steht, das unabhängig substituiert sein kann mit einer oder mehreren Gruppen, ausgewählt aus: C₁₋C₆-Alkyl, Halogen, Halogen-C₁-C₆-alkyl, C₁-C₆-Alkoxy, Halogen-C₁-C₆-alkoxy, C₁-C₆-Alkylthio, Halogen-C₁-C₆₋alkylthio und Nitril.

8. Verbindung nach Anspruch 7, wobei R³ für Phenyl steht, das unabhängig substituiert sein kann mit einer oder mehreren Gruppen, ausgewählt aus: C₁-C₆-Alkyl, Halogen, Halogen-C₁-C₆-alkyl, C₁-C₆-Alkoxy und Halogen-C₁-C₆-alkoxy.

9. Verbindung nach Anspruch 8, wobei R³ für 4-Fluorphenyl, 4-Chlorphenyl, 4-Methoxyphenyl oder 4-Methylphenyl steht.

10. Verbindung nach einem der voranstehenden Ansprüche, wobei die biolabilen esterbildenden Gruppen C₁-C₆-Alkyl, Carbocyclyl oder Heterocyclyl sind, von denen jede substituiert sein kann.

11. Verbindung nach Anspruch 10, wobei die biolabilen esterbildenden Gruppen sind: i) C₁-C₆-Alkyl, das optional substituiert ist mit Hydroxy, Oxo, Halogen, Halogen-C₁₋C₆-alkyl, C₁-C₆-Alkoxy, Halogen-C₁-C₆-alkoxy, C₁-C₆₋Alkylthio, Halogen-C₁-C₆-alkylthio, Nitril, Carbocyclyl, Heterocyclyl, Carbocyclyloxy, Heterocyclyloxy, C₁-C₇₋Alkylcarbonyloxy, Carbocyclylcarbonyloxy, Heterocyclylcarbonyloxy, Alkylcarbonylamino und Alkylaminocarbonyl, wobei jede Carbocyclyl- oder Heterocyclylgruppe optional substituiert ist mit C₁-C₆₋Alkyl, Halogen, Halogen-C₁-C₆-alkyl, C₁-C₆-Alkoxy, Halogen-C₁-C₆-alkoxy, C₁-C₆-Alkylthio, Halogen-C₁-C₆₋alkylthio oder Nitril; oder ii) Carbocyclyl oder Heterocyclyl, die gegebenenfalls substituiert sind mit C₁-C₆-Alkyl, Halogen, Halogen-C₁-C₆-alkyl, C₁-C₆-Alkoxy, Halogen-C₁-C₆-alkoxy, C₁-C₆-Alkylthio, Halogen-C₁-C₆₋alkylthio oder Nitril.

12. Verbindung nach Anspruch 11, wobei jede carbocyclische Gruppe Phenyl ist und jede heterocyclische Gruppe aromatisch ist.

13. Verbindung nach Anspruch 11, wobei die biolabilen esterbildenden Gruppen ausgewählt sind aus der Liste: Ethyl, Propyl, Butyl, Isobutyl, Cyclopentyl, Benzyl, 1-(2,2-Diethylbutyryloxy)ethyl, 2-Ethylpropionyloxymethyl, 1-(2-Ethylpropionyloxy)ethyl, 1-(2,4-Dimethylbenzoyloxy)ethyl, 1-(Benzoyloxy)benzyl, 1-(Benzoyloxy)ethyl, 2-Methyl-1-propionyloxypropyl, 2,4,6-Trimethylbenzoyloxymethyl, 1-(2,4,6-Trimethylbenzyloxy)ethyl, Pivaloyloxymethyl, Phenethyl, Phenpropyl, 2,2,2-Trifluorethyl, 1-Naphthyl, 2-Naphthyl, 2,4-Dimethylphenyl, 4-t-Butylphenyl, 5-(4-Methyl-1,3-dioxalynyl-2-onyl)methyl, N,N-Diethylaminocarbonylmethyl und 5-Indanyl.

14. Verbindung nach einem der voranstehenden Ansprüche, wobei R⁴ und R⁵ beide für Wasserstoff stehen.

15. Verbindung nach einem der voranstehenden Ansprüche, wobei die Verbindung eine Verbindung der Formel (Ia) 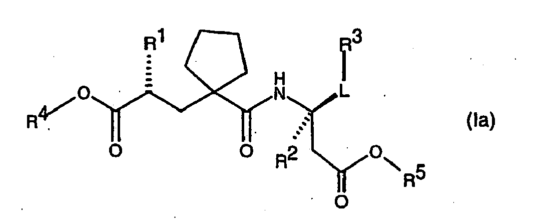 ist.

16. Verbindung nach Anspruch 15, wobei
R¹ für C₁-C₆-Alkyl oder C₁-C₆-Alkoxy-C₁-C₃-alkyl steht;
R² für Wasserstoff steht;
L für eine dreiatomige Verknüpfung, ausgewählt aus: -CH₂-O-CH₂-, -CH₂-CH₂-O- und -CH₂-CH₂-CH₂- steht, wobei die rechte Seite der Verknüpfung an R³ gebunden ist;
R³ für Phenyl steht, das unabhängig substituiert sein kann mit einer oder mehreren Gruppen, ausgewählt aus: C₁-C₆-Alkyl, Halogen, Halogen-C₁-C₆-alkyl, C₁-C₆₋Alkoxy, Halogen-C₁-C₆-alkoxy, C₁-C₆-Alkylthio, Halogen-C₁-C₆-alkylthio und Nitril; und
R⁴ und R⁵ entweder beide für Wasserstoff stehen oder eines von R⁴ und R⁵ für Wasserstoff steht und das andere für eine biolabile esterbildende Gruppe steht, die im Körper eines Patienten durch Wasserstoff ersetzt wird.

17. Verbindung nach Anspruch 16, wobei
R¹ für Propyl oder Methoxyethyl steht;
R² für Wasserstoff steht;
L für -CH₂-CH₂-O- oder -CH₂-CH₂-CH₂- steht, wobei die rechte Seite der Verknüpfung an R³ gebunden ist;
R³ für 4-Fluorphenyl, 4-Chlorphenyl, 4-Methoxyphenyl oder 4-Methylphenyl steht; und
R⁴ und R⁵ beide für Wasserstoff stehen.

18. Verbindung nach Anspruch 1, ausgewählt aus:
(2S)-2-[(1-{[((1R)-2-Carboxy-1-{[(4-Chlorbenzyl)oxy]methyl}ethyl)amino]carbonyl}cyclopentyl)methyl]-4-methoxybutansäure (Beispiel 16);
(3S)-3-[({1-[(2R)-2-Carboxypentyl]cyclopentyl}carbonyl)-amino]-5-(4-chlorphenoxy)pentansäure (Beispiel 41);
Ethyl-(3S)-3-[({1-[(2S)-2-carboxy-4-methoxybutyl]cyclopentyl}carbonyl)amino]-5-(4-chlorphenoxy)pentanoat (Beispiel 47);
(3S)-3-[({1-[(2S)-2-Carboxy-4-methoxybutyl]cyclopentyl}carbonyl)amino]-5-(4-chlorphenoxy)pentansäure (Beispiel 48);
(2S)-2-({1-[({(1S)-3-Butoxy-1-[2-(4-chlorphenoxy)ethyl]-3-oxopropyl}amino)carbonyl]cyclopentyl}methyl)-4-methoxybutansäure (Beispiel 49);
(3S)-3-[({1-[(2S)-2-Carboxy-4-methoxybutyl]cyclopentyl}-carbonyl)amino]-6-(4-chlorphenyl)hexansäure (Beispiel 53) ;
Ethyl-(3S)-3-[({1-[(2S)-2-carboxy-4-methoxybutyl]cyclopentyl}carbonyl)amino]-6-(4-chlorphenyl)hexanoat (Beispiel 52); und
(3S)-3-[({1-[(2S)-2-carboxy-4-methoxybutyl]cyclopentyl}carbonyl)amino]-6-(4-methoxyphenyl)hexansäure (Beispiel 54).

19. Verwendung einer Verbindung, die in einem der voranstehenden Ansprüche definiert ist, eines pharmazeutisch annehmbaren Salzes, Solvats, einer polymorphen Form oder eines Prodrug davon zur Herstellung eines Medikaments zur Behandlung oder Prävention einer Erkrankung, bei der eine vorteilhafte Wirkung erzielt wird durch Inhibierung der neutralen Endopeptidase.

20. Verwendung nach Anspruch 19, wobei die Erkrankung eine kardiovaskuläre Erkrankung ist.

21. Verwendung nach Anspruch 20, wobei die Erkrankung Bluthochdruck ist.

22. Verwendung nach Anspruch 19, wobei die Erkrankung sexuelle Dysfunktion bei Frauen oder erektile Dysfunktion bei Männern ist.

23. Verbindung, die in einem der Ansprüche 1 bis 18 definiert ist, pharmazeutisch annehmbares Salz, Solvat, polymorphe Form oder Prodrug davon zur Verwendung als Medikament.

24. Pharmazeutische Zusammensetzung, enthaltend eine Verbindung, die in einem der Ansprüche 1 bis 18 definiert ist, ein pharmazeutisch annehmbares Salz, Solvat, eine polymorphe Form oder ein Prodrug davon zusammen mit einem pharmazeutisch annehmbaren Hilfsstoff, Verdünnungsmittel oder Träger.

25. Kombination aus einer Verbindung, die in einem der Ansprüche 1 bis 18 definiert ist, und einem oder mehreren aktiven Bestandteilen, ausgewählt aus der Liste:
a) Angiotensinrezeptorblockern (ARB), wie zum Beispiel Losartan, Valsartan, Telmisartan, Candesartan, Irbesartan, Eprosartan und Olmesartan;
b) Kalziumkanalblockern (CCB), wie zum Beispiel Amlodipin;
c) Statinen, wie zum Beispiel Atorvastatin;
d) PDE5-Inhibitoren, wie zum Beispiel Sildenafil, Tadalafil, Vardenafil, 5-[2-Ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-on; 5-(5-Acetyl-2-butoxy-3-pyridinyl)-3-ethyl-2-(1-ethyl-3-azetidinyl)-2,6-dihydro-7H-pyrazolo[4,3-d]pyrimidin-7-on und; die Pyrazolo[4,3-d]pyrimidin-4-one, die in der WO 00/27848 beschrieben sind, insbesondere N-[[3-(4,7-Dihydro-1-methyl-7-oxo-3-propyl-1H-pyrazolo[4,3-d]pyrimidin-5-yl)-4-propoxyphenyl]sulfonyl]-1-methyl-2-pyrrolidinpropanamid [DA-8159 (Beispiel 68 aus der WO 00/27848)];
e) Beta-Blockern, wie zum Beispiel Atenolol oder Carvedilol;
f) ACE-Inhibitoren, wie zum Beispiel Quinapril, Enalapril und Lisinopril;
g) Alpha-Blockern, wie zum Beispiel Doxazosin;
h) selektiven Aldosteronrezeptorantagonisten (SARA), wie zum Beispiel Eplerenon oder Spironolacton;
i) Imidazolin-I₁-Agonisten, wie zum Beispiel Rilmenidin; und
j) Endothelin-Rezeptor-Antagonisten und Endothelin-Converting-Enzyme-Inhibitoren.

## Revendications

1. Composé de formule (I) ou un de ses sels ou produits de solvatation pharmaceutiquement acceptables 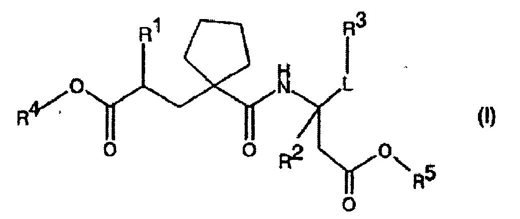 formule dans laquelle
R¹ représente un groupe alkyle en C₁ à C₆, (alkoxy en C₁ à C₆) (alkyle en C₁ à C₃) ou (alkoxy en C₁ à C₆) (alkoxy en C₁ à C₆) (alkyle en C₁ à C₃) ;
R² représente un atome d'hydrogène ou un groupe alkyle en C₁ à C₆ ;
L représente un groupe de liaison de trois atomes choisi entre des groupes -CH₂-X-CH₂- et -CH₂-CH₂-X- dans lesquels le côté droit du groupe de liaison est fixé à R³ et dans lesquels X représente un atome d'oxygène, un atome de soufre ou un groupe méthylène ;
R³ représente un groupe phényle ou un groupe hétérocyclyle aromatique, chacun de ces groupes pouvant être substitué indépendamment avec un ou plusieurs groupes choisis entre des groupes : alkyle en C₁ à C₆, halogéno, halogénalkyle en C₁ à C₆, alkoxy en C₁ à C₆, halogénalkoxy en C₁ à C₆, alkylthio en C₁ à C₆, halogénalkylthio en C₁ à C₆ et nitrile ; et
R⁴ et R⁵ représentent l'un et l'autre un atome d'hydrogène, ou bien un de R⁴ et R⁵ représente un atome d'hydrogène et l'autre représente un groupe biolabile de formation d'ester qui, dans l'organisme d'un patient, est remplacé par un atome d'hydrogène.

2. Composé suivant la revendication 1, dans lequel R¹ représente un groupe alkyle en C₁ à C₆ ou (alkoxy en C₁ à C₆) (alkyle en C₁ à C₃).

3. Composé suivant la revendication 2, dans lequel R¹ représente un groupe propyle ou méthoxyéthyle.

4. Composé suivant l'une quelconque des revendications précédentes, dans lequel R² représente un atome d'hydrogène.

5. Composé suivant l'une quelconque des revendications précédentes, dans lequel L représente un groupe de liaison de trois atomes choisi entre des groupes -CH₂-O-CH₂-, -CH₂₋CH₂-O- et CH₂-CH₂-CH₂-, dans lesquels le côté droit du groupe de liaison est fixé à R³.

6. Composé suivant la revendication 5, dans lequel L représente un groupe -CH₂-CH₂-O- ou CH₂-CH₂-CH₂-.

7. Composé suivant l'une quelconque des revendications précédentes, dans lequel R³ représente un groupe phényle qui peut être substitué indépendamment avec un ou plusieurs groupes choisis entre des groupes : alkyle en C₁ à C₆, halogéno, halogénalkyle en C₁ à C₆, alkoxy en C₁ à C₆, halogénalkoxy en C₁ à C₆, alkylthio en C₁ à C₆, halogénalkylthio en C₁ à C₆ et nitrile.

8. Composé suivant la revendication 7, dans lequel R³ représente un groupe phényle qui peut être substitué indépendamment avec un ou plusieurs groupes choisis entre des groupes : alkyle en C₁ à C₆, halogéno, halogénalkyle en C₁ à C₆, alkoxy en C₁ à C₆ et halogénalkoxy en C₁ à C₆.

9. Composé suivant la revendication 8, dans lequel R³ représente un groupe 4-fluorophényle, 4-chlorophényle, 4-méthoxypliényle ou 4-méthylphényle.

10. Composé suivant l'une quelconque des revendications précédentes, dans lequel les groupes biolabiles de formation d'ester sont des groupes alkyle en C₁ à C₆, carbocyclyle ou hétérocyclyle dont chacun peut être substitué.

11. Composé suivant la revendication 10, dans lequel les groupes biolabiles de formation d'ester sont : i) un groupe alkyle en C₁ à C₆ facultativement substitué avec un substituant hydroxy, oxo, halogéno, halogénalkyle en C₁ à C₆, alkoxy en C₁ à C₆, halogénalkoxy en C₁ à C₆, alkylthio en C₁ à C₆, halogénalkylthio en C₁ à C₆, nitrile, carbocyclyle, hétérocyclyle, carbocyclyloxy, hétérocyclyloxy, (alkyle en C₁ à C₇)carbonyloxy, carbocyclylcarbonyloxy, hétérocyclylcarbonyloxy, alkylcarbonylamino et alkylamino-carbonyle, dans lesquels n'importe quel groupe carbocyclyle ou hétérocyclyle est facultativement substitué avec un substituant alkyle en C₁ en C₆, halogéno, halogénalkyle en C₁ à C₆, alkoxy en C₁ à C₆, halogénalkoxy en C₁ à C₆, alkylthio en C₁ à C₆, halogénalkylthio en C₁ à C₆ ou nitrile ; ou ii) des groupes carbocyclyle ou hétérocyclyle facultativement substitués avec un groupe alkyle en C₁ à C₆, halogéno, halogénalkyle en C₁ à C₆, alkoxy en C₁ à C₆, halogénalkoxy en C₁ à C₆, alkylthio en C₁ à C₆, halogénalkylthio en C₁ à C₆ ou nitrile.

12. Composé suivant la revendication 11, dans lequel n'importe quel groupe carbocyclique est un groupe phényle et n'importe quel groupe hétérocyclique est aromatique.

13. Composé suivant la revendication 11, dans lequel les groupes biolabiles de formation d'ester sont choisis dans la liste : éthyle, propyle, butyle, isobutyle, cyclopentyle, benzyle, 1-(2,2-diéthylbutyryloxy)éthyle, 2-éthylpropionyloxyméthyle, 1-(2-éthylpropionyloxy)éthyle, 1-(2,4-diméthylbenzoyloxy)éthyle, 1-(benzoyloxy)benzyle, 1-(benzoyloxy) éthyle, 2-méthyl-1-propionyloxypropyle, 2,4,6-triméthylbenzoyloxyméthyle, 1-(2,4,6-triméthylbenzyloxy)-éthyle, pivaloyloxyméthyle, phénétyle, phenpropyle, 2,2,2-trifluoréthyle, 1-naphtyle, 2-naphtyle, 2,4-diméthylphényle, 4-t-butylphényle, 5-(4-méthyl-1,3-dioxalynyl-2-onyl)méthyle, N,N-diéthylaminocarbonylméthyle et 5-indanyle.

14. Composé suivant l'une quelconque des revendications précédentes, dans lequel R⁴ et R⁵ représentent l'un et l'autre un atome d'hydrogène.

15. Composé suivant l'une quelconque des revendications précédentes, le composé étant un composé de formule (Ia)

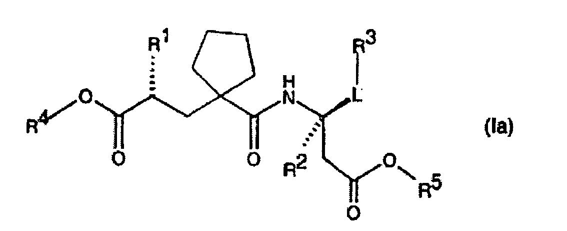

16. Composé suivant la revendication 15, dans lequel
R¹ représente un groupe alkyle en C₁ à C₆ ou (alkoxy en C₁ à C₆) (alkyle en C₁ à C₃) ;
R² représente un atome d'hydrogène ;
L représente un groupe de liaison de trois atomes choisi entre des groupes -CH₂-O-CH₂-, -CH₂-CH₂-O- et CH₂-CH₂₋CH₂-, dans lesquels le côté droit du groupe de liaison est fixé à R³ ;
R³ représente un groupe phényle qui peut être substitué indépendamment avec un ou plusieurs groupes choisis entre des groupes : alkyle en C₁ à C₆, halogéno, halogénalkyle en C₁ à C₆, alkoxy en C₁ à C₆, halogénalkoxy en C₁ à C₆, alkylthio en C₁ à C₆, halogénalkylthio en C₁ à C₆ et nitrile ; et
R⁴ et R⁵ représentent l'un et l'autre un atome d'hydrogène, ou bien un de R⁴ et R⁵ représente un atome d'hydrogène et l'autre représente un groupe biolabile de formation d'ester qui, dans l'organisme d'un patient, est remplacé par un atome d'hydrogène.

17. Composé suivant la revendication 16, dans lequel
R¹ représente un groupe propyle ou méthoxyéthyle ;
R² représente un atome d'hydrogène ;
L représente un groupe -CH₂-CH₂-O- ou CH₂-CH₂-CH₂-, dans lesquels le côté droit du groupe de liaison est fixé à R³ ;
R³ représente un groupe 4-fluorophényle, 4-chlorophényle, 4-méthoxyphényle ou 4-méthylphényle ; et
R⁴ et R⁵ représentent l'un et l'autre un atome d'hydrogène.

18. Composé suivant la revendication 1, choisi entre :
acide (2*S*)-2-[(1-{[((1*R*)-2-carboxy-1-{[(4-chlorobenzyl)-oxy]méthyl}éthyl)-amino]carbonyl}cyclopentyl)méthyl]-4-méthoxybutanoïque (exemple 16) ;
acide (3*S*)-3-[({1-[(2*R*)-2-carboxypentyl]cyclopentyl}carbonyl)-amino]-5-(4-chlorophénoxy)pentanoïque (exemple 41) ;
(3*S*)-3-[({1-[(2*S*)-2-carboxy-4-méthoxybutyl]cyclopentyl}-carbonyl)amino]-5-(4-chlorophénoxy)pentanoate d'éthyle (exemple 47) ;
acide (3*S*)-3-[({1-[(2*S*)-2-carboxy-4-méthoxybutyl]cyclopentyl}]-carbonyl)amino]-5-(4-chlorophénoxy)pentanoïque (exemple 48) ;
acide (2*S*)-2-({1-[({(1*S*)-3-butoxy-1-[2-(4-chlorophénoxy)-éthyl]-3-oxopropyl}amino)carbonyl]cyclopentyle}méthyl)-4-méthoxybutanoïque (exemple 49) ;
acide (3*S*)-3-[({1-[(2*S*)-2-carboxy-4-méthoxybutyl]cyclopentyl}-carbonyl)amino]-6-(4-chlorophényl)hexanoïque (exemple 53) ;
(3*S*)-3-[({1-[(2*S*)-2-carboxy-4-méthoxybutyl)cyclopentyl}-carbonyl)amino]-6-(4-chlorophényl)hexanoate d'éthyle (exemple 52) ; et
acide (3S)-3-[({1-[(2S)-2-carboxy-4-méthoxybutyl]cyclapentyl}-carbonyl)amino]-6-(4-méthoxyphényl)hexanoïque (exemple 54).

19. Utilisation d'un composé défini dans l'une quelconque des revendications précédentes ou d'un de ses sels, d'un de ses produits de solvatation, d'une de ses formes polymorphes ou d'un de ses précurseurs médicamenteux pharmaceutiquement acceptables, dans la production d'un médicament destiné au traitement ou à la prévention d'une affection pour laquelle une réponse bénéfique est obtenue par inhibition de l'endopeptidase neutre.

20. Utilisation suivant la revendication 19, dans laquelle l'affection est une maladie ou affection cardiovasculaire.

21. Utilisation suivant la revendication 20, dans laquelle l'affection est l'hypertension.

22. Utilisation suivant la revendication 19, dans laquelle l'affection est un dysfonctionnement sexuel féminin ou un dysfonctionnement de l'érection masculine.

23. Composé défini dans l'une quelconque des revendications 1 à 18, ou un de ses sels, un de ses produits de solvatation, une de ses formes polymorphes ou un de ses précurseurs médicamenteux pharmaceutiquement acceptables, destiné à être utilisé comme médicament.

24. Composition pharmaceutique comprenant un composé défini dans l'une quelconque des revendications 1 à 18, ou un de ses sels, un de ses produits de solvatation, une de ses formes polymorphes ou un de ses précurseurs médicamenteux pharmaceutiquement acceptables, conjointement avec un excipient, diluant ou support pharmaceutiquement acceptable.

25. Association d'un composé défini dans l'une quelconque des revendications 1 à 18 et d'un ou plusieurs ingrédients actifs choisis dans la liste :
a) des agents de blocage du récepteur d'angiotensine (ARB), tels que le losartan, le valsartan, le telmisartan, le candésartan, l'irbésartan, l'éprosartan et l'olmésartan ;
b) des agents de blocage du canal calcium (CCB) tels que l'amlodipine ;
c) des statines, telles que l'atorvastatine ;
d) des inhibiteurs de PDE5, tels que le sidénafil, le tadalafil, le vardénafil, la 5-[2-éthoxy-5-(4-éthylpipérazine-1-ylsulfonyl)pyridine-3-yl]-3-éthyl-2-[2-méthoxyéthyl]-2,6-dihydro-7H-pyrazolo[4,3-d]pyrimidine-7-one ; la 5-(5-acétyl-2-butoxy-3-pyridinyl)-3-éthyl-2-(1-éthyl-3-azétidinyl)-2,6-dihydro-7*H*-pyrazolo[4,3-d]pyrimidine-7-one et les pyrazolo-[4,3-d]pyrimidine-4-ones décrites dans le document WO00/27848, en particulier le N-[[3-(4,7-dihydro-1-méthyl-7-oxo-3-propyl-1H-pyrazolo[4,3-d]-pyrimidine-5-yl)-4-propoxyphényl]sulfony]-1-méthyl-2-pyrrolidinepropanamide [DA-8159 (exemple 68 du document WO00/27848)] ;
e) des bêta-bloquants, tels que l'aténolol ou le carvédilol ;
f) des inhibiteurs de ACE, tels que le quinapril, l'énalapril et le lisinopril ;
g) des alpha-bloquants tels que la doxazosine ;
h) des antagonistes du récepteur d'aldostérone sélectifs (SARA), tels que l'éplérénone ou la spironolactone ;
i) des agonistes des imidazolines I₁, tels que la rilménidine ; et
j) des antagonistes du récepteur d'endothéline et des inhibiteurs d'enzyme de conversion d'endothéline.
